(19) **Europäisches Patentamt / European Patent Office / Office européen des brevets**

(11) **EP 4 692 783 A1**

(12) **EUROPEAN PATENT APPLICATION**
published in accordance with Art. 153(4) EPC

(43) Date of publication:
**11.02.2026  Bulletin 2026/07**

(21) Application number: **23929625.4**

(22) Date of filing: **10.05.2023**

(51) International Patent Classification (IPC):
*G01N 30/02* (2006.01)

(52) Cooperative Patent Classification (CPC):
**G01N 30/02; G01N 30/72; G01N 33/53;**
**G01N 33/68; G01N 33/72**

(86) International application number:
**PCT/CN2023/093183**

(87) International publication number:
**WO 2024/198046 (03.10.2024 Gazette 2024/40)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **30.03.2023  CN 202310329348**
**30.03.2023  CN 202310329378**
**09.05.2023  CN 202310519559**

(71) Applicant: **Shanghai Ganning Medical and**
**Technology Co., Ltd**
**Shanghai 201318 (CN)**

(72) Inventors:
• **LI, Hai**
**Shanghai 200235 (CN)**
• **GUO, Lining**
**Cary, (US)**
• **ZHANG, Yan**
**Shanghai 200127 (CN)**

(74) Representative: **Dolphin, Kirsty Mairi et al**
**Venner Shipley LLP**
**200 Aldersgate**
**London EC1A 4HD (GB)**

(54) **BIOMARKER AND SYSTEM FOR PREDICTING EVOLUTION INTO ACLF OR DEATH OF ACLF PATIENT, AND USES THEREOF**

(57)    Provided are a biomarker and system for predicting progression to ACLF or mortality of an ACLF patient, and use thereof. Through untargeted metabolomics analysis of ACLF-related samples, a series of biomarkers capable of predicting a short-term mortality risk of the ACLF patient and a short-term progression risk to ACLF of a non-ACLF patient are found, and a biomarker combination is further screened therefrom, to construct a short-term mortality risk prediction model for ACLF patients and a short-term ACLF progression risk prediction model for non-ACLF patients, thereby achieving convenient and efficient prediction, seizing the golden window for intervention for high-risk groups, reducing economic burdens for low-risk patients, and meeting clinical needs.

Fig. 1

**Description**

CROSS-REFERENCE TO RELATED APPLICATIONS

The present application claims priority to Chinese Prior Application No. 2023103293484, filed on March 30, 2023; and Chinese Prior Application No. 2023103293785, filed on March 30, 2023, the entire content of which is incorporated herein as part of the present application.

BACKGROUND OF THE INVENTION

Field of the Invention

The present application relates to the field of biotechnology, and in particular, to a biomarker and system for predicting progression to ACLF or mortality of an ACLF patient, and use thereof.

Description of the Related Art

Acute-on-chronic liver failure (ACLF) is a rapidly progressive disease with rapid clinical deterioration and high mortality, manifesting as acute hepatic dysfunction in patients with chronic liver disease or cirrhosis and characterized by organ failures and high short-term mortality. It is present in more than 25% of patients hospitalized with cirrhosis. ACLF is known to be associated with precipitating insults such as alcohol or viral infection, extrahepatic organ failure, and systemic inflammation. Its pathophysiology is associated with sustained inflammation, immune dysregulation involving initial widespread immune activation, a state of systemic inflammatory response syndrome, and subsequently, sepsis due to immunosuppression. The prevalence of ACLF was reported to be 5.7 cases per 1,000 persons per year based on Asian Pacific criteria and 20.1 based on European criteria. Currently, there is no effective therapy for ACLF. The existing treatment options mainly include treating the precipitating events, organ support, and liver transplant. Despite intensive hospital care, ACLF's prognosis is poor. The short-term mortality within 90 days is around 50%. Meanwhile, after the initial non-ACLF diagnosis (pre-ACLF patients), up to 20% of these patients can develop ACLF within 3 months. Early identification of the pre-ACLF patients for proactive disease treatment and monitoring can significantly improve their clinical outcomes. It can be seen that early diagnosis and accurate prognosis for disease management are critical for improving ACLF survival.

The traditional scoring systems for cirrhosis, including the model for end-stage liver disease (MELD) score, the MELD-sodium (MELD-Na) score, and the Child-Turcotte Pugh (CTP) score, have limited accuracy for ACLF prognosis. The development of more accurate tests for ACLF prognosis has been a subject of intensive research in the last decade. Based on clinical and laboratory tests, the CANONIC study by the European Foundation for the study of chronic liver failure (CLIF) proposed the CLIF-C ACLF score which significantly enhanced the accuracy for the prediction of short-term mortality compared to the traditional scoring systems. The latest biomarker discovery approaches include esoteric clinical tests such as serum ammonia, inflammatory markers, D-dimer, proteomics, and metabolomics. However, the discovery and validation processes often relied on relatively small clinical cohorts and needed further validation. Therefore, it remains necessary to find a more accurate and effective ACLF prognostic test to meet clinical needs.

Chinese Acute-on-chronic liver failure Consortium discovered that among patients hospitalized with acute exacerbation of chronic liver disease, two groups of patients exhibit golden windows for intervention: one group is patients who were non-ACLF on admission but progressed to organ failure or even ACLF within 28 days of admission (i.e., those identified as pre-ACLF on admission). The phase before these patients develop ACLF is a very good window for proactive intervention. The other group is patients who were ACLF on admission but died within 29-90 days after admission. For this group, the first four weeks after admission are a golden window for pharmacological intervention.

Therefore, there is an urgent need to find a method for assessing the short-term mortality risk of ACLF patients and the short-term progression risk to ACLF of non-ACLF patients. When patients at a short-term mortality risk or a short-term progression risk to ACLF are identified, intensive therapy or early liver transplantation needs to be carried out in time, achieving precise screening and rapid proactive clinical intervention to improve the survival rate of patients. Moreover, for low-risk patients, healthcare resources can be conserved, and financial burdens can be reduced.

BRIEF SUMMARY OF THE INVENTION

Aiming at the problems in the prior art, the present application provides a biomarker and system for predicting progression to ACLF or mortality of an ACLF patient, and use thereof. Through untargeted metabolomics analysis of ACLF-related samples, a series of biomarkers capable of predicting a short-term mortality risk of the ACLF patient and/or a short-term progression risk to ACLF of a non-ACLF patient are found. Moreover, these new biomarkers are combined effectively, and a biomarker combination is further screened therefrom, to construct a short-term mortality risk prediction model for ACLF patients and a short-term ACLF progression risk prediction model for non-ACLF patients, thereby achieving convenient and efficient prediction, seizing the golden window for intervention for high-risk groups, reducing economic burdens for low-risk patients, and meeting clinical needs.

Acute-on-chronic liver failure (ACLF) is a rapidly progressive disease with rapid clinical deterioration and high mortality, manifesting as acute hepatic dysfunction in patients with chronic liver disease or cirrhosis and characterized by organ failures and high short-term mortality. It is present in more than 25% of patients hospitalized with cirrhosis.

It should be understood that the term "short-term" in "short-term progression to ACLF for non-ACLF patients" or "short-term mortality risk for ACLF patients" does not denote an absolute or fixed time point. Although clinically there is a commonly used time point such as "90-day mortality in the ACLF group and 28-day progression to ACLF in the non-ACLF group" and other related time points, this is merely a time point currently summarized based on clinical experience. As time goes by or with improvements in other therapeutic methods, this time point or the length of time is likely to change. For example, progression from non-ACLF to ACLF or development from ACLF to death could occur within 90 days, within 100 days, within one year, within 360 days, within half a year, within 180 days, within 80 days, within 20 days, within 4 weeks, within 3 weeks, within 2 weeks, or within 1 week, etc.

The existing traditional ACLF-related scoring systems often relied on relatively small clinical cohorts and were difficult to achieve better validation. A more accurate and effective ACLF prognostic test remains an unmet clinical need. In the present application, metabolomics analysis was carried out on 1,300 serum samples from chronic liver disease patients hospitalized with acute exacerbation (including 420 ACLF cases and 880 non-ACLF cases). The samples were randomly divided into two equal halves as a discovery set and a validation set. In the discovery set, statistical analysis and random forest analysis were used to determine metabolites associated with 90-day mortality in the ACLF group and 28-day progression to ACLF in the non-ACLF group. A prediction model was developed and well-validated in the validation set.

Research demonstrates that in the ACLF group and the non-ACLF group, the serum metabolome is significantly altered, particularly in pathways involving membrane lipid metabolism, steroid hormone metabolism, oxidative stress, and energy metabolism. Many metabolites in the ACLF group and/or the non-ACLF group showed significant associations with 90-day mortality in the ACLF group and 28-day progression to ACLF in the non-ACLF group. In the present application, a machine learning algorithm for predicting the 90-day mortality in the ACLF group (area under curve (AUC): 0.80) and the 28-day progression to ACLF in the non-ACLF group (AUC: 0.85) was trained and validated. In the present application, using liquid chromatography-mass spectrometry (LC-MS), targeted analysis was carried out on selected metabolites, and the constructed model is suitable for clinical implementation.

In an aspect, the present application provides use of a biomarker for preparing a reagent for predicting a mortality risk or a probability of survival of an acute-on-chronic liver failure (ACLF) patient, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, $\gamma$-carboxyethyl-hydroxychroman ($\gamma$-CEHC) (3-(6-hydroxy-2,7,8-trimethyl-3,4-dihydro-2H-chromen-2-yl)propanoic acid), thyroxine, for-miminoglutamic acid, $\gamma$-glutamylphenylalanine ($\gamma$-glu-phe), and bilirubin.

Further, the predicting the mortality risk of the ACLF patient refers to predicting a mortality risk of the ACLF patient within a short term.

The term "short term" herein may refer to the mortality risk of ACLF patients within 90 days, 180 days, or 360 days; of course, it may be within less than 90 days, e.g., within 80 days, 70 days, 60 days, or shorter; or within 1 year, 11 months, 10 months, 9 months, 8 months, 7 months, 6 months, 5 months, 4 months, 3 months, 2 months, or 1 month, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

In the present application, through untargeted metabolomics research, serum samples from ACLF patients on admission were collected. The samples were divided into a survivor group and a non-survivor group (mortality) according to a survival status within a short term. Using ultra-performance liquid chromatography-tandem mass spectrometry (UPLC-MS/MS), the serum samples on admission from the survivor group and the non-survivor group were analyzed to find a series of biomarkers capable of predicting the short-term mortality risk of the ACLF patients. Machine learning, statistical analysis, and pathway enrichment analysis (including random forest, false discovery rate (FDR) correction, and t-test) were used to determine candidate biomarkers in the discovery set and the priorities thereof. Significantly differential metabolites were screened, ultimately yielding 15 biomarkers suitable for efficient prediction of the short-term mortality risk of the ACLF patients.

In some embodiments, reagents for biomarkers predicting the mortality risk or probability of survival of the ACLF patients refer to detection agents prepared by taking the biomarkers as detection targets, such as sample pretreatment reagents, antigens or antibodies, and other biological reagents and kits applicable to the detection of the biomarkers. These reagents may also be developed into standardized reagents or kits applicable to liquid chromatography-ultraviolet (LC-UV) or liquid chromatography-mass spectrometry (LC-MS) assay of the biomarkers.

Further, the biomarker comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and $\gamma$-CEHC; or, the biomarker comprises pipecolic acid, NAAG, ureidopropionic acid, formiminoglutamic acid, $\gamma$-CEHC, and $\gamma$-glu-phe; or, the biomarker comprises pipecolic acid, NAAG, ureidopropionic acid, formiminoglutamic acid, $\gamma$-CEHC, $\gamma$-glu-phe, and 3-OH butyrylcarnitine; or, the biomarker comprises pipecolic acid, NAAG, ureidopropionic acid, formiminoglutamic acid, $\gamma$-CEHC, $\gamma$-glu-phe, 3-OH butyrylcarnitine, and estrone sulfate; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, and hydroxyproline; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, and bilirubin; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

Further, the biomarker comprises one or more of: pipecolic acid, NAAG, ureidopropionic acid, and formiminoglutamic acid.

Further, the biomarker comprises one or more of: pipecolic acid, NAAG, and ureidopropionic acid.

By evaluating the concentration differences in the serum samples on admission between the survivor group and the non-survivor group, AUC ranking, and significance ranking of the biomarkers, biomarkers capable of significantly distinguishing the survivor group from the non-survivor group were further selected from the 15 biomarkers. The biomarkers can be used for more effectively predicting the short-term mortality risk of the ACLF patients, or used for construction of a diagnostic model for predicting the short-term mortality risk of the ACLF patients.

Further, the reagent is used for detecting the biomarker in a blood, serum, plasma, or whole blood sample from the ACLF patient.

Further, the reagent is used for detecting a presence or absence, relative abundance, relative concentration, or alteration in level or quantity of the biomarker in the sample. The presence or absence or level of the biomarkers herein is a relative concept. For example, when comparing ACLF decedents to survivors, the levels of these specific biomarkers are compared relative to baseline levels in ACLF decedents or survivors. Some biomarkers may exhibit higher levels in ACLF decedents or high-mortality-risk patients compared to ACLF survivors. Such elevations have statistical differences, e.g., significant or highly significant elevations. When judging whether non-ACLF patients progress to ACLF, the levels of these specific biomarkers may serve as comparative standards relative to non-ACLF or ACLF patients. Therefore, during judgment using these biomarkers, if a single biomarker is used and the biomarker has an elevated probability of occurrence at a risk, the level of the biomarker will change. The change herein may refer to a relative increase or relative decrease. Such relative increase or relative decrease differences are significant, and of course, may also be highly significant. Therefore, regardless of the detection method employed, a predetermined value may serve as a standard (cut-off value). If exceeding this value, it is considered that the level has changed. The biomarkers having such outcomes may serve as predictive or diagnostic value.

Therefore, in some aspects, the biomarkers of the present application may be obtained by detecting marker levels in samples using any existing known method, such as liquid chromatography (LC), gas chromatography (GC), mass spectrometry (MS), LC-MS, GC-MS, collision cell-mass spectrometry (CC-MS), LC-MS-MS, nuclear magnetic resonance (NMR), immunochromatographic strips, immunoreaction chips, capillary electrophoresis, and infrared spectroscopy. Any method capable of detecting biomarker levels in samples may be used for predicting the short-term mortality risk of ACLF patients. As long as the level in a sample is detectable, the biomarker can be used for prediction or diagnosis of the probability of occurrence of a disease. It should be understood that the term "detection" herein refers to detecting an individual's sample and then comparing the result to the predetermined standard. The comparative result is used for judging or predicting the occurrence status of a disease, for example, used for predicting mortality or mortality probability of ACLF patients, or predicting survival or survival probability of ACLF patients, or predicting progression to ACLF of non-ACLF patients. Such predictions or diagnoses are for whether a clinical event occurs within a time period. Of course, such detections may be serial detections, where changes in some analytes enable the assessment of the progression of a disease.

In some embodiments, the relative abundance refers to the peak area of the biomarker in the detection profile obtained via high-performance liquid chromatography-tandem mass spectrometry (HPLC-MS/MS). For example, if the mean peak area of a biomarker is measured as 500 in control samples (from the short-term survivor group of ACLF patients) and 3,000 in samples from the short-term non-survivor group of ACLF patients, the abundance of the biomarker in the samples is considered sixfold higher than that in the control sample.

Further, the new biomarkers discovered in the present application may be combined with the following factors for prediction or judgment. The following factors may be some chemical substances or some indicators. Therefore, in some embodiments, diagnosis or prediction may further be carried out in combination with one or more factors: age, international normalized ratio (INR), creatine (CR), Sodium, albumin (ALB), neutrophil/lymphocyte count ratio (N/L ratio), overt hepatic encephalopathy (overt HE), and total bilirubin (TB).

Further, the predicting the mortality risk or the probability of survival of the ACLF patient refers to predicting a mortality risk or a probability of survival of the ACLF patient within 360 days.

Further, the predicting the mortality risk or the probability of survival of the ACLF patient refers to predicting a mortality risk or a probability of survival of the ACLF patient within 90 days.

In another aspect, the present application provides use of a biomarker for preparing a reagent for predicting progression to acute-on-chronic liver failure (ACLF) of a non-ACLF patient, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, $\gamma$-carboxyethylhydroxychroman($\gamma$-CEHC), thyroxine, formiminoglutamic acid, $\gamma$-glutamylphenylalanine ($\gamma$-glu-phe), and bilirubin.

Further, the predicting progression to ACLF of the non-ACLF patient refers to predicting progression to ACLF of the non-ACLF patient within a short term.

The term "short term" herein may refer to the progression risk to ACLF of non-ACLF patients within 180 days, 90 days, or 28 days; of course, it may be within 1 year, 11 months, 10 months, 9 months, 8 months, 7 months, 6 months, 5 months, 4 months, 3 months, 2 months, or 1 month, etc.; or within 10 weeks, 9 weeks, 8 weeks, 7 weeks, 6 weeks, 5 weeks, 4 weeks, 3 weeks, 2 weeks, or 1 weeks, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

In the present application, through untargeted metabolomics research, serum samples from non-ACLF patients on admission were collected. The samples were divided into a pre-ACLF group (short-term progression to ACLF) and a non-ACLF group according to an ACLF progression status within a short term. Using ultra-performance liquid chromatography-tandem mass spectrometry (UPLC-MS/MS), the serum samples on admission from the pre-ACLF group and the non-ACLF group were analyzed to find a series of biomarkers capable of predicting the short-term progression to ACLF of the non-ACLF patients. Machine learning, statistical analysis, and pathway enrichment analysis (including random forest, FDR correction, and t-test) were used to determine candidate biomarkers in the discovery set and the priorities thereof. Significantly differential metabolites were screened, ultimately yielding 15 biomarkers suitable for efficient prediction of the short-term progression risk to ACLF of the non-ACLF patients.

In the present application, the term "pre-ACLF" refers to patients who progress to ACLF within a short term after initial diagnosis as non-ACLF.

The biomarkers of the present application may be obtained by detecting marker levels in samples using any method, such as LC, GC, MS, LC-MS, GC-MS, CC-MS, LC-MS-MS, NMR, immunochromatographic strips, immunoreaction chips, capillary electrophoresis, and infrared spectroscopy. Any method capable of detecting biomarker levels in samples may be used for predicting the short-term progression risk to ACLF of non-ACLF patients.

Further, the biomarker comprises ureidopropionic acid and $\gamma$-CEHC; or, the biomarker comprises ureidopropionic acid, $\gamma$-CEHC, and thyroxine; or, the biomarker comprises ureidopropionic acid, $\gamma$-CEHC, and pipecolic acid; or, the biomarker comprises ureidopropionic acid, $\gamma$-CEHC, thyroxine, and pipecolic acid; or, the biomarker comprises ureidopropionic acid, $\gamma$-CEHC, thyroxine, pipecolic acid, and 3-OH butyrylcarnitine; or, the biomarker comprises ureidopropionic acid, $\gamma$-CEHC, thyroxine, pipecolic acid, 3-OH butyrylcarnitine, and 2-hydroxydadipic acid; or, the biomarker comprises ureidopropionic acid, $\gamma$-CEHC, thyroxine, pipecolic acid, 3-OH butyrylcarnitine, 2-hydroxydadipic acid, and estrone sulfate; or, the biomarker comprises ureidopropionic acid, $\gamma$-CEHC, thyroxine, pipecolic acid, 3-OH butyrylcarnitine, 2-hydroxydadipic acid, estrone sulfate, and hydroxyproline; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, and $\gamma$-glu-phe; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, and formiminoglutamic acid; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, formiminoglutamic acid, and bilirubin; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-

glu-phe, formiminoglutamic acid, bilirubin, and hexadecanedioic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and NAAG.

Further, the biomarker comprises one or more of: ureidopropionic acid, γ-CEHC, and thyroxine; or ureidopropionic acid, γ-CEHC, and pipecolic acid.

Further, the biomarker comprises one or more of: ureidopropionic acid and γ-CEHC.

By evaluating the concentration differences in the serum samples on admission between the pre-ACLF group and the non-ACLF group, AUC ranking, and significance ranking of the biomarkers, biomarkers capable of significantly distinguishing the pre-ACLF group from the non-ACLF group were further selected from the 15 biomarkers. The biomarkers can be used for more effectively predicting the short-term progression risk to ACLF of the non-ACLF patients, or used for construction of a diagnostic model for predicting the short-term progression risk to ACLF of the non-ACLF patients.

Further, the use comprises predicting progression to ACLF of the non-ACLF patient within 180 days.

In some embodiments, the risk or probability of non-ACLF patients progressing to ACLF within 180 days may be predicted. The term "within 180 days" herein may be less than, equal to, or greater than 180 days.

In some embodiments, reagents for biomarkers predicting the progression risk to ACLF of the non-ACLF patients refer to detection agents prepared by taking the biomarkers as detection targets, such as sample pretreatment reagents, antigens or antibodies, and other biological reagents and kits applicable to the detection of the biomarkers. These reagents may also be developed into standardized reagents or kits applicable to LC-UV or LC-MS assay of the biomarkers.

Further, the reagent is used for detecting the biomarker in a blood, serum, plasma, or whole blood sample from the non-ACLF patient, and used for detecting a presence or absence, relative abundance, or concentration of the biomarker in the sample.

Further, the biomarker further comprises one or more of total bilirubin (TB), international normalized ratio (INR), and Cirrhosis.

In yet another aspect, the present application provides a kit for predicting a mortality risk or a probability of survival of an acute-on-chronic liver failure (ACLF) patient, comprising a detection reagent for a biomarker for the use mentioned above.

Further, the detection reagent for the biomarker comprises a standard for the biomarker.

In yet another aspect, the present application provides a kit for predicting progression to acute-on-chronic liver failure (ACLF) of a non-ACLF patient, comprising a detection reagent for a biomarker for the use mentioned above.

Further, the detection reagent for the biomarker comprises a standard for the biomarker.

In yet another aspect, the present application provides a biomarker combination for predicting a mortality risk of an acute-on-chronic liver failure (ACLF) patient, comprising the following biomarkers: pipecolic acid, N-acetylaspartylglutamate (NAAG), and ureidopropionic acid; or pipecolic acid, NAAG, ureidopropionic acid, and formiminoglutamic acid; or pipecolic acid, NAAG, ureidopropionic acid, formiminoglutamic acid, and γ-carboxyethylhydroxychroman (γ-CEHC); or pipecolic acid, NAAG, ureidopropionic acid, formiminoglutamic acid, γ-CEHC, and γ-glutamylphenylalanine (γ-glu-phe); or pipecolic acid, NAAG, ureidopropionic acid, formiminoglutamic acid, γ-CEHC, γ-glu-phe, and 3-OH butyrylcarnitine; or pipecolic acid, NAAG, ureidopropionic acid, formiminoglutamic acid, γ-CEHC, γ-glu-phe, 3-OH butyrylcarnitine, and estrone sulfate; or pipecolic acid, NAAG, ureidopropionic acid, formiminoglutamic acid, γ-CEHC, γ-glu-phe, 3-OH butyrylcarnitine, estrone sulfate, and hydroxyproline; or pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

Further, the biomarker combination comprises the following biomarkers: pipecolic acid, NAAG, ureidopropionic acid, and formiminoglutamic acid; or pipecolic acid, NAAG, and ureidopropionic acid.

Further, the biomarker combination further comprises any one or more of age, international normalized ratio (INR), creatine, Sodium (Na), albumin (ALB), neutrophil/lymphocyte count ratio (N/L ratio), overt hepatic encephalopathy (overt HE), and total bilirubin (TB).

In yet another aspect, the present application provides a biomarker combination for predicting progression to acute-on-chronic liver failure (ACLF) of a non-ACLF patient, comprising the following biomarkers: ureidopropionic acid and γ-carboxyethylhydroxychroman (γ-CEHC); or ureidopropionic acid, γ-CEHC, and thyroxine; or ureidopropionic acid, γ-CEHC, and pipecolic acid; or ureidopropionic acid, γ-CEHC, thyroxine, and pipecolic acid; or ureidopropionic acid, γ-CEHC, thyroxine, pipecolic acid, and 3-OH butyrylcarnitine; or ureidopropionic acid, γ-CEHC, thyroxine, pipecolic acid, 3-OH butyrylcarnitine, and 2-hydroxydadipic acid; or ureidopropionic acid, γ-CEHC, thyroxine, pipecolic acid, 3-OH butyrylcarnitine, 2-hydroxydadipic acid, and estrone sulfate; or ureidopropionic acid, γ-CEHC, thyroxine, pipecolic acid, 3-OH butyrylcarnitine, 2-hydroxydadipic acid, estrone sulfate, and hydroxyproline; or ureidopropionic acid, γ-CEHC, thyroxine, pipecolic acid, 3-OH butyrylcarnitine, 2-hydroxydadipic acid, estrone sulfate, hydroxyproline, and quinolinic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glutamylphenylalanine (γ-glu-phe); or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and N-acetylaspartylglutamate (NAAG).

Further, the biomarker combination comprises the following biomarkers: ureidopropionic acid, γ-CEHC, and thyroxine; or ureidopropionic acid, γ-CEHC, and pipecolic acid; or ureidopropionic acid and γ-CEHC.

Further, the biomarker combination further comprises any one or more of total bilirubin (TB), international normalized ratio (INR), and Cirrhosis.

In yet another aspect, the present application provides a system for predicting a mortality risk or a probability of survival of an acute-on-chronic liver failure (ACLF) patient, comprising a data analysis module, wherein the data analysis module is configured to analyze a detected value of a biomarker, and the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman(γ-CEHC), thyroxine, formiminoglutamic acid, γ-glutamylphenylalanine (γ-glu-phe), and bilirubin.

Further, the detected value of the biomarker refers to detection of the biomarker in a blood, serum, plasma, or whole blood sample from the ACLF patient, and is used for detecting a presence or absence, relative abundance, relative concentration, or relative level of the biomarker in the sample.

Further, the biomarker comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and γ-CEHC; or, the biomarker comprises pipecolic acid, NAAG, ureidopropionic acid, formiminoglutamic acid, γ-CEHC, and γ-glu-phe; or, the biomarker comprises pipecolic acid, NAAG, ureidopropionic acid, formiminoglutamic acid, γ-CEHC, γ-glu-phe, and 3-OH butyrylcarnitine; or, the biomarker comprises pipecolic acid, NAAG, ureidopropionic acid, formiminoglutamic acid, γ-CEHC, γ-glu-phe, 3-OH butyrylcarnitine, and estrone sulfate; or, the biomarker comprises pipecolic acid, NAAG, ureidopropionic acid, formiminoglutamic acid, γ-CEHC, γ-glu-phe, 3-OH butyrylcarnitine, estrone sulfate, and hydroxyproline; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

Further, the data analysis module calculates a prediction value for predicting a mortality risk of the ACLF patient by substituting the detected value of the biomarker into a regression equation, thereby assessing the mortality risk of the ACLF patient.

In the present application, the detected values of biomarkers can be analyzed through any method (such as computer software, manual expert assessment, etc.) to derive certain equations. Subsequently, the test results are substituted into the equations to automatically generate predicted result values. The result values are then compared with predetermined values to judge the probability of disease occurrence or progression. For example, if the value is below the predetermined value, it is predicted that the disease may not occur or has a low probability of occurrence; if the value exceeds the predetermined value, it is predicted that the disease may occur or has a high probability of occurrence. Of course, the inverse scenario is also possible. For example, if the value is below the predetermined value, it is predicted that the disease may occur or has a high probability of occurrence; if the value exceeds the predetermined value, it is predicted that the disease may not occur or has a low probability of occurrence. Therefore, prediction values for predicting the mortality risk of ACLF patients or progression risk to ACLF of non-ACLF patients are calculated. These equations or computer programs are typically generated through analysis between determined groups, followed by validation of accuracy of equation operational results between the determined groups. During validation, based on the prediction values and the predetermined values, the equation operational values from the experimental group and the predetermined values infinitely approach the reagent conditions in the validation group during validation in the validation group. For example, equation operation is trained using mortality and survivor groups of ACLF patients. Subsequently, another mortality and survivor groups of ACLF patients are selected for validation. Through the accuracy of results, an ideal equation is obtained for operation.

It should be understood that as more data becomes available, the equations can undergo continuous refinement or AI self-learning adjustment, enabling the predictive accuracy to be higher, more in line with the actual situation. Consequently, the self-refining equations generate more accurate new result values. Of course, the predetermined values may also change. Therefore, any model or equation can be applied to the present application to enable automatic output of prediction results. Therefore, multiple operational algorithms for computers are available for selection, for example, mixture discriminant analysis (mda), gradient boosting machine (gbm), penalized logistic regression (plr), generalized linear models network (glmnet), random forests (ranger), Naïve_Bayes, neural networks (avNNet), and support vector machines (svmRadial). Any of these operational algorithms may be used for the biomarkers or biomarker combinations of the present application for prediction or diagnosis. When test values of biomarkers (including both new biomarkers discovered in the present application and/or existing biochemical biomarkers) from a subject's sample are substituted into the equations for operation to derive a result value. Through trained and validated data, the result value is compared with a predetermined value to indicate prediction of the probability of disease occurrence. It should be understood that as sample sizes increase, the result values change, and the predetermined values also change. These programs can self-amend or self-refine. Thus, the prediction result is more in line with the actual result, infinitely approaching 100% accuracy. This would have been conceivable and readily achievable to a person of ordinary skill in the art.

Further, the predicting the mortality risk of the ACLF patient refers to predicting a mortality risk of the ACLF patient within a short term.

The term "short term" herein may refer to the mortality risk of ACLF patients within 90 days, 180 days, or 360 days; of course, it may be within less than 90 days, e.g., within 80 days, 70 days, 60 days, or shorter; or within 1 year, 11 months, 10 months, 9 months, 8 months, 7 months, 6 months, 5 months, 4 months, 3 months, 2 months, or 1 month, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

Further, the regression equation is a logistic regression equation.

Further, the logistic regression equation is obtained by constructing and training with detected values of biomarkers from known samples.

Further, the training is to construct a training set and train the training set using a training method to obtain the logistic regression equation; and the training set comprises serum samples from ACLF patients.

Further, the training method comprises: dividing the training set into a survivor group and a non-survivor group according to a survival status within a short term; detecting biomarker levels in the survivor group and the non-survivor group respectively to obtain the detected values; performing logistic regression according to the detected values of the biomarkers to analyze relationships between the detected values in the survivor group and the non-survivor group; and constructing the logistic regression equation.

The training set of the present application may comprise serum samples collected from ACLF patients under any circumstances. That is, the training set may comprise serum samples collected from ACLF patients under different time points, different geographical regions, different sizes, different genders, and different ages.

Further, the short term comprises any one or more of within 90 days, within 180 days, within 360 days, within 80 days, within 70 days, within 60 days, within three months, within half a year, within one year, within two months, and within one month.

Further, the data analysis module calculates a prediction value for predicting a mortality risk of the ACLF patient within 360 days by substituting the detected value of the biomarker into the logistic regression equation, thereby assessing the mortality risk of the ACLF patient within 360 days.

Further, the logistic regression equation is:

P = -20.584 + 0.846 * pipecolic acid + 1.254 * NAAG + 0.454 * ureidopropionic acid - 0.468 * formiminoglutamic acid - 0.385 * γ-CEHC + 0.485 * γ-glu-phe + 0.447 * 3-OH butyrylcarnitine + 0.081 * estrone sulfate + 0.524 * hydroxyproline + 0.079 * bilirubin - 0.066 * 2-hydroxydadipic acid - 0.015 * methionine sulfoxide - 0.089 * hexadecanedioic acid - 0.219 * quinolinic acid + 0.942 * thyroxine;

or P = -14.286 + 0.804 * pipecolic acid + 0.791 * NAAG + 0.448 * ureidopropionic acid - 0.396 * formiminoglutamic acid - 0.338 * γ-CEHC + 0.434 * γ-glu-phe + 0.425 * 3-OH butyrylcarnitine + 0.107 * estrone sulfate + 0.356 * hydroxyproline + 0.091 * bilirubin - 0.082 * 2-hydroxydadipic acid + 0.015 * methionine sulfoxide - 0.066 * hexadecanedioic acid - 0.154 * quinolinic acid;

or P = -14.392 + 0.799 * pipecolic acid + 0.792 * NAAG + 0.383 * ureidopropionic acid - 0.403 * formiminoglutamic acid - 0.352 * γ-CEHC + 0.428 * γ-glu-phe + 0.388 * 3-OH butyrylcarnitine + 0.121 * estrone sulfate + 0.353 * hydroxyproline + 0.095 * bilirubin - 0.153 * 2-hydroxydadipic acid + 0.009 * methionine sulfoxide - 0.025 * hexadecanedioic acid;

or P = -14.5 + 0.794 * pipecolic acid + 0.806 * NAAG + 0.376 * ureidopropionic acid-0.403 * formiminoglutamic acid - 0.352 * γ-CEHC + 0.429 * γ-glu-phe + 0.378 * 3-OH butyrylcarnitine + 0.114 * estrone sulfate + 0.362 * hydroxyproline + 0.095 * bilirubin - 0.16 * 2-hydroxydadipic acid + 0.007 * methionine sulfoxide;

or P = -14.476 + 0.795 * pipecolic acid + 0.807 * NAAG + 0.376 * ureidopropionic acid - 0.404 * formiminoglutamic acid - 0.353 * γ-CEHC + 0.43 * γ-glu-phe + 0.375 * 3-OH butyrylcarnitine + 0.115 * estrone sulfate + 0.364 * hydroxyproline + 0.096 * bilirubin - 0.157 * 2-hydroxydadipic acid;

or P = -14.315 + 0.777 * pipecolic acid + 0.77 * NAAG + 0.342 * ureidopropionic acid - 0.411 * formiminoglutamic acid - 0.352 * γ-CEHC + 0.432 * γ-glu-phe + 0.325 * 3-OH butyrylcarnitine + 0.109 * estrone sulfate + 0.357 * hydroxyproline + 0.1 * bilirubin;

or P = -13.898 + 0.825 * pipecolic acid + 0.744 * NAAG + 0.322 * ureidopropionic acid + -0.391 * formiminoglutamic acid + -0.269 * γ-CEHC + 0.424 * γ-glu-phe + 0.345 * 3-OH butyrylcarnitine + 0.162 * estrone sulfate + 0.276 * hydroxyproline;

or P = -12.473 + 0.857 * pipecolic acid + 0.781 * NAAG + 0.415 * ureidopropionic acid - 0.405 * formiminoglutamic acid - 0.257 * γ-CEHC + 0.42 * γ-glu-phe + 0.343 * 3-OH butyrylcarnitine + 0.14 * estrone sulfate;

or P = -13.147 + 0.947 * pipecolic acid + 0.832 * NAAG + 0.435 * ureidopropionic acid - 0.407 * formiminoglutamic acid - 0.292 * γ-CEHC + 0.428 * γ-glu-phe + 0.334 * 3-OH butyrylcarnitine;

or P = -12.838 + 0.839 * pipecolic acid + 1.125 * NAAG + 0.49 * ureidopropionic acid - 0.39 * formiminoglutamic acid - 0.263 * γ-CEHC + 0.39 * γ-glu-phe;

or P=-10.874 + 0.685 * pipecolic acid + 1.105 * NAAG + 0.51 * ureidopropionic acid-0.214 * formiminoglutamic acid - 0.22 * γ-CEHC;

or P = -11.911 + 0.81 * pipecolic acid + 0.992 * NAAG + 0.559 * ureidopropionic acid - 0.2 * formiminoglutamic acid;

or

$$\overline{P} = -11.25 + 0.73 * \text{pipecolic acid} + 0.917 * \text{NAAG} + 0.435 * \text{ureidopropionic acid},$$

wherein P represents the prediction value for predicting the mortality risk of the ACLF patient within 360 days.

Further, the logistic regression equation is:

P = -11.911 + 0.81 * pipecolic acid + 0.992 * NAAG + 0.559 * ureidopropionic acid - 0.2 * formiminoglutamic acid; or P = -11.25 + 0.73 * pipecolic acid + 0.917 * NAAG + 0.435 * ureidopropionic acid.

Further, P represents a prediction value for predicting a mortality risk of the ACLF patient within 90 days.

Further, when the logistic regression equation is:

P = -11.911 + 0.81 * pipecolic acid + 0.992 * NAAG + 0.559 * ureidopropionic acid - 0.2 * formiminoglutamic acid, when P is greater than 0.331, the mortality risk of the ACLF patient within 90 days is predicted to be high; and when P is less than 0.331, the mortality risk of the ACLF patient within 90 days is predicted to be low;

and

when the logistic regression equation is:

$$P = -11.25 + 0.73 * \text{pipecolic acid} + 0.917 * \text{NAAG} + 0.435 * \text{ureidopropionic acid,}$$

when P is greater than 0.320, the mortality risk of the ACLF patient within 90 days is predicted to be high; and when P is less than 0.320, the mortality risk of the ACLF patient within 90 days is predicted to be low.

Further, the logistic regression equation further comprises one or more of clinical indicators: age, international normalized ratio (INR), creatine, Sodium (Na), albumin (ALB), neutrophil/lymphocyte count ratio (N/L ratio), overt hepatic encephalopathy (overt HE), and total bilirubin (TB).

Further, the system further comprises a data storage module, a data input interface, and a data output interface, wherein the data storage module is configured to store the detected value of the biomarker; the data input interface is configured to input the detected value of the biomarker; and the data output interface is configured to output a prediction result.

In yet another aspect, the present application provides a system for predicting progression to acute-on-chronic liver failure (ACLF) of a non-ACLF patient, comprising a data analysis module, wherein the data analysis module is configured to analyze a detected value of a biomarker, and the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

Further, the detected value of the biomarker refers to detection of the biomarker in a blood, serum, plasma, or whole blood sample from the non-ACLF patient, and is used for detecting a presence or absence, relative abundance, or concentration of the biomarker in the sample.

Further, the predicting progression to ACLF of the non-ACLF patient refers to predicting progression to ACLF of the non-ACLF patient within a short term.

The term "short term" herein may refer to the progression risk to ACLF of non-ACLF patients within 180 days, 90 days, or 28 days; of course, it may be within 1 year, 11 months, 10 months, 9 months, 8 months, 7 months, 6 months, 5 months, 4 months, 3 months, 2 months, or 1 month, etc.; or within 10 weeks, 9 weeks, 8 weeks, 7 weeks, 6 weeks, 5 weeks, 4 weeks, 3 weeks, 2 weeks, or 1 weeks, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

Further, the biomarker comprises ureidopropionic acid and γ-CEHC; or, the biomarker comprises ureidopropionic acid, γ-CEHC, and thyroxine; or, the biomarker comprises ureidopropionic acid, γ-CEHC, and pipecolic acid; or, the biomarker comprises ureidopropionic acid, γ-CEHC, thyroxine, and pipecolic acid; or, the biomarker comprises ureidopropionic acid, γ-CEHC, thyroxine, pipecolic acid, and 3-OH butyrylcarnitine; or, the biomarker comprises ureidopropionic acid, γ-CEHC, thyroxine, pipecolic acid, 3-OH butyrylcarnitine, and 2-hydroxydadipic acid; or, the biomarker comprises ureidopropionic acid, γ-CEHC, thyroxine, pipecolic acid, 3-OH butyrylcarnitine, 2-hydroxydadipic acid, and estrone sulfate; or, the biomarker comprises ureidopropionic acid, γ-CEHC, thyroxine, pipecolic acid, 3-OH butyrylcarnitine, 2-hydroxydadipic acid, estrone sulfate, and hydroxyproline; or, the biomarker comprises ureidopropionic acid, γ-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glu-phe; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and NAAG.

Further, the data analysis module calculates a prediction value for predicting progression to ACLF of the non-ACLF patient by substituting the detected value of the biomarker into a regression equation, thereby assessing a progression risk to ACLF of the non-ACLF patient.

Further, the regression equation is a logistic regression equation.

Further, the logistic regression equation is obtained by constructing and training with detected values of biomarkers from known samples.

Further, the training is to construct a training set and train the training set using a training method to obtain the logistic regression equation; and the training set comprises serum samples from non-ACLF patients.

Further, the training method comprises: dividing the training set into a pre-ACLF group and a non-ACLF group according to an ACLF progression status within a short term; detecting biomarker levels in the pre-ACLF group and the non-ACLF group respectively to obtain the detected values; performing logistic regression according to the detected values of the biomarkers to analyze relationships between the detected values in the pre-ACLF group and the non-ACLF group; and constructing the logistic regression equation.

The training set of the present application may comprise serum samples collected from non-ACLF patients under any circumstances. That is, the training set may comprise serum samples collected from non-ACLF patients under different time points, different geographical regions, different sizes, different genders, and different ages.

Further, the short term comprises any one or more of within 180 days, within 90 days, within 80 days, within 70 days, within 60 days, within 28 days, within 3 months, within 4 months, within 5 months, within half a year, within 7 months, within 8 months, within 9 months, within 10 weeks, within 9 weeks, within 8 weeks, within 7 weeks, within 6 weeks, within 5 weeks, within 4 weeks, within 3 weeks, within 2 weeks, and within 1 week.

Further, the data analysis module calculates a prediction value for predicting progression to ACLF of the non-ACLF patient within 180 days by substituting the detected value of the biomarker into the logistic regression equation, thereby assessing a progression risk to ACLF of the non-ACLF patient within 180 days.

Further, the logistic regression equation is:

P = -3.271 + 0.716 * ureidopropionic acid - 0.512 * γ-CEHC - 1.1 * thyroxine + 0.357 * pipecolic acid + 0.134 * 3-OH butyrylcarnitine - 1.225 * 2-hydroxydadipic acid + 0.029 * estrone sulfate + 0.168 * hydroxyproline - 0.04 * quinolinic acid + 0.078 * γ-glu-phe - 0.014 * formiminoglutamic acid - 0.03 * bilirubin + 0.519 * hexadecanedioic acid - 0.139 * methionine sulfoxide + 0.653 * NAAG;

or P = -1.518 + 0.724 * ureidopropionic acid - 0.494 * γ-CEHC - 1.216 * thyroxine + 0.409 * pipecolic acid + 0.157 * 3-OH butyrylcarnitine - 1.131 * 2-hydroxydadipic acid + 0.051 * estrone sulfate + 0.202 * hydroxyproline - 0.024 * quinolinic acid + 0.11 * γ-glu-phe - 0.007 * formiminoglutamic acid + 0.009 * bilirubin + 0.479 * hexadecanedioic acid - 0.17 * methionine sulfoxide;

or P = -2.436 + 0.726 * ureidopropionic acid + -0.498 * γ-CEHC - 1.18 * thyroxine + 0.382 * pipecolic acid + 0.195 * 3-OH butyrylcarnitine - 1.121 * 2-hydroxydadipic acid + 0.044 * estrone sulfate + 0.199 * hydroxyproline - 0.009 * quinolinic acid + 0.046 * γ-glu-phe - 0.014 * formiminoglutamic acid - 0.012 * bilirubin + 0.446 * hexadecanedioic acid;

or P = -2.837 + 0.819 * ureidopropionic acid - 0.514 * γ-CEHC - 1.019 * thyroxine + 0.399 * pipecolic acid + 0.362 * 3-OH butyrylcarnitine - 0.932 * 2-hydroxydadipic acid + 0.173 * estrone sulfate + 0.235 * hydroxyproline - 0.08 * quinolinic acid + 0.074 * γ-glu-phe + 0.001 * formiminoglutamic acid - 0.02 * bilirubin;

or P = -2.894 + 0.816 * ureidopropionic acid - 0.512 * γ-CEHC - 1.016 * thyroxine + 0.395 * pipecolic acid + 0.362 * 3-OH butyrylcarnitine - 0.93 * 2-hydroxydadipic acid + 0.166 * estrone sulfate + 0.238 * hydroxyproline - 0.076 * quinolinic acid + 0.071 * γ-glu-phe + 0.001 * formiminoglutamic acid;

or P = -2.899 + 0.816 * ureidopropionic acid - 0.512 * γ-CEHC - 1.015 * thyroxine + 0.396 * pipecolic acid + 0.362 * 3-OH butyrylcarnitine - 0.929 * 2-hydroxydadipic acid + 0.166 * estrone sulfate + 0.238 * hydroxyproline - 0.076 * quinolinic acid + 0.071 * γ-glu-phe;

or P = -2.647 + 0.828 * ureidopropionic acid - 0.512 * γ-CEHC - 1.039 * thyroxine + 0.388 * pipecolic acid + 0.347 * 3-OH butyrylcarnitine - 0.926 * 2-hydroxydadipic acid + 0.163 * estrone sulfate + 0.241 * hydroxyproline - 0.047 * quinolinic acid;

or P = -2.647 + 0.816 * ureidopropionic acid - 0.513 * γ-CEHC - 1.049 * thyroxine + 0.393 * pipecolic acid + 0.34 * 3-OH butyrylcarnitine - 0.939 * 2-hydroxydadipic acid + 0.164 * estrone sulfate + 0.23 * hydroxyproline;

or P = -0.998 + 0.856 * ureidopropionic acid - 0.502 * γ-CEHC - 1.104 * thyroxine + 0.408 * pipecolic acid + 0.325 * 3-OH butyrylcarnitine + -0.916 * 2-hydroxydadipic acid + 0.154 * estrone sulfate;

or P = -1.935 + 0.919 * ureidopropionic acid - 0.537 * γ-CEHC - 1.018 * thyroxine + 0.464 * pipecolic acid + 0.296 * 3-OH butyrylcarnitine - 0.867 * 2-hydroxydadipic acid;

or P = -2.077 + 0.745 * ureidopropionic acid - 0.539 * γ-CEHC - 0.806 * thyroxine + 0.191 * pipecolic acid + 0.088 * 3-OH butyrylcarnitine;

or P = -1.878 + 0.77 * ureidopropionic acid - 0.54 * γ-CEHC - 0.827 * thyroxine + 0.188 * pipecolic acid;

or

$$P = -0.983 + 0.845 * \text{ureidopropionic acid} - 0.565 * \gamma\text{-CEHC} - 0.835 * \text{thyroxine};$$

or

$$P = -4.744 + 1.015 * \text{ureidopropionic acid} - 0.584 * \gamma\text{-CEHC} + 0.122 * \text{pipecolic acid};$$

or

$$P = -4.803 + 0.930 * \text{ureidopropionic acid} - 0.563 * \gamma\text{-CEHC},$$

wherein P represents a prediction value for predicting progression to ACLF of the non-ACLF patient within 180 days.

Further, the logistic regression equation is:

$$P = -0.983 + 0.845 * \text{ureidopropionic acid} - 0.565 * \gamma\text{-CEHC} - 0.835 * \text{thyroxine};$$

or

$$P = -4.803 + 0.930 * \text{ureidopropionic acid} - 0.563 * \gamma\text{-CEHC}.$$

Further, P represents a prediction value for predicting progression to ACLF of the non-ACLF patient.

Further, when the logistic regression equation is: P = -0.983 + 0.845 * ureidopropionic acid - 0.565 * γ-CEHC - 0.835 * thyroxine, when P is greater than 0.150, a progression risk to ACLF of the non-ACLF patient within 28 days is predicted to be high; and when P is less than 0.150, a progression risk to ACLF of the non-ACLF patient within 28 days is predicted to be low; and

when the logistic regression equation is: P = -4.803 + 0.930 * ureidopropionic acid - 0.563 * $\gamma$-CEHC, when P is greater than 0.123, a progression risk to ACLF of the non-ACLF patient within 28 days is predicted to be high; and when P is less than 0.123, a progression risk to ACLF of the non-ACLF patient within 28 days is predicted to be low.

Further, the logistic regression equation further comprises one or more of clinical indicators: total bilirubin (TB), international normalized ratio (INR), and Cirrhosis.

Further, the system further comprises a data storage module, a data input interface, and a data output interface, wherein the data storage module is configured to store the detected value of the biomarker; the data input interface is configured to input the detected value of the biomarker; and the data output interface is configured to output a prediction result.

In yet another aspect, the present application provides a method for predicting a mortality risk of an acute-on-chronic liver failure (ACLF) patient, used for predicting the mortality risk of the ACLF patient by analyzing a detected value of a biomarker, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, $\gamma$-carboxyethylhydroxychroman ($\gamma$-CEHC), thyroxine, formiminoglutamic acid, $\gamma$-glutamylphenylalanine ($\gamma$-glu-phe), and bilirubin.

Further, the detected value of the biomarker refers to detection of the biomarker in a blood, serum, plasma, or whole blood sample from the ACLF patient, and is used for detecting a presence or absence, relative abundance, or concentration of the biomarker in the sample.

Further, the biomarker comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and $\gamma$-CEHC; or, the biomarker comprises pipecolic acid, NAAG, ureidopropionic acid, formiminoglutamic acid, $\gamma$-CEHC, and $\gamma$-glu-phe; or, the biomarker comprises pipecolic acid, NAAG, ureidopropionic acid, formiminoglutamic acid, $\gamma$-CEHC, $\gamma$-glu-phe, and 3-OH butyrylcarnitine; or, the biomarker comprises pipecolic acid, NAAG, ureidopropionic acid, formiminoglutamic acid, $\gamma$-CEHC, $\gamma$-glu-phe, 3-OH butyrylcarnitine, and estrone sulfate; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, and hydroxyproline; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, and bilirubin; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, $\gamma$-CEHC, $\gamma$-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

Further, the method comprises calculating a prediction value for predicting a mortality risk of the ACLF patient by substituting the detected value of the biomarker into a regression equation, thereby assessing the mortality risk of the ACLF patient.

Further, the predicting the mortality risk of the ACLF patient refers to predicting a mortality risk of the ACLF patient within a short term.

The term "short term" herein may refer to the mortality risk of ACLF patients within 90 days, 180 days, or 360 days; of course, it may be within less than 90 days, e.g., within 80 days, 70 days, 60 days, or shorter; or within 1 year, 11 months, 10 months, 9 months, 8 months, 7 months, 6 months, 5 months, 4 months, 3 months, 2 months, or 1 month, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

Further, the regression equation is a logistic regression equation.

Further, the logistic regression equation is obtained by constructing and training with detected values of biomarkers from known samples.

Further, the training is to construct a training set and train the training set using a training method to obtain the logistic regression equation; and the training set comprises serum samples from ACLF patients.

Further, the training method comprises: dividing the training set into a survivor group and a non-survivor group according to a survival status within a short term; detecting biomarker levels in the survivor group and the non-survivor group respectively to obtain the detected values; performing logistic regression according to the detected values of the biomarkers to analyze relationships between the detected values in the survivor group and the non-survivor group; and constructing the logistic regression equation.

Further, the short term comprises any one or more of within 90 days, within 180 days, within 360 days, within 80 days, within 70 days, within 60 days, within three months, within half a year, within one year, within two months, and within one month.

Further, the data analysis module calculates a prediction value for predicting a mortality risk of the ACLF patient within 360 days by substituting the detected value of the biomarker into the logistic regression equation, thereby assessing the mortality risk of the ACLF patient within 360 days.

In yet another aspect, the present application provides a method for predicting progression to acute-on-chronic liver failure (ACLF) of a non-ACLF patient, used for predicting a short-term progression risk to ACLF of the non-ACLF patient by analyzing a detected value of a biomarker, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, $\gamma$-carboxyethylhydroxychroman ($\gamma$-CEHC), thyroxine, formiminoglutamic acid, $\gamma$-glutamylphenylalanine ($\gamma$-glu-phe), and bilirubin.

Further, the detected value of the biomarker refers to detection of the biomarker in a blood, serum, plasma, or whole blood sample from the non-ACLF patient, and is used for detecting a presence or absence, relative abundance, or concentration of the biomarker in the sample.

Further, the predicting progression to ACLF of the non-ACLF patient refers to predicting progression to ACLF of the non-ACLF patient within a short term.

The term "short term" herein may refer to the progression risk to ACLF of non-ACLF patients within 180 days, 90 days, or 28 days; of course, it may be within 1 year, 11 months, 10 months, 9 months, 8 months, 7 months, 6 months, 5 months, 4 months, 3 months, 2 months, or 1 month, etc.; or within 10 weeks, 9 weeks, 8 weeks, 7 weeks, 6 weeks, 5 weeks, 4 weeks, 3 weeks, 2 weeks, or 1 weeks, etc.

The short term herein cannot be predetermined and is a time point summarized based on current clinical experience. As time goes by or with improvements in other therapeutic methods, this time point or the length of time is likely to change. It may also be changed according to the patient's length of hospitalization.

Further, the biomarker comprises ureidopropionic acid and $\gamma$-CEHC; or, the biomarker comprises ureidopropionic acid, $\gamma$-CEHC, and thyroxine; or, the biomarker comprises ureidopropionic acid, $\gamma$-CEHC, and pipecolic acid; or, the biomarker comprises ureidopropionic acid, $\gamma$-CEHC, thyroxine, and pipecolic acid; or, the biomarker comprises ureidopropionic acid, $\gamma$-CEHC, thyroxine, pipecolic acid, and 3-OH butyrylcarnitine; or, the biomarker comprises ureidopropionic acid, $\gamma$-CEHC, thyroxine, pipecolic acid, 3-OH butyrylcarnitine, and 2-hydroxydadipic acid; or, the biomarker comprises ureidopropionic acid, $\gamma$-CEHC, thyroxine, pipecolic acid, 3-OH butyrylcarnitine, 2-hydroxydadipic acid, and estrone sulfate; or, the biomarker comprises ureidopropionic acid, $\gamma$-CEHC, thyroxine, pipecolic acid, 3-OH butyrylcarnitine, 2-hydroxydadipic acid, estrone sulfate, and hydroxyproline; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, and $\gamma$-glu-phe; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, and formiminoglutamic acid; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, formiminoglutamic acid, and bilirubin; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, formiminoglutamic acid, bilirubin, and hexadecanedioic acid; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, formiminoglutamic acid, bilirubin, hexadecanedioic acid, and methionine sulfoxide; or, the biomarker comprises ureidopropionic acid, $\gamma$-CEHC, thyroxine, pipecolic acid, 3-OH butyrylcarnitine, 2-hydroxydadipic acid, estrone sulfate, hydroxyproline, quinolinic acid, $\gamma$-glu-phe, formiminoglutamic acid, bilirubin, hexadecanedioic acid, methionine sulfoxide, and NAAG.

Further, the data analysis module calculates a prediction value for predicting progression to ACLF of the non-ACLF patient by substituting the detected value of the biomarker into a regression equation, thereby assessing a progression risk to ACLF of the non-ACLF patient.

Further, the regression equation is a logistic regression equation.

Further, the logistic regression equation is obtained by constructing and training with detected values of biomarkers from known samples.

Further, the training is to construct a training set and train the training set using a training method to obtain the logistic regression equation; and the training set comprises serum samples from non-ACLF patients.

Further, the training method comprises: dividing the training set into a pre-ACLF group and a non-ACLF group according to an ACLF progression status within a short term; detecting biomarker levels in the pre-ACLF group and the non-ACLF group respectively to obtain the detected values; performing logistic regression according to the detected values of the biomarkers to analyze relationships between the detected values in the pre-ACLF group and the non-ACLF group; and constructing the logistic regression equation.

Further, the short term comprises any one or more of within 180 days, within 90 days, within 80 days, within 70 days, within 60 days, within 28 days, within 3 months, within 4 months, within 5 months, within half a year, within 7 months, within 8 months, within 9 months, within 10 weeks, within 9 weeks, within 8 weeks, within 7 weeks, within 6 weeks, within 5 weeks, within 4 weeks, within 3 weeks, within 2 weeks, and within 1 week.

Further, the data analysis module calculates a prediction value for predicting progression to ACLF of the non-ACLF patient within 180 days by substituting the detected value of the biomarker into the logistic regression equation, thereby assessing a progression risk to ACLF of the non-ACLF patient within 180 days.

The ACLF prognosis prediction system provided by the present application has the following beneficial effects:

1. 15 new metabolic biomarkers capable of early prediction of the mortality risk of ACLF patients and the progression risk to ACLF of non-ACLF patients are screened, particularly for predicting the short-term mortality risk of ACLF patients and the short-term progression risk to ACLF of non-ACLF patients;

2. 4 biomarkers are screened to construct a short-term mortality risk prediction model for ACLF patients, demonstrating superior predictive performance with AUC values up to 0.79. In further combination with clinical indicators such as age, INR, CR, Na, ALB, N/L ratio, overt HE, and TB, AUC values can be further increased, substantially reaching above 0.8;

3. 3 biomarkers are screened to construct a short-term ACLF progression risk prediction model for non-ACLF patients, demonstrating superior predictive performance with AUC values up to 0.80. In further combination with clinical indicators such as TB, INR, and Cirrhosis, AUC values can be further increased, substantially reaching above 0.84;

4. Convenient and efficient prediction is achieved, seizing the golden window for intervention for high-risk groups, reducing economic burdens for low-risk patients, and meeting clinical needs.

BRIEF DESCRIPTION OF THE DRAWINGS

Fig. 1 shows a flowchart of screening, model construction, and validation of ACLF-related metabolomics biomarkers in Example 1;

Fig. 2 shows a schematic diagram of significant differential changes in metabolites between an ACLF group and a non-ACLF group in Example 1;

Fig. 3 shows a schematic diagram of significantly affected metabolite categories in Example 1;

Fig. 4 shows a schematic diagram of changes in hepatic metabolic pathways of ACLF patients in Example 1;

Fig. 5 shows a schematic diagram of significant differential changes in metabolites between a non-survivor group and a survivor group of ACLF patients in Example 2;

Fig. 6 shows a schematic diagram of random forest ranking results for metabolites distinguishing a non-survivor group from a survivor group of ACLF patients in Example 2;

Fig. 7 shows a schematic diagram of significant differential changes in metabolites between a pre-ACLF group and a non-ACLF group in Example 3;

Fig. 8 shows a schematic diagram of random forest ranking results for metabolites distinguishing a pre-ACLF group from a non-ACLF group in Example 3;

Fig. 9 shows an ROC curve obtained from detection results of 4 metabolites in a discovery set in Example 5;

Fig. 10 shows an ROC curve obtained from detection results of 4 metabolites in a validation set in Example 5;

Fig. 11 shows an ROC curve obtained from detection results of 3 metabolites in a discovery set in Example 6;

Fig. 12 shows an ROC curve obtained from detection results of 3 metabolites in a validation set in Example 6;

Fig. 13 shows a comparison of assessment performance of a 4-metabolite model for predicting 90-day mortality of ACLF patients in a discovery set in Example 7;

Fig. 14 shows a comparison of assessment performance of a 4-metabolite model for predicting 90-day mortality of ACLF patients in a validation set in Example 7;

Fig. 15 shows a comparison of assessment performance of a 3-metabolite model for predicting 28-day progression risk to ACLF of non-ACLF patients in a discovery set in Example 7;

Fig. 16 shows a comparison of assessment performance of a 3-metabolite model for predicting 28-day progression risk to ACLF of non-ACLF patients in a validation set in Example 7.

DETAILED DESCRIPTION OF THE INVENTION

(1) Diagnosis or Detection

The terms "diagnosis" or "detection", and "prediction" herein refer to detecting or assaying biomarkers in a sample, or the levels of target biomarkers, e.g., an absolute level or relative level. Then, the presence or absence or levels of the target biomarkers indicate whether the sample-providing individual may have or develop a disease, or the likelihood of having a disease. The diagnosis and detection herein are interchangeable in meaning. The results of such detections or diagnoses serve as intermediate findings rather than direct results for developing a disease. If direct results are obtained, confirmation of a disease requires pathological, anatomical, or other supplementary methods. For example, the present application provides multiple new biomarkers correlated with ACLF patients, or mortality or survival of ACLF patients. Variations in the levels of these biomarkers have a direct correlative relationship with developing ACLF, or developing ACLF and mortality.

(2) Association of Markers or Biomarkers with ACLF

The terms "markers" and "biomarkers" are synonymous in the present application. The association herein refers to a direct correlation between the presence or level variation of a biomarker in samples and a particular disease or disease progression. For example, a relative increase or decrease in levels represents a higher probability of developing the disease compared to healthy individuals, or represents that the disease progression manifests as increased severity or transition from one stage to another. For example, a single biomarker or a biomarker combination of the multiple new biomarkers of the present application can be used for predicting progression to ACLF of non-ACLF patients, or mortality or non-mortality of ACLF patients.

The concurrent presence or relative level change of multiple different markers in a sample represents a higher probability of developing the disease compared to healthy individuals. That is, in types of biomarkers, some biomarkers have a strong correlation with developing a disease, some biomarkers have a weak correlation with developing a disease, or some biomarkers are irrelevant to a specific disease. One or more of these biomarkers with strong correlation can serve as biomarkers for disease diagnosis, and these biomarkers with weak correlation can be combined with these biomarkers with strong correlation for diagnosis of a disease, improving the accuracy of detection results. The term "disease" herein may refer to disease occurrence or progression, for example, transition from a good stage of a disease to an increased exacerbation or severity stage, even terminal mortality.

Regarding the multiple biomarkers in serum discovered in the present application, all can be used for distinguishing non-ACLF patients from ACLF patients, or predicting progression from non-ACLF to ACLF; or used for diagnosis of prediction of the probability or likelihood of progression or mortality of ACLF patients. The biomarkers herein may be individually used for direct detection or diagnosis as individual biomarkers. Selection of such biomarkers indicates that the relative level changes of the biomarkers have a strong correlation with progression to ACLF of non-ACLF patients, or progression or mortality of ACLF patients. Of course, it should be understood that one or more biomarkers associated with progression or mortality of ACLF patients or progression to ACLF of non-ACLF patients may be selected for concurrent detection. It should be normally understood that in some embodiments, the selection of biomarkers with strong correlation for detection or diagnosis can achieve the accuracy at a certain standard, for example, 60%, 65%, 70%, 80%, 85%, 90%, or 95% accuracy. It can indicate that through these biomarkers, intermediate values for diagnosis of a disease can be obtained, but it does not represent direct confirmation of developing the disease.

Of course, differential biomarkers with greater receiver operating characteristic (ROC) values may be selected for diagnosis. The term "strong" or "weak" is typically confirmed through some computational algorithms, such as contribution rates or weight analysis of analyses on ACLF, or mortality probability of ACLF patients. Such computational methods may include significance analysis (p-value or FDR value) and fold change; and multivariate statistical analysis mainly including principal component analysis (PCA), partial least squares discriminant analysis (PLS-DA), and orthogonal partial least squares discriminant analysis (OPLS-DA).

(3) ACLF Patients

Acute-on-chronic liver failure (ACLF) is a rapidly progressive disease with rapid clinical deterioration and high mortality, manifesting as acute hepatic dysfunction in patients with chronic liver disease or cirrhosis and characterized by organ failures and high short-term mortality. It is present in more than 25% of patients hospitalized with cirrhosis. ACLF is known to be associated with precipitating insults such as alcohol or viral infection, extrahepatic organ failure, and systemic inflammation. Its pathophysiology is associated with sustained inflammation, immune dysregulation involving initial widespread immune activation, a state of systemic inflammatory response syndrome, and subsequently, sepsis due to immunosuppression.

The term "chronic liver disease" primarily refers to that a patient is diseased in the liver (relative to healthy states), for example, patients with hepatitis, patients with alcoholic hepatitis, post-liver transplantation patients, patients with cirrhosis, or other patients diseased in the liver. Chronic liver disease represents an umbrella term encompassing a spectrum of hepatic disorders characterized by diverse etiologies, heterogeneous clinical manifestations, varied therapeutic approaches, and distinct prognostic outcomes. The common characteristic of chronic liver disease involves variable degrees of hepatic necrosis and inflammatory response, which may progress to hepatic fibrosis and ultimately advance to cirrhosis. The extent of chronic liver injury can be assessed through liver biopsy. Cirrhosis is also a clinically common chronic progressive liver disease characterized by diffuse hepatic injury resulting from persistent or recurrent exposure to one or more etiological factors. The common etiological factors include hepatitis, and may also be related to alcoholic cirrhosis and schistosomiasis cirrhosis.

Diagnosis of chronic liver disease requires meeting at least one of the following criteria:

1) Liver enzyme abnormalities beyond a specified duration (e.g., above 1-3 months, above 6 months, etc.), where the liver enzymes include any one or more of alanine transaminase (ALT), aspartate transaminase (AST), alkaline phosphatase, or $\gamma$-glutamyl transferase. Of course, it should be understood that there are also other abnormalities associated with liver enzymes. The abnormality refers to values exceeding the upper limit of normal. The abnormality may refer to recurrent abnormality or high-frequency abnormality within certain time periods, e.g., within 3 months, within 2 months, or within 80% of the time period.
2) Hepatic fibrosis indicated by liver ultrasound elastography (liver stiffness measurement, including but not limited to: Fibroscan or Fibrotouch);
3) Hepatic fibrosis discovered by liver biopsy;
4) Hepatic fibrosis indicated by non-invasive blood indicator detection (including but not limited to: FIB-4);
5) Cirrhosis indicated clinically or by imageology or gastrointestinal endoscopy or hepatic histopathology.

(4) Non-ACLF Patients

The non-ACLF patients herein refer to patients without ACLF, or patients exhibiting clinical manifestations of liver disease without ACLF, or patients having chronic liver disease without progression to liver failure. That is, within the cohort of chronic liver disease patients, a part of the patients may progress to ACLF, while the other part may not progress to ACLF. Thus, the occurrence or progression from non-ACLF to ACLF may be within any time period within 360 days.

DETAILED DESCRIPTION OF THE EMBODIMENTS

The present application will be further described in detail below in conjunction with the drawings and embodiments. It needs to be noted that the embodiments described below are intended to facilitate the understanding of the present application without any limiting effect. The reagents used in the examples are all known commercially available products.

Example 1. Preliminary Screening of Biomarkers Using Metabolomics

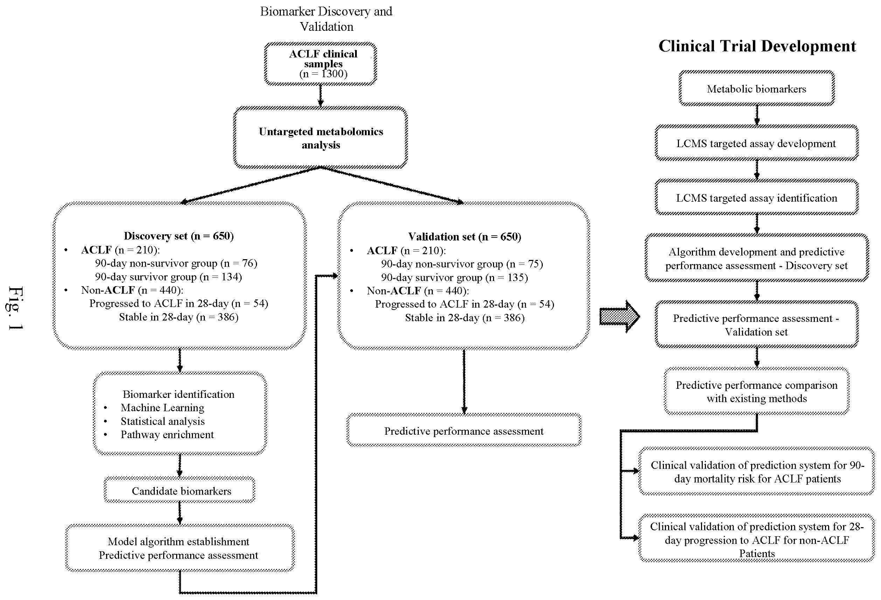

In this example, a research consortium including 14 hospitals was established and collected 1,300 ACLF-related samples within three years for metabolomics biomarker screening, predictive model construction, and validation. The detailed flow is shown in Fig. 1.

Key clinical parameters on admission for ACLF and non-ACLF patients are presented in Table 1. It can be seen that Hepatitis B virus (HBV) constitutes the primary etiology and accounts for around 75% of cases, and the remaining cases involve alcohol and other causes; for ACLF patients, the 90-day liver transplant-free mortality is significantly elevated, the organ failure rate is increased, particularly for hepatic failure and coagulation failure, TB, ALT, AST, CR, INR, and C-reactive protein (CRP) are elevated, and the scores of MELD-Na and CLIF-C ACLF are elevated ($p < 0.05$).

Table 1. Baseline Clinical Parameters of ACLF and Non-ACLF Groups

| Baseline characteristics | ACLF (n=420) | Non-ACLF (n=880) | P-value |
|---|---|---|---|
| Age, median (interquartile range (IQR)) | 49.00 (41.00,57.00) | 49.00 (40.00,58.00) | 0.911 |
| Male, n (%) | 337 (80.2) | 629 (71.5) | 0.001 |
| Cirrhosis, n (%) | 371 (88.3) | 591 (67.2) | <0.001 |
| Etiology, n (%) | | | 0.031 |
| HBV | 335 (79.8) | 644 (73.2) | |
| Alcohol-associated liver disease | 42 (10.0) | 108 (12.3) | |

(continued)

| Baseline characteristics | ACLF (n=420) | Non-ACLF (n=880) | P-value |
|---|---|---|---|
| Other | 43 (10.2) | 128 (14.5) | |
| Organ failure, n (%) | | | |
| Hepatic failure | 236 (56.2) | 52 (5.9) | <0.001 |
| Coagulation failure | 283 (67.4) | 6 (0.7) | <0.001 |
| Renal failure | 43 (10.4) | 12 (1.4) | <0.001 |
| Cerebral failure | 28 (6.7) | 2 (0.2) | <0.001 |
| Circulatorv failure | 8 (1.9) | 3 (0.3) | 0.007 |
| Respiratory failure | 0 (0.0) | 5 (0.6) | 0.182 |
| Laboratory Tests, median (IQR) | | | |
| Total bilirubin (mg/dL) | 19.31[9.92, 27.20] | 2.91[1.47, 7.89] | <0.001 |
| INR | 2.91[1.70, 2.68] | 1.34[1.18, 1.59] | <0.001 |
| Creatine (mg/dL) | 0.86[0.69, 1.15] | 0.76[0.63, 0.89] | <0.001 |
| Blood urea nitrogen (mmol/L) | 4.66[3.40, 7.76] | 4.40[3.40, 5.95] | 0.013 |
| ALT (U/L) | 129.00[43.92, 438.78] | 78.05[32.00, 352.28] | 0.002 |
| AST (U/L) | 157.00[70.70, 341.50] | 91.00[46.00, 249.35] | <0.001 |
| ALB (g/L) | 29.90[26.30, 33.40] | 32.30[28.42, 36.57] | <0.001 |
| Prealbumin (g/L) | 38.45[21.00, 63.00] | 71.30[45.33, 104.97] | <0.001 |
| White blood cell count (*$10^9$/L) | 6.36[4.44, 9.00] | 4.72[3.38, 6.37] | <0.001 |
| N/L ratio | 3.84[2.39, 6.53] | 2.27[1.46, 3.51] | <0.001 |
| CRP (mg/L) | 11.70[6.48, 19.00] | 6.29[2.88, 13.30] | <0.001 |
| Procalcitonin (ng/mL) | 0.55[0.25, 1.05] | 0.19[0.10, 0.46] | <0.001 |
| Platelet count (*$10^9$/L) | 89.00[55.00, 128.50] | 97.00[61.00, 153.00] | 0.001 |
| Hemoglobin (g/L) | 116.00[96.75, 131.00] | 118.00[100.00, 136.00] | 0.023 |
| Serum sodium (mmol/L) | 137.00[133.50, 139.30] | 139.00[136.10, 141.00] | <0.001 |
| Score, median (IQR) | | | |
| MELD-Na | 27.00[24.00, 31.00] | 15.00[11.00, 20.00] | <0.001 |
| CLIF-C ACLF | 40.99[36.13, 45.49] | 30.75[26.59, 35.61] | <0.001 |
| Liver transplant-free mortality, n (%) | | | |
| 28-day | 87 (20.7) | 19 (2.2) | <0.001 |
| 90-day | 151 (36.0) | 44 (5.1) | <0.001 |
| 365-day | 184 (44.4) | 90 (10.4) | <0.001 |

In this example, 1,300 samples (including 420 ACLF samples and 880 non-ACLF samples) were randomly divided into two equal halves as a discovery set (650 samples including 210 ACLF samples and 440 non-ACLF samples) and a validation set (650 samples including 210 ACLF samples and 440 non-ACLF samples). Baseline serum samples on admission from the discovery set were analyzed using untargeted metabolomics via HPLC-MS/MS.

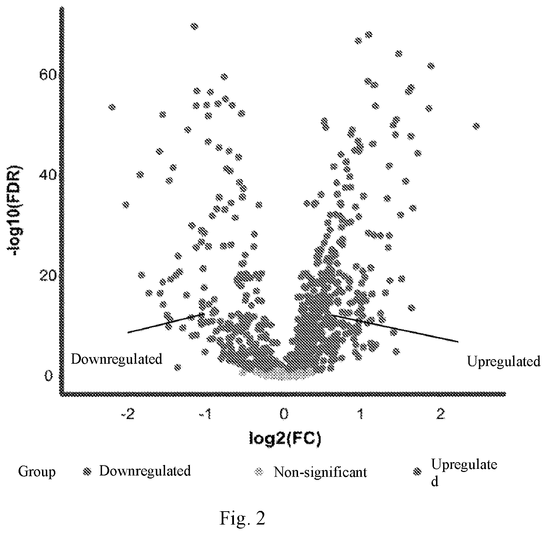

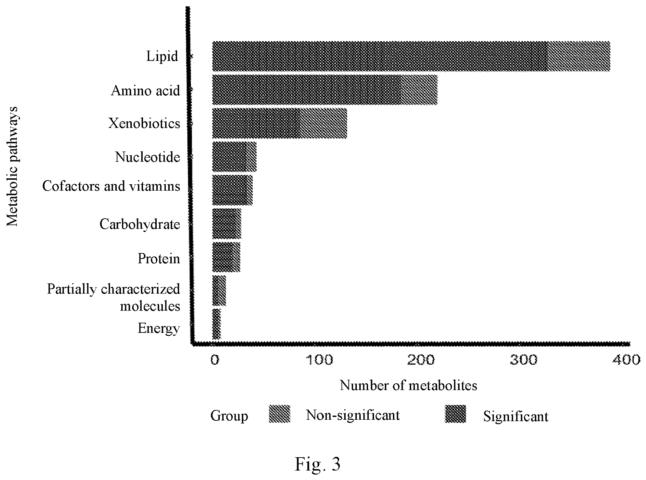

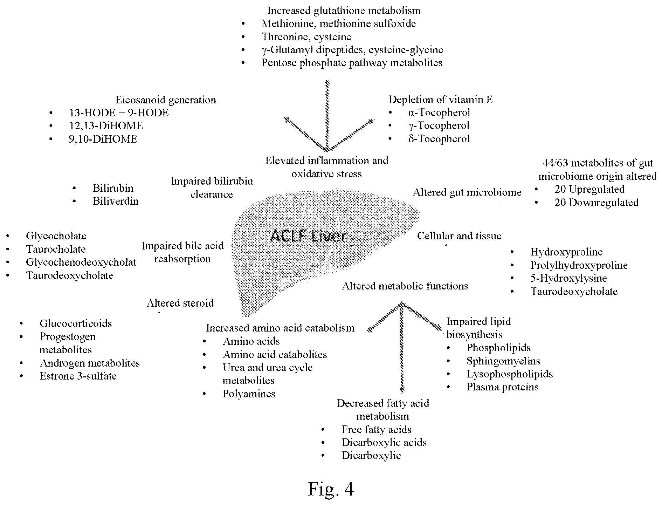

The untargeted metabolomics platform totally identified 1,072 structurally identified metabolites encompassing diverse biochemical pathways, including amino acid, lipid, carbohydrate, nucleotide, xenobiotic, and gut microbiome metabolism. Following the removal of metabolites detected in fewer than 20% of samples, analysis proceeded on the remaining 896 metabolites. It was observed that ACLF had a profound impact on the serum metabolome, with 626 of the 896 metabolites (69.9%) exhibiting significantly altered levels between ACLF and non-ACLF groups (FDR < 0.05, Fig. 2), affecting nearly all metabolite categories (Fig. 3). Following the mapping of metabolites to canonical biochemical pathways, it became evident that the majority of alterations occurred within pathways associated with the liver's diverse metabolic and physiological functions (Fig. 4). The main observation results are as follows:

1. Membrane lipid biosynthesis: among the top 100 most significant metabolites (FDR < $1.41 * 10^{-14}$) in FDR ranking, 34 metabolites are essential lipids for plasma lipoprotein particles and cellular membrane structures, including phospholipids, lysophospholipids, and sphingomyelins. These lipid species were significantly decreased in the ACLF group. As the liver is the primary organ for complex lipid synthesis and central to lipoprotein synthesis, reduced lipid species indicate that lipoprotein biosynthesis may be affected by ACLF. Research results confirm that decreased high-density lipoprotein and low-density lipoprotein levels correlate with hepatic failure and ACLF-related mortality.

2. Oxidative stress and inflammation: one of the characteristics of ACLF is systemic inflammation, manifesting as elevated white blood cell counts, CRPs, pro-inflammatory cytokines, and damage-associated molecular patterns (DAMPs). The oxidative stress biomarker methionine sulfoxide was significantly increased (FDR = $2.54 * 10^{-27}$); the antioxidant vitamin E and the vitamin E metabolites $\alpha$-tocopherol (FDR = $3.43 * 10^{-17}$), $\gamma$-tocopherol/$\beta$-tocopherol (FDR = $6.32 * 10^{-9}$), $\gamma$-carboxyethylhydroxychroman ($\gamma$-CEHC) (FDR = $1.27 * 10^{-23}$), and $\delta$-CEHC (FDR = $8.36 * 10^{-18}$) were all decreased; metabolite levels in glutathione metabolism and pentose phosphate pathways exhibited accelerated elevation.

3. Thyroxine and steroid hormones: the most significant alteration was decreased thyroxine levels in the ACLF group (FDR = $1.94 * 10^{-39}$). It was found that cortisol and corticosterone in the ACLF group were significantly decreased (FDR = $1.11 * 10^{-5}$), conjugated androgen and estrogen metabolites in the ACLF group were significantly decreased; the only exception was significantly increased estrone-3-sulfate (FDR = $2.13 * 10^{-23}$).

4. Energy metabolism: the liver serves as the central regulator of energy homeostasis. The amino acid, amino acid metabolite, urea cycle metabolite, and polyamine levels in the ACLF group were increased, indicating accelerated amino acid catabolism; free fatty acids in the ACLF group were increased, but the principal product 3-hydroxybutyrate of fatty acid $\beta$-oxidation exhibited no alteration. Meanwhile, in the ACLF group, $\omega$-oxidation products such as dicarboxylic acids, dicarboxylic carnitines, hydroxy fatty acids, and hydroxy fatty acid carnitines were elevated. This is because fatty acid $\omega$-oxidation is typically activated by impaired fatty acid $\beta$-oxidation or mitochondrial dysfunction.

5. Gut microbiome: it was observed that gut microbiome-derived metabolites in serum samples were severely disrupted, with 44 out of the total 63 gut microbiome-derived metabolites exhibiting significant alterations between the ACLF and non-ACLF groups. Affected pathways included tryptophan and tyrosine metabolism, secondary bile acids and trimethylamine N-oxide (TMAO) biosynthesis, and xenobiotic metabolism.

6. Bilirubin and primary bile acids: another manifestation of ACLF was elevated bilirubin (FDR = $2.14 * 10^{-31}$) along with its precursor biliverdin (FDR = $1.05 * 10^{-28}$). Under healthy conditions, the liver removes bilirubin via glucuronidation in hepatocytes; elevated bilirubin is a sign of hepatic dysfunction. Furthermore, in the ACLF group, the primary bile acids taurodeoxycholate (FDR = $1.98 * 10^{-15}$), glycochenodeoxycholate (FDR = $4.53 * 10^{-17}$), glycocholate (false omission rate (FOR) = 0.008), and taurocholate (false rejection rate (FRR) = $5.75 * 10^{-5}$) were increased. Primary bile acids are synthesized in the liver, stored in the gallbladder, and secreted into the intestine for food digestion. They are then actively recirculated back to the liver. Elevated primary bile acids are associated with various liver diseases.

Example 2. Development and Validation of Metabolic Biomarkers for Predicting Short-Term Mortality of ACLF Patients

In this example, based on the metabolomic biomarkers preliminarily screened in Example 1, 420 ACLF patient samples were researched to screen and validate metabolic biomarkers capable of predicting the 90-day mortality of ACLF patients.

Among the 420 patients diagnosed with ACLF on admission, 151 died within 90 days. The samples were randomly divided into two equal halves as a discovery set (134 survivors and 76 non-survivors) and a validation set (135 survivors and 75 non-survivors). According to key clinical parameters on admission between the two groups of patients, non-survivors exhibited significantly higher proportions of hepatic failure and coagulation failure, along with elevated TB, INR, white blood cell count, and N/L ratio, and higher MELD-Na and CLIF-C ACLF scores ($p < 0.05$).

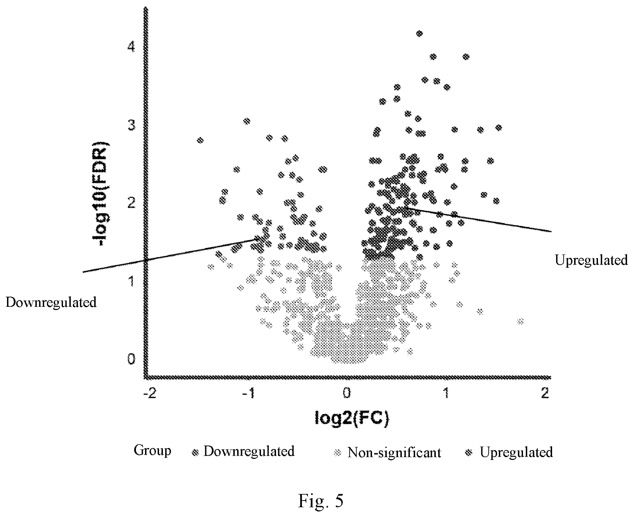

Detection results demonstrated that 234 metabolites were significantly altered between the non-survivor and survivor groups (FDR < 0.05, Fig. 5). These significantly differential metabolites were primarily associated with amino acid degradation, fatty acid $\omega$-oxidation, oxidative stress pathways, and hormone pathways.

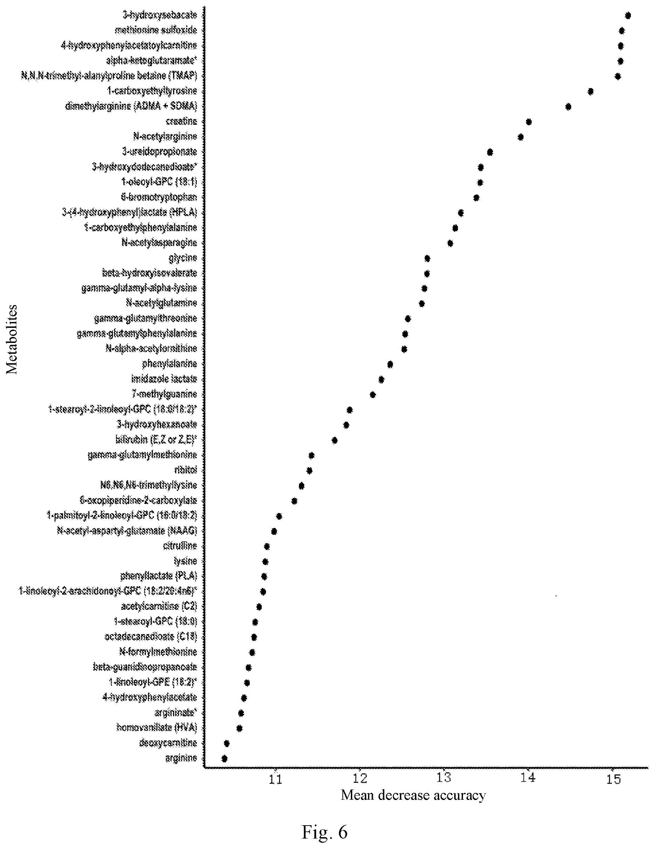

Using random forest, metabolites with maximum power were ranked to distinguish the non-survivor group from the survivor group, with the ranking results shown in Fig. 6. The top 100 metabolites were assessed as potential biomarkers through t-test and random forest ranking. Metabolites for which commercially sourced, ready-to-use chemical and isotopic standards were available were prioritized, as these are more suitable for targeted assay development. The top 15 metabolites among them are listed in Table 2. To identify differential metabolites, the fold change, p-value, and estimated FDR were calculated for each metabolite. Applying a two-sided Wilcoxon rank-sum test, p-values were adjusted through multiple comparison tests via Benjamini-Hochberg (BH) to compute estimated FDR. Analysis confirmed significant associations between these 15 metabolites and the 90-day mortality of ACLF patients, with analysis results shown in Table 3.

Table 2. 15 Metabolic Biomarkers Associated with 90-Day Mortality of ACLF Patients

| No. | Chinese Name | English Name | CAS No. | Molecular formula |
|---|---|---|---|---|
| 1 | 2-Qiang Ji Er Bing Suan | 2-hydroxydadipic.acid | 18294-85-4 | $C_6H_{10}O_5$ |
| 2 | Shi liu wan er suan | hexadecanedioic.acid | 505-54-4 | $C_{16}H_{30}O_4$ |
| 3 | Qiang Fu An Suan | hydroxyproline | 51-35-4 | $C_5H_9NO_3$ |
| 4 | Jia Liu Mi | methionine.sulfoxide | 3226-65-1 | $C_5H_{11}NO_3S$ |
| 5 | 3-Qiang Ji Ding Ji Rou Jian | 3-OH.butyrylcarnitine | 1469900-92-2 | $C_{11}H_{21}NO_5$ |
| 6 | Guan Jie Chang Suan | pipecolic.acid | 535-75-1 | $C_6H_{11}NO_2$ |
| 7 | Liu Suan Ci Tong | estrone.sulfate | 481-97-0 | $C_{18}H_{22}O_5S$ |
| 8 | N-Yi Xian An Ji-Gu An Suan | NAAG | 3106-85-2 | $C_{11}H_{16}N_2O_8$ |
| 9 | Kui Lin Suan | quinolinic.acid | 89-00-9 | $C_7H_5NO_4$ |
| 10 | Niao Ji Bing Suan | ureidopropionic.acid | 462-88-4 | $C_4H_8N_2O_3$ |
| 11 | $\gamma$-CEHC | gamma-carboxyethylhydroxychroman | 178167-75-4 | $C_{15}H_{20}O_4$ |
| 12 | Jia Zhuang Xian Su | thyroxine | 51-48-9 | $C_{15}H_{11}I_4NO_4$ |
| 13 | Jia An Ji Gu An Suan | formiminoglutamic.acid | 816-90-0 | $C_6H_{10}N_2O_4$ |
| 14 | $\Gamma$-3 Gu An Xian Er Tai | gamma-glu-phe | 7432-24-8 | $C_{14}H_{18}N_2O_5$ |
| 15 | Dan Hong Su | Bilirubin | 69853-44-7 | $C_{33}H_{36}N_4O_6$ |

Table 3. Comparison of Detection Results of Correlation Between 15 Metabolites and 90-Day Mortality of ACLF Patients

| No. | Biomarker | RI | MASS | p-value | FDR | Fold Change |
|---|---|---|---|---|---|---|
| 1 | 2-Hydroxydadipic acid | 3000 | 161.0455 | 0.00043 | 0.005931 | 2.106265 |
| 2 | Hexadecanedioic acid | 4615 | 285.2071 | 0.000324 | 0.005023 | 1.699285 |
| 3 | Hydroxyproline | 1064 | 132.0655 | 0.051648837 | 0.124067983 | 1.155849411 |
| 4 | Methionine sulfoxide | 1272 | 166.0533 | 2.49467E-06 | 0.00319318 | 2.006169391 |
| 5 | 3-OH butyrylcarnitine | 2400 | 248.1493 | 0.000168 | 0.003602 | 1.980332 |
| 6 | Pipecolic acid | 2200 | 130.0863 | 7.15E-05 | 0.002464 | 1.593726 |
| 7 | Estrone sulfate | 4417.3 | 349.1115 | 0.000103749 | 0.002789889 | 2.722069235 |
| 8 | NAAG | 1035 | 305.098 | 1.72325E-05 | 0.00110288 | 2.119413468 |
| 9 | Quinolinic acid | 845 | 168.0291 | 0.00073 | 0.007879 | 1.585904 |
| 10 | Ureidopropionic acid | 875 | 133.0608 | 1.15E-06 | 0.000257 | 1.717584 |
| 11 | $\gamma$-CEHC | 3843 | 263.1289 | 3.90293E-05 | 0.001520447 | 0.357239443 |
| 12 | Thyroxine | 4808 | 775.6794 | 0.028389914 | 0.081248518 | 0.737049142 |
| 13 | Formiminoglutamic acid | 1880 | 175.0713 | 0.000873 | 0.009097 | 1.824112 |
| 14 | $\gamma$-glu-phe | 1825 | 293.1143 | 8.13E-05 | 0.002602 | 1.562759 |
| 15 | Bilirubin | 1840 | 585.2708 | 0.0099778203 | 0.198669489 | 1.200212292 |

The predictive efficacy of the 15 metabolic biomarkers for the 90-day mortality of ACLF patients was assessed by ROC curves in the discovery set and the validation set (including 269 survivors and 151 non-survivors), with the results shown in Table 4.

Table 4. ROC Analysis Results of 15 Metabolites for Predicting 90-Day Mortality of ACLF Patients

| No. | Biomarker | AUC value |
|---|---|---|
| 1 | 2-Hydroxydadipic acid | 0.669 |
| 2 | Hexadecanedioic acid | 0.65 |
| 3 | Hydroxyproline | 0.632 |
| 4 | Methionine sulfoxide | 0.61 |
| 5 | 3-OH butyrylcarnitine | 0.644 |
| 6 | Pipecolic acid | 0.694 |
| 7 | Estrone sulfate | 0.694 |
| 8 | NAAG | 0.686 |
| 9 | Quinolinic acid | 0.612 |
| 10 | Ureidopropionic acid | 0.719 |
| 11 | $\gamma$-CEHC | 0.633 |
| 12 | Thyroxine | 0.664 |
| 13 | Formiminoglutamic acid | 0.627 |
| 14 | $\gamma$-glu-phe | 0.69 |
| 15 | Bilirubin | 0.601 |

The strength of association between alterations in concentration of the 15 biomarkers and 90-day survival of ACLF patients can be distinguished using RI, MASS, p-value, FDR, and Fold Change in Table 3 or using AUC values in Table 4, with FDR and AUC values being particularly intuitive and significant. The lower the FDR value, the stronger the impact of the biomarker on the non-survivors within 90 days in ACLF patients compared to survivors within 90 days in ACLF patients. The higher the AUC value, the greater the accuracy of the biomarker in distinguishing non-survivors from survivors within 90 days in ACLF patients.

It can be seen from Table 3 that alterations in concentration of 15 biomarkers exhibit significant associations with 90-day survival of ACLF patients, where ureidopropionic acid exhibits the strongest association, with FDR value reaching 0.000257, followed by NAAG, with FDR value reaching 0.00110288.

It can be seen from Table 4 that individually using any one of 15 biomarkers to distinguish non-survivors from survivors within 90 days of ACLF patients through concentration alterations, all can achieve AUC values exceeding 0.6 and exhibit higher accuracy, where ureidopropionic acid exhibits the highest AUC value reaching 0.719, followed by pipecolic acid and estrone sulfate with AUC values reaching 0.694.

Furthermore, in this example, the 15 biomarkers screened also have a good effect of predicting the 360-day mortality risk of ACLF patients, with AUC values exceeding 0.5.

Example 3. Development and Validation of Metabolic Biomarkers for Predicting Short-Term Progression Risk to ACLF of non-ACLF Patients

In this example, based on the metabolomic biomarkers preliminarily screened in Example 1, 880 non-ACLF patient samples were researched to screen and validate metabolic biomarkers capable of predicting the 28-day progression risk to ACLF of non-ACLF patients.

The non-ACLF group included 880 patients, among whom 108 patients developed ACLF within 28 days after non-ACLF diagnosis (pre-ACLF). The samples were randomly divided into two equal halves as a discovery set and a validation set (each including 54 pre-ACLF patients and 386 non-ACLF patients).

Compared to patients maintaining stable conditions during this period, those progressing to ACLF exhibited significantly elevated TB, INR, and N/L ratio, as well as higher MELD-Na and CLIF-C ACLF-D scores ($p < 0.05$).

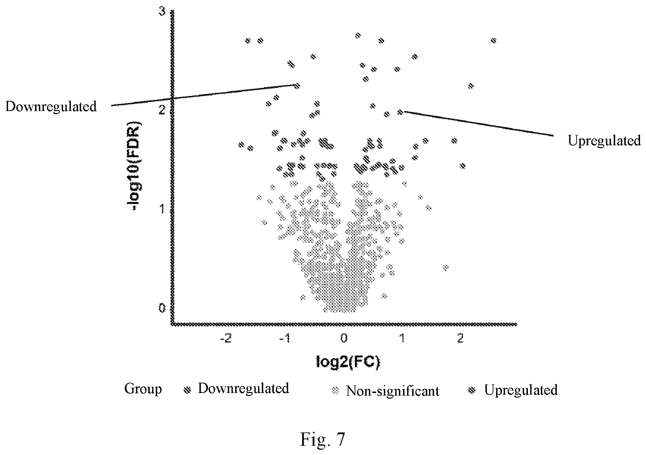

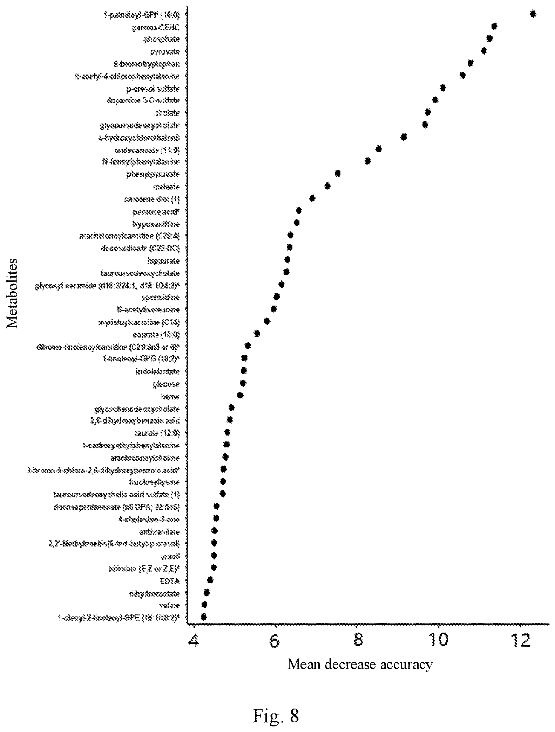

A total of 89 metabolites exhibited significant alterations in level between the pre-ACLF and non-ACLF groups (FDR < 0.05, Fig. 7). Metabolites associated with fatty acid $\omega$-oxidation, oxidative stress pathways, bilirubin metabolism, bile acid metabolism, and steroid hormone pathways constituted the majority of significant alterations. Using a method analogous to that used for ACLF mortality biomarker identification, random forest was used to rank metabolites for distinguishing the pre-ACLF group from the non-ACLF group (Fig. 8). The top 89 metabolites ranked by t-test and/or random forest were further assessed as potential biomarkers, metabolites lacking commercially available chemical and isotopic standards were excluded, and the top 15 ranked metabolites were selected. These 15 metabolites were consistent with those predicting the 90-day mortality of ACLF patients. To identify differential metabolites, the fold change, p-value, and estimated FDR were calculated for each metabolite. Applying a two-sided Wilcoxon rank-sum test, p-values were adjusted through multiple comparison tests via Benjamini-Hochberg (BH) to compute estimated FDR. Analysis confirmed significant associations between these 15 metabolites and the 28-day progression risk to ACLF of non-ACLF patients, with analysis results shown in Table 5.

Table 5. Comparison of Detection Results of Correlation Between 15 Metabolites and 28-Day Progression Risk to ACLF of Non-ACLF Patients

| No. | Biomarker | RI | MASS | p-value | FDR | Fold Change |
|---|---|---|---|---|---|---|
| 1 | 2-Hydroxydadipic acid | 3000 | 161.0455 | 0.015924 | 0.092754 | 1.663287 |
| 2 | Hexadecanedioic acid | 4615 | 285.2071 | 0.018446 | 0.102023 | 1.473388 |
| 3 | Hydroxyproline | 1064 | 132.0655 | 0.751630289 | 0.867244843 | 1.021273795 |
| 4 | Methionine sulfoxide | 1272 | 166.0533 | 0.000641548 | 0.01957662 | 1.357941699 |
| 5 | 3-OH butyrylcarnitine | 2400 | 248.1493 | 0.33344 | 0.563702 | 1.160571 |
| 6 | Pipecolic acid | 2200 | 130.0863 | 0.148843 | 0.353749 | 1.190887 |
| 7 | Estrone sulfate | 4417.3 | 349.1115 | 0.005572803 | 0.052845904 | 2.059286596 |
| 8 | NAAG | 1035 | 305.098 | 0.913227761 | 0.959263862 | 0.999207206 |
| 9 | Quinolinic acid | 845 | 168.0291 | 0.875164 | 0.939098 | 1.039392 |
| 10 | Ureidopropionic acid | 875 | 133.0608 | 1.07E-05 | 0.001914 | 1.556914 |
| 11 | $\gamma$-CEHC | 3843 | 263.1289 | 0.003324966 | 0.037239622 | 0.465725777 |
| 12 | Thyroxine | 4808 | 775.6794 | 2.1074E-05 | 0.0027813 | 0.692212526 |
| 13 | Formiminoglutamic acid | 1880 | 175.0713 | 0.185424 | 0.397093 | 1.213627 |
| 14 | $\gamma$-glu-phe | 1825 | 293.1143 | 0.044516 | 0.16392 | 1.10889 |
| 15 | Bilirubin | 1840 | 585.2708 | 0.000184722 | 0.008711105 | 1.409553609 |

The predictive efficacy of the 15 metabolic biomarkers for the 28-day progression risk to ACLF of non-ACLF patients was assessed by ROC curves in the discovery set and the validation set (including 108 pre-ACLF patients (who developed ACLF within 28 days after non-ACLF diagnosis) and 772 non-ACLF patients (who did not develop ACLF within 28 days after non-ACLF diagnosis)), with the results shown in Table 6.

Table 6. ROC Analysis Results of 15 Metabolites for Predicting 28-Day Progression Risk to ACLF of Non-ACLF Patients

| No. | Biomarker | AUC value |
|---|---|---|
| 1 | 2-Hydroxydadipic acid | 0.682 |
| 2 | Hexadecanedioic acid | 0.649 |
| 3 | Hydroxyproline | 0.683 |
| 4 | Methionine sulfoxide | 0.663 |
| 5 | 3-OH butyrylcarnitine | 0.685 |
| 6 | Pipecolic acid | 0.684 |
| 7 | Estrone sulfate | 0.649 |
| 8 | NAAG | 0.601 |
| 9 | Quinolinic acid | 0.644 |
| 10 | Ureidopropionic acid | 0.723 |
| 11 | $\gamma$-CEHC | 0.724 |

(continued)

| No. | Biomarker | AUC value |
|---|---|---|
| 12 | Thyroxine | 0.689 |
| 13 | Formiminoglutamic acid | 0.695 |
| 14 | $\gamma$-glu-phe | 0.614 |
| 15 | Bilirubin | 0.668 |

The strength of association between alterations in concentration of the 15 biomarkers and 28-day progression to ACLF of non-ACLF patients can be distinguished using RI, MASS, p-value, FDR, and Fold Change in Table 5 or using AUC values in Table 6, with FDR and AUC values being particularly intuitive and significant. The lower the FDR value, the stronger the impact of the biomarker on the non-ACLF patients progressing to ACLF within 28 days compared to the non-ACLF patients not progressing to ACLF within 28 days. The higher the AUC value, the greater the accuracy of the biomarker in distinguishing whether the non-ACLF patients progressed to ACLF within 28 days.

It can be seen from Table 5 that alterations in concentration of 15 biomarkers exhibit significant associations with 28-day progression to ACLF of non-ACLF patients, where ureidopropionic acid exhibits the strongest association, with FDR value reaching 0.001914, followed by thyroxine, with FDR value reaching 0.0027813.

It can be seen from Table 6 that individually using any one of 15 biomarkers to distinguish whether the non-ACLF patients progressed to ACLF within 28 days through concentration alterations, all can achieve AUC values exceeding 0.6 and exhibit higher accuracy, where $\gamma$-CEHC exhibits the highest AUC value reaching 0.724, followed by ureidopropionic acid with AUC values reaching 0.723.

Furthermore, in this example, the 15 biomarkers screened also have a good effect of predicting the 180-day progression risk to ACLF of non-ACLF patients, with AUC values exceeding 0.5.

Example 4. Comparison of Targeted and Untargeted Detection Performance for 15-Metabolite Combination

To translate metabolomics discoveries into clinical applications, LC-MS was used to perform untargeted and targeted detection for the 15 biomarkers for predicting the 90-day mortality of ACLF patients and the 28-day progression risk to ACLF of non-ACLF patients, including $\gamma$-CEHC, NAAG, pipecolic acid, thyroxine, and ureidopropionic acid. The detection accuracy, precision, stability, and predictive performance of these 15 biomarkers were further assessed.

In targeted detection, absolute qualitative and quantitative analysis of analytes was performed using standards. The specific analytical procedure included: adding internal standard solution containing a stable isotope-labeled analyte analogue to plasma samples, followed by protein precipitation with acidified methanol (1% formic acid in methanol). Following centrifugation, the supernatant was removed, evaporated, and reconstituted. An aliquot of the extract was injected into the SciexExion LC/Sciex 5500 + triple quadrupole LC-MS/MS system. Using C18 reversed-phase chromatography, 3-ureidopropionate, formiminoglutamate, NAAG, thyroxine, and $\gamma$-CEHC were analyzed via a water (0.1% formic acid)/acetonitrile gradient within 6.0 minutes with the mass spectrometer operating in negative mode. A second aliquot of the sample was injected into the identical LC-MS/MS system for pipecolic acid analysis. C18 reversed-phase ion-pairing chromatography was performed using a water (0.05% nonafluoropentanoic acid)/acetonitrile (0.05% nonafluoropentanoic acid) gradient within 4.0 minutes, with the mass spectrometer operating in positive mode.

The targeted detection analysis has a short runtime on LC-MS and is suitable for fast data turnaround and large-scale detection in clinical testing.

During targeted analysis, authentic chemical and isotopic standards were added to each sample, enabling absolute quantitation of metabolites in samples through comparison with calibration curves from eight concentrations of metabolite standards. The eight concentrations referred to a concentration at the beginning of the analytical run, a concentration at the end of the analytical run, and six individual concentration levels of quality control samples, to monitor analytical performance. The peak areas of precursor-to-product ion transitions were measured relative to corresponding internal standards, with quantitation performed using weighted linear least squares regression analysis.

During the initial screening of metabolic biomarkers, untargeted determination was employed for screening. In this example, concurrent performing of targeted and untargeted detection demonstrated alteration correlation between the two detection methods, confirming consistent overall trends in untargeted and targeted detection. The specific detection results were converted through correlation coefficients. Thus, in subsequent examples, targeted detection was used for model construction and analysis of metabolic biomarkers to achieve more precise quantitation.

The quantitative data generated by targeted detection exhibited an alteration correlation with untargeted metabolomics data. Correlation coefficients ranged from 0.502 (formiminoglutamic acid) to 0.725 (pipecolic acid).

Example 5. Development and Validation of Prediction Model for 90-Day Mortality of ACLF Patients

In this example, the 15 metabolites screened in Example 2 were combined, and logistic regression was used to construct a prediction model for 90-day mortality of ACLF patients. The goal is to construct the model using as few metabolites as possible while achieving satisfactory predictive performance.

(1) Model with 15-metabolite combination

Initially, 15 metabolites were combined to construct a prediction model for 90-day mortality of ACLF patients, and its predictive performance was assessed.

15 Metabolites: pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

The model equation was:

P = -20.584 + 0.846 * pipecolic acid + 1.254 * NAAG + 0.454 * ureidopropionic acid - 0.468 * formiminoglutamic acid - 0.385 * γ-CEHC + 0.485 * γ-glu-phe + 0.447 * 3-OH butyrylcarnitine + 0.081 * estrone sulfate + 0.524 * hydroxyproline + 0.079 * bilirubin - 0.066 * 2-hydroxydadipic acid - 0.015 * methionine sulfoxide - 0.089 * hexadecanedioic acid - 0.219 * quinolinic acid + 0.942 * thyroxine

In the discovery set, the model constructed with the 15 metabolites yielded an AUC of 0.829 for predicting 90-day mortality of ACLF patients; in the validation set, the AUC for predicting 90-day mortality of ACLF patients was 0.734.

(2) Model with 14-metabolite combination

Based on the 15 metabolites, any one metabolite was excluded, with a total of 15 exclusion methods. The remaining 14 metabolites were used to construct prediction models for 90-day mortality of ACLF patients. After comparing the prediction models constructed by 15 exclusion methods, the prediction model with the best combined performance and the highest AUC was selected, yielding the following 14-metabolite combination:
pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid

The model equation was:

P = -14.286 + 0.804 * pipecolic acid + 0.791 * NAAG + 0.448 * ureidopropionic acid - 0.396 * formiminoglutamic acid - 0.338 * γ-CEHC + 0.434 * γ-glu-phe + 0.425 * 3-OH butyrylcarnitine + 0.107 * estrone sulfate + 0.356 * hydroxyproline + 0.091 * bilirubin - 0.082 * 2-hydroxydadipic acid + 0.015 * methionine sulfoxide - 0.066 * hexadecanedioic acid - 0.154 * quinolinic acid

In the discovery set, the model constructed with the 14 metabolites yielded an AUC of 0.822 for predicting 90-day mortality of ACLF patients; in the validation set, the AUC for predicting 90-day mortality of ACLF patients was 0.760.

(3) Model with 13-metabolite combination

Based on the 14 metabolites, any one metabolite was excluded, with a total of 14 exclusion methods. The remaining 13 metabolites were used to construct prediction models for 90-day mortality of ACLF patients. After comparing the prediction models constructed by 14 exclusion methods, the prediction model with the best combined performance and the highest AUC was selected, yielding the following 13-metabolite combination:
pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid

The model equation was:

P = -14.392 + 0.799 * pipecolic acid + 0.792 * NAAG + 0.383 * ureidopropionic acid - 0.403 * formiminoglutamic acid - 0.352 * γ-CEHC + 0.428 * γ-glu-phe + 0.388 * 3-OH butyrylcarnitine + 0.121 * estrone sulfate + 0.353 * hydroxyproline + 0.095 * bilirubin - 0.153 * 2-hydroxydadipic acid + 0.009 * methionine sulfoxide - 0.025 * hexadecanedioic acid

In the discovery set, the model constructed with the 13 metabolites yielded an AUC of 0.819 for predicting 90-day mortality of ACLF patients; in the validation set, the AUC for predicting 90-day mortality of ACLF patients was 0.726.

(4) Model with 12-metabolite combination

Based on the 13 metabolites, any one metabolite was excluded, with a total of 13 exclusion methods. The remaining 12 metabolites were used to construct prediction models for 90-day mortality of ACLF patients. After comparing the prediction models constructed by 13 exclusion methods, the prediction model with the best combined performance and the highest AUC was selected, yielding the following 12-metabolite combination:
pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide
The model equation was:

P = -14.5 + 0.794 * pipecolic acid + 0.806 * NAAG + 0.376 * ureidopropionic acid-0.403 * formiminoglutamic acid - 0.352 * γ-CEHC + 0.429 * γ-glu-phe + 0.378 * 3-OH butyrylcarnitine + 0.114 * estrone sulfate + 0.362 * hydroxyproline + 0.095 * bilirubin - 0.16 * 2-hydroxydadipic acid + 0.007 * methionine sulfoxide

In the discovery set, the model constructed with the 12 metabolites yielded an AUC of 0.819 for predicting 90-day mortality of ACLF patients; in the validation set, the AUC for predicting 90-day mortality of ACLF patients was 0.763.

(5) Model with 11-metabolite combination

Based on the 12 metabolites, any one metabolite was excluded, with a total of 12 exclusion methods. The remaining 11 metabolites were used to construct prediction models for 90-day mortality of ACLF patients. After comparing the prediction models constructed by 12 exclusion methods, the prediction model with the best combined performance and the highest AUC was selected, yielding the following 11-metabolite combination:
pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid
The model equation was:

P = -14.476 + 0.795 * pipecolic acid + 0.807 * NAAG + 0.376 * ureidopropionic acid - 0.404 * formiminoglutamic acid - 0.353 * γ-CEHC + 0.43 * γ-glu-phe + 0.375 * 3-OH butyrylcarnitine + 0.115 * estrone sulfate + 0.364 * hydroxyproline + 0.096 * bilirubin - 0.157 * 2-hydroxydadipic acid

In the discovery set, the model constructed with the 11 metabolites yielded an AUC of 0.819 for predicting 90-day mortality of ACLF patients; in the validation set, the AUC for predicting 90-day mortality of ACLF patients was 0.763.

(6) Model with 10-metabolite combination

Based on the 11 metabolites, any one metabolite was excluded, with a total of 11 exclusion methods. The remaining 10 metabolites were used to construct prediction models for 90-day mortality of ACLF patients. After comparing the prediction models constructed by 11 exclusion methods, the prediction model with the best combined performance and the highest AUC was selected, yielding the following 10-metabolite combination:
pipecolic acid, NAAG, ureidopropionic acid, formiminoglutamic acid, γ-CEHC, γ-glu-phe, 3-OH butyrylcarnitine, estrone sulfate, hydroxyproline, and bilirubin
The model equation was:

P = -14.315 + 0.777 * pipecolic acid + 0.77 * NAAG + 0.342 * ureidopropionic acid - 0.411 * formiminoglutamic acid - 0.352 * γ-CEHC + 0.432 * γ-glu-phe + 0.325 * 3-OH butyrylcarnitine + 0.109 * estrone sulfate + 0.357 * hydroxyproline + 0.1 * bilirubin

In the discovery set, the model constructed with the 10 metabolites yielded an AUC of 0.817 for predicting 90-day mortality of ACLF patients; in the validation set, the AUC for predicting 90-day mortality of ACLF patients was 0.763.

(7) Model with 9-metabolite combination

Based on the 10 metabolites, any one metabolite was excluded, with a total of 10 exclusion methods. The remaining 9 metabolites were used to construct prediction models for 90-day mortality of ACLF patients. After comparing the prediction models constructed by 10 exclusion methods, the prediction model with the best combined performance and the highest AUC was selected, yielding the following 9-metabolite combination:
pipecolic acid, NAAG, ureidopropionic acid, formiminoglutamic acid, γ-CEHC, γ-glu-phe, 3-OH butyrylcarnitine, estrone sulfate, and hydroxyproline

The model equation was:

P = -13.898 + 0.825 * pipecolic acid + 0.744 * NAAG + 0.322 * ureidopropionic acid + -0.391 * formiminoglutamic acid + -0.269 * γ-CEHC + 0.424 * γ-glu-phe + 0.345 * 3-OH butyrylcarnitine + 0.162 * estrone sulfate + 0.276 * hydroxyproline

In the discovery set, the model constructed with the 9 metabolites yielded an AUC of 0.815 for predicting 90-day mortality of ACLF patients; in the validation set, the AUC for predicting 90-day mortality of ACLF patients was 0.763.

(8) Model with 8-metabolite combination

Based on the 9 metabolites, any one metabolite was excluded, with a total of 9 exclusion methods. The remaining 8 metabolites were used to construct prediction models for 90-day mortality of ACLF patients. After comparing the prediction models constructed by 9 exclusion methods, the prediction model with the best combined performance and the highest AUC was selected, yielding the following 8-metabolite combination:
pipecolic acid, NAAG, ureidopropionic acid, formiminoglutamic acid, γ-CEHC, γ-glu-phe, 3-OH butyrylcarnitine, and estrone sulfate

The model equation was:

P = -12.473 + 0.857 * pipecolic acid + 0.781 * NAAG + 0.415 * ureidopropionic acid - 0.405 * formiminoglutamic acid - 0.257 * γ-CEHC + 0.42 * γ-glu-phe + 0.343 * 3-OH butyrylcarnitine + 0.14 * estrone sulfate

In the discovery set, the model constructed with the 8 metabolites yielded an AUC of 0.813 for predicting 90-day mortality of ACLF patients; in the validation set, the AUC for predicting 90-day mortality of ACLF patients was 0.759.

(9) Model with 7-metabolite combination

Based on the 8 metabolites, any one metabolite was excluded, with a total of 8 exclusion methods. The remaining 7 metabolites were used to construct prediction models for 90-day mortality of ACLF patients. After comparing the prediction models constructed by 8 exclusion methods, the prediction model with the best combined performance and the highest AUC was selected, yielding the following 7-metabolite combination:
pipecolic acid, NAAG, ureidopropionic acid, formiminoglutamic acid, γ-CEHC, γ-glu-phe, and 3-OH butyrylcarnitine

The model equation was:

P = -13.147 + 0.947 * pipecolic acid + 0.832 * NAAG + 0.435 * ureidopropionic acid - 0.407 * formiminoglutamic acid - 0.292 * γ-CEHC + 0.428 * γ-glu-phe + 0.334 * 3-OH butyrylcarnitine

In the discovery set, the model constructed with the 7 metabolites yielded an AUC of 0.812 for predicting 90-day mortality of ACLF patients; in the validation set, the AUC for predicting 90-day mortality of ACLF patients was 0.755.

(10) Model with 6-metabolite combination

Based on the 7 metabolites, any one metabolite was excluded, with a total of 7 exclusion methods. The remaining 6 metabolites were used to construct prediction models for 90-day mortality of ACLF patients. After comparing the prediction models constructed by 7 exclusion methods, the prediction model with the best combined performance and the highest AUC was selected, yielding the following 6-metabolite combination:
pipecolic acid, NAAG, ureidopropionic acid, formiminoglutamic acid, γ-CEHC, and γ-glu-phe

The model equation was:

P = -12.838 + 0.839 * pipecolic acid + 1.125 * NAAG + 0.49 * ureidopropionic acid - 0.39 * formiminoglutamic acid - 0.263 * γ-CEHC + 0.39 * γ-glu-phe

In the discovery set, the model constructed with the 6 metabolites yielded an AUC of 0.802 for predicting 90-day mortality of ACLF patients; in the validation set, the AUC for predicting 90-day mortality of ACLF patients was 0.746.

(11) Model with 5-metabolite combination

Based on the 6 metabolites, any one metabolite was excluded, with a total of 6 exclusion methods. The remaining 5 metabolites were used to construct prediction models for 90-day mortality of ACLF patients. After comparing the prediction models constructed by 6 exclusion methods, the prediction model with the best combined performance and the highest AUC was selected, yielding the following 5-metabolite combination:
pipecolic acid, NAAG, ureidopropionic acid, formiminoglutamic acid, and γ-CEHC
The model equation was:

P = -10.874 + 0.685 * pipecolic acid + 1.105 * NAAG + 0.51 * ureidopropionic acid-0.214 * formiminoglutamic acid - 0.22 * γ-CEHC

In the discovery set, the model constructed with the 5 metabolites yielded an AUC of 0.793 for predicting 90-day mortality of ACLF patients; in the validation set, the AUC for predicting 90-day mortality of ACLF patients was 0.736.

(12) Model with 4-metabolite combination

Based on the 5 metabolites, any one metabolite was excluded, with a total of 5 exclusion methods. The remaining 4 metabolites were used to construct prediction models for 90-day mortality of ACLF patients. After comparing the prediction models constructed by 5 exclusion methods, the prediction model with the best combined performance and the highest AUC was selected, yielding the following 4-metabolite combination:
pipecolic acid, NAAG, ureidopropionic acid, and formiminoglutamic acid
The model equation was:

P = -11.911 + 0.81 * pipecolic acid + 0.992 * NAAG + 0.559 * ureidopropionic acid - 0.2 * formiminoglutamic acid

In the discovery set, the model constructed with the 4 metabolites yielded an AUC of 0.790 for predicting 90-day mortality of ACLF patients; in the validation set, the AUC for predicting 90-day mortality of ACLF patients was 0.724.

(13) Model with 3-metabolite combination

Based on the 4 metabolites, any one metabolite was excluded, with a total of 4 exclusion methods. The remaining 3 metabolites were used to construct prediction models for 90-day mortality of ACLF patients. After comparing the prediction models constructed by 4 exclusion methods, the prediction model with the best combined performance and the highest AUC was selected, yielding the following 3-metabolite combination:
pipecolic acid, NAAG, and ureidopropionic acid
The model equation was:

$$P = -11.25 + 0.73 * \text{pipecolic acid} + 0.917 * \text{NAAG} + 0.435 * \text{ureidopropionic acid}$$

In the discovery set, the model constructed with the 3 metabolites yielded an AUC of 0.775 for predicting 90-day mortality of ACLF patients; in the validation set, the AUC for predicting 90-day mortality of ACLF patients was 0.722.
It can be seen that the prediction model for 90-day mortality of ACLF patients constructed with the 3 metabolites also achieves a good predictive effect, requiring fewer metabolite types and lower costs.
However, upon further removal of one metabolite and retaining only two metabolites, the AUC for predicting 90-day mortality of ACLF patients significantly decreased in both the discovery set and the validation set, falling below 0.7. Therefore, it is not recommended to construct a prediction model for 90-day mortality of ACLF patients using a 2-metabolite combination.
In summary, as the number of metabolite types decreases, during the prediction of the constructed prediction models for 90-day mortality of ACLF patients, the AUC values exhibit a slight decrease in both the discovery set and the validation set. However, employing excessive metabolites increases the burden on the detection process. After evaluating the trade-offs, models constructed with 3-4 metabolites are preferred.

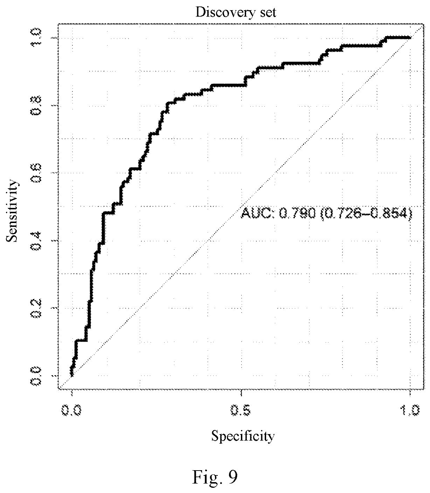

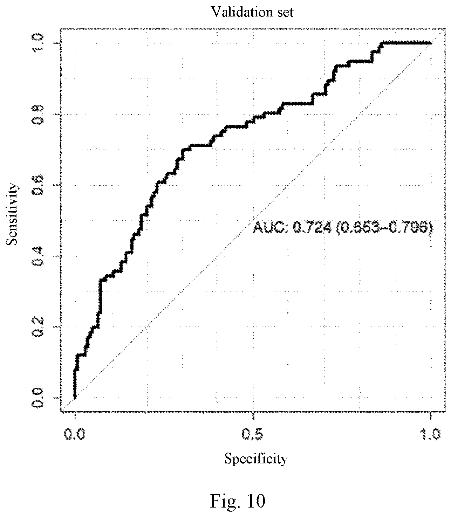

When the model constructed with 4 indicators, pipecolic acid, NAAG, ureidopropionic acid, and formiminoglutamic acid, was selected, higher AUC values were maintained in both the discovery set and the validation set. In the discovery set, the AUC for predicting 90-day mortality of ACLF patients was 0.79 (Fig. 9). This result was precisely replicated in the validation set, with an AUC of 0.724 (Fig. 10). Research demonstrates that for the model constructed with 4 metabolites, prediction value P greater than 0.331 indicates predicting a high risk of 90-day mortality of ACLF patients; prediction value P less than 0.331 indicates predicting a low risk of 90-day mortality of ACLF patients.

When the model constructed with 3 indicators, pipecolic acid, NAAG, and ureidopropionic acid, was selected, higher AUC values were maintained in both the discovery set and the validation set. In the discovery set, the AUC for predicting 90-day mortality of ACLF patients was 0.775. This result was precisely replicated in the validation set, with an AUC of 0.722. Research demonstrates that for the model constructed with 3 metabolites, prediction value P greater than 0.320 indicates predicting a high risk of 90-day mortality of ACLF patients; prediction value P less than 0.320 indicates predicting a low risk of 90-day mortality of ACLF patients.

Furthermore, in this example, the screened 3-metabolite model (P = -11.25 + 0.73 * pipecolic acid + 0.917 * NAAG + 0.435 * ureidopropionic acid) and 4-metabolite model (P = -11.911 + 0.81 * pipecolic acid + 0.992 * NAAG + 0.559 * ureidopropionic acid - 0.2 * formiminoglutamic acid) also have a good effect of predicting the 360-day mortality risk of ACLF patients, with AUC values exceeding 0.6.

Example 6. Development and Validation of Prediction Model for 28-Day Progression Risk to ACLF of Non-ACLF Patients

In this example, the 15 metabolites screened in Example 3 were combined, and logistic regression was used to construct a prediction model for 28-day progression risk to ACLF of non-ACLF patients. The goal is to construct the model using as few metabolites as possible while achieving satisfactory predictive performance.

(1) Model with 15-metabolite combination

Initially, 15 metabolites were combined to construct a prediction model for 28-day progression to ACLF of non-ACLF patients, and its predictive performance was assessed.

15 Metabolites: ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, NAAG, and methionine sulfoxide

The model equation was:

P = -3.271 + 0.716 * ureidopropionic acid - 0.512 * γ-CEHC - 1.1 * thyroxine + 0.357 * pipecolic acid + 0.134 * 3-OH butyrylcarnitine - 1.225 * 2-hydroxydadipic acid + 0.029 * estrone sulfate + 0.168 * hydroxyproline - 0.04 * quinolinic acid + 0.078 * γ-glu-phe - 0.014 * formiminoglutamic acid - 0.03 * bilirubin + 0.519 * hexadecanedioic acid - 0.139 * methionine sulfoxide + 0.653 * NAAG

In the discovery set, the model constructed with the 15 metabolites yielded an AUC of 0.838 for predicting 28-day progression to ACLF of non-ACLF patients; in the validation set, the AUC for predicting 28-day progression to ACLF of non-ACLF patients was 0.741.

(2) Model with 14-metabolite combination

Based on the 15 metabolites, any one metabolite was excluded, with a total of 15 exclusion methods. The remaining 14 metabolites were used to construct prediction models for 28-day progression risk to ACLF of non-ACLF patients. After comparing the prediction models constructed by 15 exclusion methods, the prediction model with the best combined performance and the highest AUC was selected, yielding the following 14-metabolite combination:

ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, methionine sulfoxide, bilirubin, and hexadecanedioic acid

The model equation was:

P = -1.518 + 0.724 * ureidopropionic acid - 0.494 * γ-CEHC - 1.216 * thyroxine + 0.409 * pipecolic acid + 0.157 * 3-OH butyrylcarnitine - 1.131 * 2-hydroxydadipic acid + 0.051 * estrone sulfate + 0.202 * hydroxyproline - 0.024 * quinolinic acid + 0.11 * γ-glu-phe - 0.007 * formiminoglutamic acid + 0.009 * bilirubin + 0.479 * hexadecanedioic acid - 0.17 * methionine sulfoxide

In the discovery set, the model constructed with the 14 metabolites yielded an AUC of 0.830 for predicting 28-day progression risk to ACLF of non-ACLF patients; in the validation set, the AUC for predicting 28-day progression risk to ACLF of non-ACLF patients was 0.746.

(3) Model with 13-metabolite combination

Based on the 14 metabolites, any one metabolite was excluded, with a total of 14 exclusion methods. The remaining 13 metabolites were used to construct prediction models for 28-day progression risk to ACLF of non-ACLF patients. After comparing the prediction models constructed by 14 exclusion methods, the prediction model with the best combined performance and the highest AUC was selected, yielding the following 13-metabolite combination: ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid
The model equation was:

P = -2.436 + 0.726 * ureidopropionic acid + -0.498 * γ-CEHC - 1.18 * thyroxine + 0.382 * pipecolic acid + 0.195 * 3-OH butyrylcarnitine - 1.121 * 2-hydroxydadipic acid + 0.044 * estrone sulfate + 0.199 * hydroxyproline - 0.009 * quinolinic acid + 0.046 * γ-glu-phe - 0.014 * formiminoglutamic acid - 0.012 * bilirubin + 0.446 * hexadecanedioic acid

In the discovery set, the model constructed with the 13 metabolites yielded an AUC of 0.833 for predicting 28-day progression risk to ACLF of non-ACLF patients; in the validation set, the AUC for predicting 28-day progression risk to ACLF of non-ACLF patients was 0.748.

(4) Model with 12-metabolite combination

Based on the 13 metabolites, any one metabolite was excluded, with a total of 13 exclusion methods. The remaining 12 metabolites were used to construct prediction models for 28-day progression risk to ACLF of non-ACLF patients. After comparing the prediction models constructed by 13 exclusion methods, the prediction model with the best combined performance and the highest AUC was selected, yielding the following 12-metabolite combination: ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin
The model equation was:

P = -2.837 + 0.819 * ureidopropionic acid - 0.514 * γ-CEHC - 1.019 * thyroxine + 0.399 * pipecolic acid + 0.362 * 3-OH butyrylcarnitine - 0.932 * 2-hydroxydadipic acid + 0.173 * estrone sulfate + 0.235 * hydroxyproline - 0.08 * quinolinic acid + 0.074 * γ-glu-phe + 0.001 * formiminoglutamic acid - 0.02 * bilirubin

In the discovery set, the model constructed with the 12 metabolites yielded an AUC of 0.824 for predicting 28-day progression risk to ACLF of non-ACLF patients; in the validation set, the AUC for predicting 28-day progression risk to ACLF of non-ACLF patients was 0.751.

(5) Model with 11-metabolite combination

Based on the 12 metabolites, any one metabolite was excluded, with a total of 12 exclusion methods. The remaining 11 metabolites were used to construct prediction models for 28-day progression risk to ACLF of non-ACLF patients. After comparing the prediction models constructed by 12 exclusion methods, the prediction model with the best combined performance and the highest AUC was selected, yielding the following 11-metabolite combination: ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid
The model equation was:

P = -2.894 + 0.816 * ureidopropionic acid - 0.512 * γ-CEHC - 1.016 * thyroxine + 0.395 * pipecolic acid + 0.362 * 3-OH butyrylcarnitine - 0.93 * 2-hydroxydadipic acid + 0.166 * estrone sulfate + 0.238 * hydroxyproline - 0.076 * quinolinic acid + 0.071 * γ-glu-phe + 0.001 * formiminoglutamic acid

In the discovery set, the model constructed with the 11 metabolites yielded an AUC of 0.824 for predicting 28-day progression risk to ACLF of non-ACLF patients; in the validation set, the AUC for predicting 28-day progression risk to ACLF of non-ACLF patients was 0.751.

(6) Model with 10-metabolite combination

Based on the 11 metabolites, any one metabolite was excluded, with a total of 11 exclusion methods. The remaining 10 metabolites were used to construct prediction models for 28-day progression risk to ACLF of non-ACLF patients. After comparing the prediction models constructed by 11 exclusion methods, the prediction model with the best combined performance and the highest AUC was selected, yielding the following 10-metabolite combination: ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glu-phe

The model equation was:

P = -2.899 + 0.816 * ureidopropionic acid - 0.512 * γ-CEHC - 1.015 * thyroxine + 0.396 * pipecolic acid + 0.362 * 3-OH butyrylcarnitine - 0.929 * 2-hydroxydadipic acid + 0.166 * estrone sulfate + 0.238 * hydroxyproline - 0.076 * quinolinic acid + 0.071 * γ-glu-phe

In the discovery set, the model constructed with the 10 metabolites yielded an AUC of 0.824 for predicting 28-day progression risk to ACLF of non-ACLF patients; in the validation set, the AUC for predicting 28-day progression risk to ACLF of non-ACLF patients was 0.751.

(7) Model with 9-metabolite combination

Based on the 10 metabolites, any one metabolite was excluded, with a total of 10 exclusion methods. The remaining 9 metabolites were used to construct prediction models for 28-day progression risk to ACLF of non-ACLF patients. After comparing the prediction models constructed by 10 exclusion methods, the prediction model with the best combined performance and the highest AUC was selected, yielding the following 9-metabolite combination: ureidopropionic acid, γ-CEHC, thyroxine, pipecolic acid, 3-OH butyrylcarnitine, 2-hydroxydadipic acid, estrone sulfate, hydroxyproline, and quinolinic acid

The model equation was:

P = -2.647 + 0.828 * ureidopropionic acid - 0.512 * γ-CEHC - 1.039 * thyroxine + 0.388 * pipecolic acid + 0.347 * 3-OH butyrylcarnitine - 0.926 * 2-hydroxydadipic acid + 0.163 * estrone sulfate + 0.241 * hydroxyproline - 0.047 * quinolinic acid

In the discovery set, the model constructed with the 9 metabolites yielded an AUC of 0.825 for predicting 28-day progression risk to ACLF of non-ACLF patients; in the validation set, the AUC for predicting 28-day progression risk to ACLF of non-ACLF patients was 0.750.

(8) Model with 8-metabolite combination

Based on the 9 metabolites, any one metabolite was excluded, with a total of 9 exclusion methods. The remaining 8 metabolites were used to construct prediction models for 28-day progression risk to ACLF of non-ACLF patients. After comparing the prediction models constructed by 9 exclusion methods, the prediction model with the best combined performance and the highest AUC was selected, yielding the following 8-metabolite combination: ureidopropionic acid, γ-CEHC, thyroxine, pipecolic acid, 3-OH butyrylcarnitine, 2-hydroxydadipic acid, estrone sulfate, and hydroxyproline

The model equation was:

P = -2.647 + 0.816 * ureidopropionic acid - 0.513 * γ-CEHC - 1.049 * thyroxine + 0.393 * pipecolic acid + 0.34 * 3-OH butyrylcarnitine - 0.939 * 2-hydroxydadipic acid + 0.164 * estrone sulfate + 0.23 * hydroxyproline

In the discovery set, the model constructed with the 8 metabolites yielded an AUC of 0.825 for predicting 28-day progression risk to ACLF of non-ACLF patients; in the validation set, the AUC for predicting 28-day progression risk to ACLF of non-ACLF patients was 0.750.

(9) Model with 7-metabolite combination

Based on the 8 metabolites, any one metabolite was excluded, with a total of 8 exclusion methods. The remaining 7 metabolites were used to construct prediction models for 28-day progression risk to ACLF of non-ACLF patients. After comparing the prediction models constructed by 8 exclusion methods, the prediction model with the best combined performance and the highest AUC was selected, yielding the following 7-metabolite combination: ureidopropionic acid, γ-CEHC, thyroxine, pipecolic acid, 3-OH butyrylcarnitine, 2-hydroxydadipic acid, and estrone sulfate
The model equation was:

P = -0.998 + 0.856 * ureidopropionic acid - 0.502 * γ-CEHC - 1.104 * thyroxine + 0.408 * pipecolic acid + 0.325 * 3-OH butyrylcarnitine + -0.916 * 2-hydroxydadipic acid + 0.154 * estrone sulfate

In the discovery set, the model constructed with the 7 metabolites yielded an AUC of 0.827 for predicting 28-day progression risk to ACLF of non-ACLF patients; in the validation set, the AUC for predicting 28-day progression risk to ACLF of non-ACLF patients was 0.748.

(10) Model with 6-metabolite combination

Based on the 7 metabolites, any one metabolite was excluded, with a total of 7 exclusion methods. The remaining 6 metabolites were used to construct prediction models for 28-day progression risk to ACLF of non-ACLF patients. After comparing the prediction models constructed by 7 exclusion methods, the prediction model with the best combined performance and the highest AUC was selected, yielding the following 6-metabolite combination: ureidopropionic acid, γ-CEHC, thyroxine, pipecolic acid, 3-OH butyrylcarnitine, and 2-hydroxydadipic acid
The model equation was:

P = -1.935 + 0.919 * ureidopropionic acid - 0.537 * γ-CEHC - 1.018 * thyroxine + 0.464 * pipecolic acid + 0.296 * 3-OH butyrylcarnitine - 0.867 * 2-hydroxydadipic acid

In the discovery set, the model constructed with the 6 metabolites yielded an AUC of 0.823 for predicting 28-day progression risk to ACLF of non-ACLF patients; in the validation set, the AUC for predicting 28-day progression risk to ACLF of non-ACLF patients was 0.746.

(11) Model with 5-metabolite combination

Based on the 6 metabolites, any one metabolite was excluded, with a total of 6 exclusion methods. The remaining 5 metabolites were used to construct prediction models for 28-day progression risk to ACLF of non-ACLF patients. After comparing the prediction models constructed by 6 exclusion methods, the prediction model with the best combined performance and the highest AUC was selected, yielding the following 5-metabolite combination: ureidopropionic acid, γ-CEHC, thyroxine, pipecolic acid, and 3-OH butyrylcarnitine
The model equation was:

P = -2.077 + 0.745 * ureidopropionic acid - 0.539 * γ-CEHC - 0.806 * thyroxine + 0.191 * pipecolic acid + 0.088 * 3-OH butyrylcarnitine

In the discovery set, the model constructed with the 5 metabolites yielded an AUC of 0.803 for predicting 28-day progression risk to ACLF of non-ACLF patients; in the validation set, the AUC for predicting 28-day progression risk to ACLF of non-ACLF patients was 0.743.

(12) Model with 4-metabolite combination

Based on the 5 metabolites, any one metabolite was excluded, with a total of 5 exclusion methods. The remaining 4 metabolites were used to construct prediction models for 28-day progression risk to ACLF of non-ACLF patients. After comparing the prediction models constructed by 5 exclusion methods, the prediction model with the best combined performance and the highest AUC was selected, yielding the following 4-metabolite combination: ureidopropionic acid, γ-CEHC, thyroxine, and pipecolic acid
The model equation was:

P = -1.878 + 0.77 * ureidopropionic acid - 0.54 * γ-CEHC - 0.827 * thyroxine + 0.188 * pipecolic acid

In the discovery set, the model constructed with the 4 metabolites yielded an AUC of 0.801 for predicting 28-day progression risk to ACLF of non-ACLF patients; in the validation set, the AUC for predicting 28-day progression risk to ACLF of non-ACLF patients was 0.740.

(13) Model with 3-metabolite combination

Based on the 4 metabolites, any one metabolite was excluded, with a total of 4 exclusion methods. The remaining 3 metabolites were used to construct prediction models for 28-day progression risk to ACLF of non-ACLF patients. After comparing the prediction models constructed by 4 exclusion methods, the two prediction models with the best combined performance and the highest AUC were selected, yielding the two following 3-metabolite combinations:

① ureidopropionic acid, γ-CEHC, and thyroxine

The model equation was:

$$P = -0.983 + 0.845 * \text{ureidopropionic acid} - 0.565 * \gamma\text{-CEHC} - 0.835 * \text{thyroxine}$$

In the discovery set, the model constructed with the 3 metabolites yielded an AUC of 0.800 for predicting 28-day progression risk to ACLF of non-ACLF patients; in the validation set, the AUC for predicting 28-day progression risk to ACLF of non-ACLF patients was 0.734.

② ureidopropionic acid, γ-CEHC, and pipecolic acid

The model equation was:

$$P = -4.744 + 1.015 * \text{ureidopropionic acid} - 0.584 * \gamma\text{-CEHC} + 0.122 * \text{pipecolic acid};$$

In the discovery set, the model constructed with the 3 metabolites yielded an AUC of 0.789 for predicting 28-day progression risk to ACLF of non-ACLF patients; in the validation set, the AUC for predicting 28-day progression risk to ACLF of non-ACLF patients was 0.742.

(14) Model with 2-metabolite combination

Based on the 3 metabolites, any one metabolite was excluded, with a total of 3 exclusion methods. The remaining 2 metabolites were used to construct prediction models for 28-day progression risk to ACLF of non-ACLF patients. After comparing the prediction models constructed by 3 exclusion methods, the prediction model with the best combined performance and the highest AUC was selected, yielding the following 2-metabolite combination:
ureidopropionic acid and γ-CEHC
The model equation was:

$$P = -4.803 + 0.930 * \text{ureidopropionic acid} - 0.563 * \gamma\text{-CEHC}$$

In the discovery set, the model constructed with the 2 metabolites yielded an AUC of 0.786 for predicting 28-day progression risk to ACLF of non-ACLF patients; in the validation set, the AUC for predicting 28-day progression risk to ACLF of non-ACLF patients was 0.745.
However, compared to 1-metabolite models, the model constructed with the 2 metabolites for predicting 28-day progression risk to ACLF of non-ACLF patients indeed exhibits significantly enhanced predictive performance, and this combination has the two optimal metabolites selected from the 15 metabolites. The AUC for predicting 28-day progression risk to ACLF of non-ACLF patients exceeded 0.7 in both the discovery set and the validation set.
In summary, as the number of metabolite types decreases, during the prediction of the constructed prediction models for 28-day progression risk to ACLF of non-ACLF patients, the AUC values exhibit a slight decrease in both the discovery set and the validation set. However, employing excessive metabolites increases the burden on the detection process. After evaluating the trade-offs, models constructed with 2 or 3 metabolites are preferred, requiring only 3 indicators, ureidopropionic acid, γ-CEHC, and thyroxine, or ureidopropionic acid, γ-CEHC, and pipecolic acid to be detected, or requiring only 2 indicators, ureidopropionic acid and γ-CEHC, to be detected.

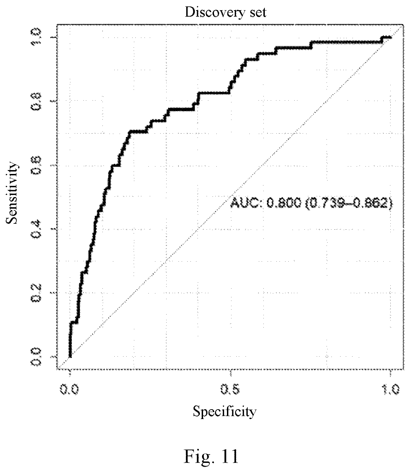

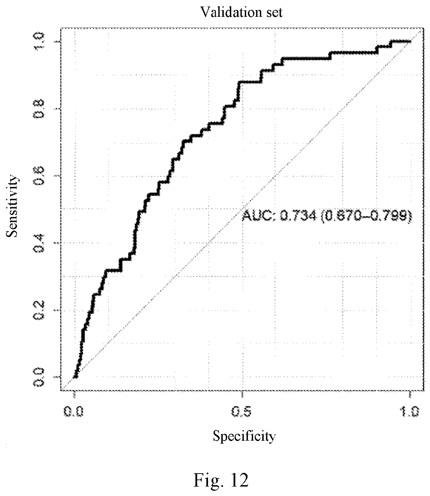

The model ① with 3 detected indicators, ureidopropionic acid, γ-CEHC, and thyroxine, maintained higher AUC values in both the discovery set and the validation set. In the discovery set, the AUC for predicting 28-day progression risk to ACLF of non-ACLF patients was 0.800 (Fig. 11). This result was precisely replicated in the validation set, with an AUC of 0.734 (Fig. 12). Research demonstrates that for the model ① constructed with 3 metabolites, prediction value P greater than 0.150 indicates predicting a high risk of 28-day progression to ACLF of non-ACLF patients; prediction value P less than 0.150 indicates predicting a low risk of 28-day progression to ACLF of non-ACLF patients.

For the model constructed with 2 metabolites (ureidopropionic acid and γ-CEHC), prediction value P greater than 0.123 indicates predicting a high risk of 28-day progression to ACLF of non-ACLF patients; prediction value P less than 0.123 indicates predicting a low risk of 28-day progression to ACLF of non-ACLF patients.

Furthermore, in this example, the screened 2-metabolite model (P = -4.803 + 0.930 * ureidopropionic acid - 0.563 * γ-CEHC) and 3-metabolite model (P = -0.983 + 0.845 * ureidopropionic acid - 0.565 * γ-CEHC - 0.835 * thyroxine, or P = -4.744 + 1.015 * ureidopropionic acid - 0.584 * γ-CEHC + 0.122 * pipecolic acid) also have a good effect of predicting the 180-day progression risk to ACLF of non-ACLF patients, with AUC values exceeding 0.6.

Example 7. Optimization of Prediction Models

(1) Optimization of 4-Metabolite Combination Model for Predicting 90-Day Mortality of ACLF Patients

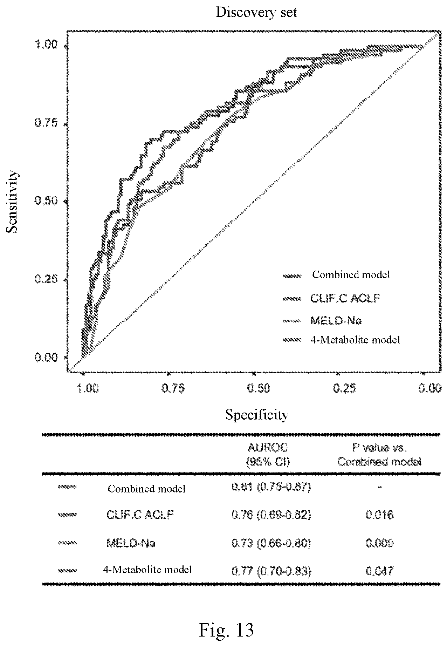

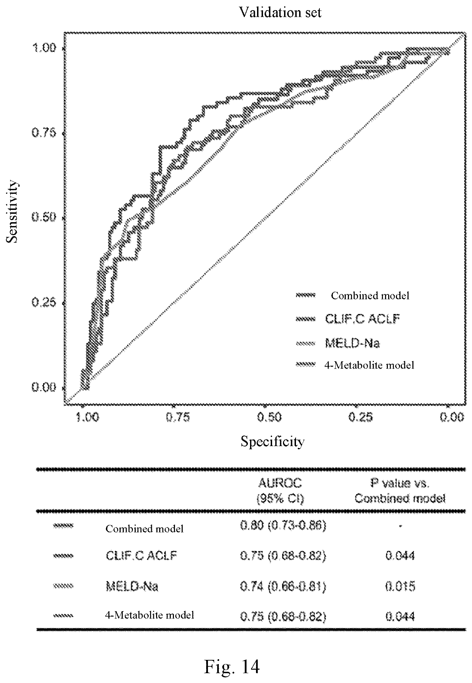

Subsequently, in both the discovery set and the validation set, for the 4-metabolite combination in Example 5, based on quantitative data generated by targeted assays, the prognostic performance of the 4-metabolite model for predicting 90-day mortality of ACLF patients was reassessed using 1. a combined model (TB, INR, N/L ratio, and age was added to the 4-metabolite combination, and the model equation was: P = 9.143 + 2.196 * age + 1.944 * INR - 0.432 * creatine - 6.076 * Na + 0.052 *ALB + 0.441 * N/L ratio + 0.218 * overt HE + 0.687 * pipecolic acid + 1.166 * NAAG + 0.143 * ureidopropionic acid - 0.119 * formiminoglutamic acid); 2. CLIF-C ACLF; 3, MELD-Na; 4. the 4-metabolite model, with the results shown in Figs. 13 and 14.

The AUCs for the 4-metabolite model (pipecolic acid, NAAG, ureidopropionic acid, and formiminoglutamic acid) were 0.77 and 0.75, respectively (Figs. 13 and 14). This result was higher than the AUC based on untargeted metabolomics data, likely due to the improved data quality from targeted analysis. However, these results were not significantly (p < 0.05) superior to the CLIF-C ACLF and MELD-Na scores. Subsequently, whether the metabolite model could be improved in combination with clinical parameters was assessed. By adding TB, INR, N/L ratio, and age to the 4-metabolite combination, using logistic regression analysis, it was found that the 4-metabolite combination, added with TB, INR, N/L ratio, and age, achieved an increased AUC of 0.81. This was significantly superior to the CLIF-C ACLF (AUC of 0.76), MELD-Na (AUC of 0.43), and the 4-metabolite model alone (Fig. 13). The performance of this model was well validated in the validation set (Fig. 14).

(2) Optimization of 3-Metabolite Combination Model for Predicting 28-Day Progression Risk to ACLF of non-ACLF Patients

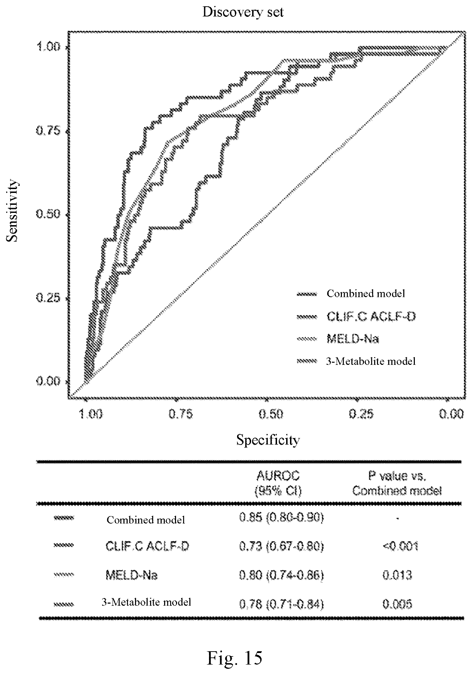

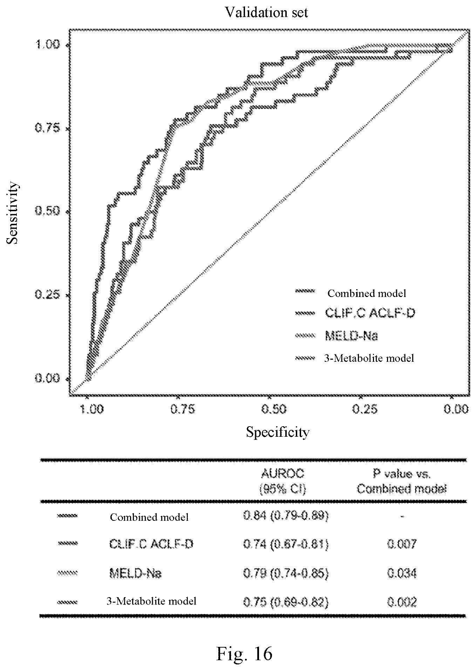

For pre-ACLF diagnosis, in both the discovery set and the validation set, for the 3-metabolite combination (model ①) in Example 6, based on quantitative data generated by targeted assays, the prognostic performance of the 3-metabolite model for predicting 28-Day Progression Risk to ACLF of non-ACLF patients was reassessed using 1. a combined model (TB, INR, and Cirrhosis was added to the 3-metabolite combination, and the model equation was: P = -6.916 + 0.173 * TB + 1.619 * INR + 0.375 * Cirrhosis + 0.735 * ureidopropionic acid - 0.101 * CEHC + 0.099 * thyroxine); 2. CLIF-C ACLF; 3, MELD-Na; 4. the 3-metabolite combined model, with the results shown in Figs. 15 and 16.

The AUCs for the 3-metabolite model (ureidopropionic acid, γ-CEHC, and thyroxine) based on targeted assay data were 0.78 and 0.75 in the discovery set and the validation set, respectively. These results were similar to those of the CLIF-C ACLF development score and the MELD-Na score. However, by adding TB, INR, and Cirrhosis to the metabolite combination, it was found that the AUC of the combination increased to 0.85. This was significantly higher than the CLIF-C ACLF (AUC of 0.73), MELD-Na (AUC of 0.8), and the 3-metabolite combination alone (Fig. 15). The performance of this model was well validated in the validation set (Fig. 16).

Example 8. Construction of Models with Newly Discovered Metabolic Biomarkers of the present Application and Combination of Other Clinical Indicators

In this example, appropriate indicators were selected from clinical indicators including age, INR, CR, Sodium, ALB, N/L ratio, overt HE, TB, and Cirrhosis, to be combined with the 15 metabolites screened in Example 1 to construct models, where the combination of age, INR, CR, Sodium, ALB, N/L ratio, and overt HE, or the combination of age, TB, INR, and N/L ratio was used to construct a model for predicting short-term mortality of ACLF patients, and the combination of TB, INR, and Cirrhosis is used to construct a model for predicting short-term progression risk to ACLF of non-ACLF patients.

1. Model for Predicting 90-Day Mortality of ACLF Patients

1) The models constructed with the combination of age, INR, CR, Sodium, ALB, N/L ratio, and overt HE and any one of the 15 metabolites for predicting 90-day mortality of ACLF patients are shown in Table 7.

Table 7. Models Constructed with the Combination of age, INR, CR, Sodium, ALB, N/L ratio, and overt HE and Any One of Metabolites

| Metabolite | Model | AUC value (Discovery set) | AUC value (Validation set) |
|---|---|---|---|
| None | P = 53.596 + 1.61 * age + 2.445 * INR + 0.138 * CR + - 12.643 * Sodium + -0.34 * ALB + 0.624 * N/L ratio + 0.151 * overt HE | 0.794 | 0.74 |
| 2-Hydroxydadipic acid | P = 40.162 + 1.707 * age + 2.481 * INR - 0.066 * CR - 10.343 * Sodium - 0.255 * ALB + 0.516 * N/L ratio + 0.219 * overt HE + 0.493 * 2-hvdroxvdadipic acid | 0.8 | 0.757 |
| Hexadecanedioic acid | P = 50.243 + 1.681 * age + 2.319 * INR + 0.163 * CR - 12.099 * Sodium - 0.419 * ALB + 0.572 * N/L ratio + 0.217 * overt HE + 0.179 * hexadecanedioic acid | 0.797 | 0.745 |
| Hydroxyproline | P = 42.412 + 1.528 * age + 2.223 * INR - 0.126 * CR - 11.274 * Sodium - 0.207 * ALB + 0.69 * N/L ratio + 0.271 * overt HE + 0.553 * hydroxyproline | 0.806 | 0.756 |
| Methionine sulfoxide | P = 49.131 + 1.642 * age + 2.196 * INR + 0.094 * CR - 12.032 * Sodium - 0.506 * ALB + 0.606 * N/L ratio + 0.238 * overt HE + 0.27 * methionine sulfoxide | 0.797 | 0.748 |
| 3-OH butyrylcarnitine | P = 47.769 + 1.633 * age + 2.622 * INR + 0.018 * CR - 11.847 * Sodium - 0.05 * ALB + 0.528 * N/L ratio + 0.038 * overt HE + 0.314 * 3-OH butyrylcarnitine | 0.803 | 0.745 |
| Pipecolic acid | P = 36.39 + 1.759 * age + 1.931 * INR - 0.096 * CR - 10.227 * Sodium - 0.452 * ALB + 0.574 * N/L ratio + 0.326 * overt HE + 0.826 * pipecolic acid | 0.81 | 0.767 |
| Estrone sulfate | P = 44.712 + 1.909 * age + 2.035 * INR + 0.163 * CR - 10.904 * Sodium - 0.583 * ALB + 0.556 * N/L ratio + 0.257 * overt HE + 0.3 * estrone sulfate | 0.807 | 0.762 |
| NAAG | P = 19.154 + 2.191 * age + 2.468 * INR - 0.35 * CR - 7.406 * Sodium + 0.18 * ALB + 0.474 * N/L ratio + 0.056 * overt HE + 1.326 * NAAG | 0.818 | 0.755 |
| Quinolinic acid | P = 52.178 + 1.601 * age + 2.467 * INR + 0.064 * CR - 12.479 * Sodium - 0.262 * ALB + 0.605 * N/L ratio + 0.165 * overt HE + 0.08 * quinolinic acid | 0.794 | 0.743 |
| Ureidopropionic acid | P = 38.958 + 1.608 * age + 2.097 * INR - 0.125 * CR - 10.352 * Sodium - 0.06 * ALB + 0.57 * N/L ratio + 0.279 * overt HE + 0.513 * ureidopropionic acid | 0.807 | 0.775 |
| γ-CEHC | P = 49.875 + 1.726 * age + 2.238 * INR + 0.278 * CR - 11.782 * Sodium - 0.469 * ALB + 0.62 * N/L ratio + 0.229 * overt HE - 0.261 * γ-CEHC | 0.802 | 0.75 |

(continued)

| Metabolite | Model | AUC value (Discovery set) | AUC value (Validation set) |
|---|---|---|---|
| Thyroxine | P = 59.92 + 1.527 * age + 2.513 * INR + 0.204 * CR - 14.043 * Sodium - 0.579 * ALB + 0.612 * N/L ratio + 0.24 * overt HE + 0.415 * thyroxine | 0.795 | 0.745 |
| Formiminoglutamic acid | P = 53.57 + 1.611 * age + 2.445 * INR + 0.138 * CR - 12.639 * Sodium - 0.34 * ALB + 0.624 * N/L ratio + 0.151 * overt HE + 0.001 * formiminoglutamic acid | 0.794 | 0.74 |
| $\gamma$-glu-phe | P = 53.366 + 1.628 * age + 2.502 * INR + 0.179 * CR - 12.49 * Sodium - 0.412 * ALB + 0.632 * N/L ratio + 0.192 * overt HE - 0.093 * $\gamma$-glu-phe | 0.795 | 0.746 |
| Bilirubin | P = 57.748 + 1.792 * age + 2.539 * INR + 0.097 * CR - 13.68 * Sodium - 0.323 * ALB + 0.568 * N/L ratio + 0.171 * overt HE + 0.102 * bilirubin | 0.802 | 0.742 |

It can be seen from Table 7 that the models constructed with any one of the 15 newly discovered metabolic biomarkers of the present application and the combination of age, INR, CR, Sodium, ALB, N/L ratio, and overt HE significantly enhance the AUC values in both the discovery set and the validation set compared to models without metabolites. This demonstrates that the 15 metabolites provided by the present application have tangible effects in predicting short-term mortality of ACLF patients. Moreover, it should be understood that based on the combined models provided in Table 7, further increasing the number of metabolites could further improve the effect of predicting short-term mortality of ACLF patients, and AUC values could be further elevated.

2) The models constructed with the combination of age, TB, INR, and N/L ratio and any one of the 15 metabolites for predicting 90-day mortality of ACLF patients are shown in Table 8.

Table 8. Models Constructed with the Combination of age, TB, INR, and N/L ratio and Any One of Metabolites

| Metabolite | Model | AUC value (Discovery set) | AUC value (Validation set) |
|---|---|---|---|
| None | P = -11.878 + 1.863 * age + 0.468 * TB + 2.296 * INR + 0.703 * N/L ratio | 0.783 | 0.743 |
| 2-Hydroxydadipic acid | P = -13.415 + 1.863 * age + 0.407 * TB + 2.355 * INR + 0.525 * N/L ratio + 0.597 * 2-hydroxydadipic acid | 0.799 | 0.757 |
| Hexadecanedioic acid | P = -12.187 + 1.879 * age + 0.444 * TB + 2.259 * INR + 0.685 * N/L ratio + 0.078 * hexadecanedioic acid | 0.785 | 0.748 |
| Hydroxyproline | P = -15.791 + 1.636 * age + 0.446 * TB + 2.081 * INR + 0.714 * N/L ratio + 0.633 * hydroxyproline | 0.797 | 0.753 |
| Methionine sulfoxide | P = -12.556 + 1.836 * age + 0.409 * TB + 2.216 * INR + 0.701 * N/L ratio + 0.129 * methionine sulfoxide | 0.785 | 0.742 |
| 3-OH butyrylcarnitine | P = -14.244 + 1.995 * age + 0.638 * TB + 2.461 * INR + 0.446 * N/L ratio + 0.609 * 3-OH butyrylcarnitine | 0.817 | 0.746 |
| Pipecolic acid | P = -15.999 + 1.819 * age + 0.275 * TB + 1.951 * INR + 0.628 * N/L ratio + 0.767 * pipecolic acid | 0.8 | 0.766 |

(continued)

| Metabolite | Model | AUC value (Discovery set) | AUC value (Validation set) |
|---|---|---|---|
| Estrone sulfate | P = -12.042 + 1.989 * age + 0.319 * TB + 2.062 * INR + 0.668 * N/L ratio + 0.227 * estrone sulfate | 0.793 | 0.76 |
| NAAG | P = -18.925 + 2.439 * age + 0.473 * TB + 2.287 * INR + 0.425 * N/L ratio + 1.416 * NAAG | 0.827 | 0.752 |
| Quinolinic acid | P = -12.669 + 1.761 * age + 0.463 * TB + 2.381 * INR + 0.608 * N/L ratio + 0.263 * quinolinic acid | 0.792 | 0.75 |
| Ureidopropionic acid | P = -13.137 + 1.669 * age + 0.322 * TB + 2.05 * INR + 0.611 * N/L ratio + 0.508 * ureidopropionic acid | 0.797 | 0.775 |
| $\gamma$-CEHC | P = -11.395 + 1.879 * age + 0.364 * TB + 2.208 * INR + 0.724 * N/L ratio + -0.165 * $\gamma$-CEHC | 0.785 | 0.753 |
| Thyroxine | P = -11.324 + 1.906 * age + 0.484 * TB + 2.246 * INR + 0.69 * N/L ratio + -0.18 * thyroxine | 0.783 | 0.747 |
| Formiminoglutamic acid | P = -11.89 + 1.864 * age + 0.466 * TB + 2.297 * INR + 0.702 * N/L ratio + 0.004 * formiminoglutamic acid | 0.785 | 0.744 |
| $\gamma$-glu-phe | P = -11.795 + 1.869 * age + 0.464 * TB + 2.319 * INR + 0.709 * N/L ratio + -0.027 * $\gamma$-glu-phe | 0.785 | 0.744 |
| Bilirubin | P = -12.193 + 1.936 * age + 0.44 * TB + 2.363 * INR + 0.674 * N/L ratio + 0.057 * bilirubin | 0.784 | 0.742 |

It can be seen from Table 8 that the models constructed with any one of the 15 newly discovered metabolic biomarkers of the present application and the combination of age, TB, INR, and N/L ratio significantly enhance the AUC values in both the discovery set and the validation set compared to models without metabolites. This demonstrates that the 15 metabolites provided by the present application have tangible effects in predicting short-term mortality of ACLF patients. Moreover, it should be understood that based on the combined models provided in Table 8, further increasing the number of metabolites could further improve the effect of predicting short-term mortality of ACLF patients, and AUC values could be further elevated.

3) The models constructed with the combination of TB, INR, and Cirrhosis and any one of the 15 metabolites for predicting 28-day progression risk to ACLF of non-ACLF patients are shown in Table 9.

Table 9. Models Constructed with the Combination of TB, INR, and Cirrhosis and Any One of Metabolites

| Metabolite | Model | AUC value (Discovery set) | AUC value (Validation set) |
|---|---|---|---|
| None | P = -6.323 + 1.327 * TB + 1.768 * INR + 2.111 * Cirrhosis | 0.841 | 0.81 |
| 2-Hydroxydadipic acid | P = -5.597 + 1.384 * TB + 1.85 * INR + 2.146 * Cirrhosis + -0.337 * 2-hydroxydadipic acid | 0.842 | 0.802 |

(continued)

| Metabolite | Model | AUC value (Discovery set) | AUC value (Validation set) |
|---|---|---|---|
| Hexadecanedioic acid | P = -6.937 + 1.213 * TB + 1.698 * INR + 2.143 * Cirrhosis + 0.205 * hexadecanedioic acid | 0.844 | 0.812 |
| Hydroxyproline | P = -8.759 + 1.328 * TB + 1.682 * INR + 2.063 * Cirrhosis + 0.325 * hydroxyproline | 0.843 | 0.81 |
| Methionine sulfoxide | P = -5.699 + 1.341 * TB + 1.89 * INR + 2.098 * Cirrhosis + -0.093 * methionine sulfoxide | 0.841 | 0.818 |
| 3-OH butyrylcarnitine | P = -7.252 + 1.338 * TB + 1.723 * INR + 2.138 * Cirrhosis + 0.371 * 3-OH butyrylcarnitine | 0.847 | 0.818 |
| Pipecolic acid | P = -7.252 + 1.281 * TB + 1.633 * INR + 2.134 * Cirrhosis + 0.169 * pipecolic acid | 0.842 | 0.813 |
| Estrone sulfate | P = -6.38 + 1.379 * TB + 1.78 * INR + 2.075 * Cirrhosis + - 0.064 * estrone sulfate | 0.841 | 0.817 |
| NAAG | P = -9.194 + 1.285 * TB + 1.562 * INR + 2.204 * Cirrhosis + 0.871 * NAAG | 0.847 | 0.814 |
| Quinolinic acid | P = -6.486 + 1.321 * TB + 1.773 * INR + 2.107 * Cirrhosis + 0.038 * quinolinic acid | 0.841 | 0.819 |
| Ureidopropionic acid | P = -8.638 + 1.195 * TB + 1.201 * INR + 2.085 * Cirrhosis + 0.584 * ureidopropionic acid | 0.852 | 0.819 |
| $\gamma$-CEHC | P = -4.814 + 1.114 * TB + 1.356 * INR + 2.288 * Cirrhosis + -0.436 * $\gamma$-CEHC | 0.857 | 0.814 |
| Thyroxine | P = -2.533 + 1.409 * TB + 1.157 * INR + 1.969 * Cirrhosis + -0.881 * thyroxine | 0.85 | 0.797 |
| Formiminoglutamic acid | P = -6.352 + 1.323 * TB + 1.759 * INR + 2.116 * Cirrhosis + 0.011 * formiminoglutamic acid | 0.841 | 0.817 |
| $\gamma$-glu-phe | P = -6.762 + 1.331 * TB + 1.653 * INR + 2.09 * Cirrhosis + 0.118 * $\gamma$-glu-phe | 0.841 | 0.811 |
| Bilirubin | P = -5.83 + 1.483 * TB + 1.969 * INR + 2.064 * Cirrhosis + -0.299 * bilirubin | 0.846 | 0.814 |

It can be seen from Table 9 that the models constructed with any one of the 15 newly discovered metabolic biomarkers of the present application and the combination of TB, INR, and Cirrhosis significantly enhance the AUC values in both the discovery set and the validation set compared to models without metabolites. This demonstrates that the 15 metabolites provided by the present application have tangible effects in predicting short-term progression risk to ACLF of non-ACLF patients. Moreover, it should be understood that based on the combined models provided in Table 9, further increasing the number of metabolites could further improve the effect of predicting short-term progression risk to ACLF of non-ACLF patients, and AUC values could be further elevated.

**Claims**

1. Use of a biomarker for preparing a reagent for predicting a mortality risk or a probability of survival of an acute-on-chronic liver failure (ACLF) patient, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

2. The use according to claim 1, wherein the biomarker comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and γ-CEHC; or, the biomarker comprises pipecolic acid, NAAG, ureidopropionic acid, formiminoglutamic acid, γ-CEHC, and γ-glu-phe; or, the biomarker comprises pipecolic acid, NAAG, ureidopropionic acid, formiminoglutamic acid, γ-CEHC, γ-glu-phe, and 3-OH butyrylcarnitine; or, the biomarker comprises pipecolic acid, NAAG, ureidopropionic acid, formiminoglutamic acid, γ-CEHC, γ-glu-phe, 3-OH butyrylcarnitine, and estrone sulfate; or, the biomarker comprises pipecolic acid, NAAG, ureidopropionic acid, formiminoglutamic acid, γ-CEHC, γ-glu-phe, 3-OH butyrylcarnitine, estrone sulfate, and hydroxyproline; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

3. The use according to claim 1, wherein the biomarker comprises one or more of: pipecolic acid, NAAG, ureidopropionic acid, and formiminoglutamic acid.

4. The use according to claim 3, wherein the biomarker comprises one or more of: pipecolic acid, NAAG, and ureidopropionic acid.

5. The use according to claim 1, wherein the reagent is used for detecting the biomarker in a blood, serum, plasma, or whole blood sample from the ACLF patient; and the reagent is used for detecting a presence or absence, relative abundance, or concentration of the biomarker in the sample.

6. The use according to claim 1, wherein the biomarker further comprises one or more of age, international normalized ratio (INR), creatine, Sodium, albumin, neutrophil/lymphocyte count ratio, overt hepatic encephalopathy, and total bilirubin (TB).

7. The use according to claim 1, comprising predicting a mortality risk or a probability of survival of the ACLF patient within 360 days.

8. The use according to claim 1, comprising predicting a mortality risk or a probability of survival of the ACLF patient within 90 days.

9. Use of a biomarker for preparing a reagent for predicting progression to acute-on-chronic liver failure (ACLF) of a non-ACLF patient, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

10. The use according to claim 9, wherein the biomarker comprises ureidopropionic acid and γ-CEHC; or, the biomarker comprises ureidopropionic acid, γ-CEHC, and thyroxine; or, the biomarker comprises ureidopropionic acid, γ-CEHC, and pipecolic acid; or, the biomarker comprises ureidopropionic acid, γ-CEHC, thyroxine, and pipecolic acid; or, the biomarker comprises ureidopropionic acid, γ-CEHC, thyroxine, pipecolic acid, and 3-OH butyrylcarnitine; or, the biomarker comprises ureidopropionic acid, γ-CEHC, thyroxine, pipecolic acid, 3-OH butyrylcarnitine, and 2-hydroxydadipic acid; or, the biomarker comprises ureidopropionic acid, γ-CEHC, thyroxine, pipecolic acid, 3-OH butyrylcarnitine, 2-hydroxydadipic acid, and estrone sulfate; or, the biomarker comprises ureidopropionic acid, γ-CEHC, thyroxine, pipecolic acid, 3-OH butyrylcarnitine, 2-hydroxydadipic acid, estrone sulfate, and hydroxyproline; or, the biomarker comprises ureidopropionic acid, γ-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glu-phe; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and NAAG.

11. The use according to claim 9, wherein the biomarker comprises one or more of: ureidopropionic acid, γ-CEHC, and thyroxine; or ureidopropionic acid, γ-CEHC, and pipecolic acid.

12. The use according to claim 9, wherein the biomarker comprises one or more of: ureidopropionic acid and γ-CEHC.

13. The use according to claim 9, comprising predicting progression to ACLF of the non-ACLF patient within 180 days.

14. The use according to claim 9, comprising predicting progression to ACLF of the non-ACLF patient within 28 days.

15. The use according to claim 9, wherein the biomarker further comprises one or more of total bilirubin (TB), international normalized ratio (INR), and Cirrhosis.

16. A kit for predicting a mortality risk or a probability of survival of an acute-on-chronic liver failure (ACLF) patient, comprising a detection reagent for a biomarker for the use according to any one of claims 1-8.

17. The kit according to claim 16, wherein the detection reagent for the biomarker comprises a standard for the biomarker.

18. A kit for predicting progression to acute-on-chronic liver failure (ACLF) of a non-ACLF patient, comprising a detection reagent for a biomarker for the use according to any one of claims 9-15.

19. The kit according to claim 18, wherein the detection reagent for the biomarker comprises a standard for the biomarker.

20. A biomarker combination for predicting a mortality risk or a probability of survival of an acute-on-chronic liver failure (ACLF) patient, comprising the following biomarkers: pipecolic acid, N-acetylaspartylglutamate (NAAG), and ureidopropionic acid; or pipecolic acid, NAAG, ureidopropionic acid, and formiminoglutamic acid; or pipecolic acid, NAAG, ureidopropionic acid, formiminoglutamic acid, and γ-carboxyethylhydroxychroman (γ-CEHC); or pipecolic acid, NAAG, ureidopropionic acid, formiminoglutamic acid, γ-CEHC, and γ-glutamylphenylalanine (γ-glu-phe); or pipecolic acid, NAAG, ureidopropionic acid, formiminoglutamic acid, γ-CEHC, γ-glu-phe, and 3-OH butyrylcarnitine; or pipecolic acid, NAAG, ureidopropionic acid, formiminoglutamic acid, γ-CEHC, γ-glu-phe, 3-OH butyrylcarnitine, and estrone sulfate; or pipecolic acid, NAAG, ureidopropionic acid, formiminoglutamic acid, γ-CEHC, γ-glu-phe, 3-OH butyrylcarnitine, estrone sulfate, and hydroxyproline; or pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

21. The biomarker combination according to claim 20, comprising the following biomarkers: pipecolic acid, NAAG, ureidopropionic acid, and formiminoglutamic acid; or pipecolic acid, NAAG, and ureidopropionic acid.

22. The biomarker combination according to claim 20, further comprising one or more of age, international normalized ratio (INR), creatine, Sodium, albumin, neutrophil/lymphocyte count ratio, overt hepatic encephalopathy, and total bilirubin (TB).

23. A biomarker combination for predicting progression to acute-on-chronic liver failure (ACLF) of a non-ACLF patient, comprising the following biomarkers: ureidopropionic acid and γ-carboxyethylhydroxychroman (γ-CEHC); or ureidopropionic acid, γ-CEHC, and thyroxine; or ureidopropionic acid, γ-CEHC, and pipecolic acid; or ureidopropionic acid, γ-CEHC, thyroxine, and pipecolic acid; or ureidopropionic acid, γ-CEHC, thyroxine, pipecolic acid, and 3-OH butyrylcarnitine; or ureidopropionic acid, γ-CEHC, thyroxine, pipecolic acid, 3-OH butyrylcarnitine, and 2-hydroxydadipic acid; or ureidopropionic acid, γ-CEHC, thyroxine, pipecolic acid, 3-OH butyrylcarnitine, 2-hydroxydadipic acid, and estrone sulfate; or ureidopropionic acid, γ-CEHC, thyroxine, pipecolic acid, 3-OH butyrylcarnitine, 2-hydroxydadipic acid, estrone sulfate, and hydroxyproline; or ureidopropionic acid, γ-CEHC, thyroxine, pipecolic acid, 3-OH butyrylcarnitine, 2-hydroxydadipic acid, estrone sulfate, hydroxyproline, and quinolinic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glutamylphenylalanine (γ-glu-phe); or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and N-acetylaspartylglutamate (NAAG).

24. The biomarker combination according to claim 23, comprising the following biomarkers: ureidopropionic acid, γ-CEHC, and thyroxine; or ureidopropionic acid, γ-CEHC, and pipecolic acid; or ureidopropionic acid and γ-CEHC.

25. The biomarker combination according to claim 23, further comprising one or more of total bilirubin (TB), international normalized ratio (INR), and Cirrhosis.

26. A system for predicting a mortality risk or a probability of survival of an acute-on-chronic liver failure (ACLF) patient, comprising a data analysis module, wherein the data analysis module is configured to analyze a detected value of a biomarker, and the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

27. The system according to claim 26, wherein the detected value of the biomarker refers to detection of the biomarker in a blood, serum, plasma, or whole blood sample from the ACLF patient, and is used for detecting a presence or absence, relative abundance, or concentration of the biomarker in the sample.

28. The system according to claim 26, wherein the biomarker comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and γ-CEHC; or, the biomarker comprises pipecolic acid, NAAG, ureidopropionic acid, formiminoglutamic acid, γ-CEHC, and γ-glu-phe; or, the biomarker comprises pipecolic acid, NAAG, ureidopropionic acid, formiminoglutamic acid, γ-CEHC, γ-glu-phe, and 3-OH butyrylcarnitine; or, the biomarker comprises pipecolic acid, NAAG, ureidopropionic acid, formiminoglutamic acid, γ-CEHC, γ-glu-phe, 3-OH butyrylcarnitine, and estrone sulfate; or, the biomarker comprises pipecolic acid, NAAG, ureidopropionic acid, formiminoglutamic acid, γ-CEHC, γ-glu-phe, 3-OH butyrylcarnitine, estrone sulfate, and hydroxyproline; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

29. The system according to claim 26, wherein the data analysis module calculates a prediction value for predicting a mortality risk of the ACLF patient by substituting the detected value of the biomarker into an equation, thereby assessing the mortality risk of the ACLF patient.

30. The system according to claim 29, wherein the equation is a logistic regression equation.

31. The system according to claim 30, wherein the logistic regression equation is obtained by constructing and training with detected values of biomarkers from known samples.

32. The system according to claim 31, wherein the training is to construct a training set and train the training set using a training method to obtain the logistic regression equation; and the training set comprises serum samples from ACLF patients.

33. The system according to claim 32, wherein the training method comprises: dividing the training set into a survivor group and a non-survivor group according to a survival status within a short term; detecting biomarker levels in the survivor group and the non-survivor group respectively to obtain the detected values; performing logistic regression according to the detected values of the biomarkers to analyze relationships between the detected values in the survivor group and the non-survivor group; and constructing the logistic regression equation.

34. The system according to claim 33, wherein the short term comprises any one or more of within 90 days, within 180 days, within 360 days, within 80 days, within 70 days, within 60 days, within three months, within half a year, within one year, within two months, and within one month.

35. The system according to claim 34, wherein the data analysis module calculates a prediction value for predicting a mortality risk of the ACLF patient within 360 days by substituting the detected value of the biomarker into the logistic regression equation, thereby assessing the mortality risk of the ACLF patient within 360 days.

36. The system according to claim 30, wherein the logistic regression equation is:

P = -20.584 + 0.846 * pipecolic acid + 1.254 * NAAG + 0.454 * ureidopropionic acid - 0.468 * formiminoglutamic acid - 0.385 * γ-CEHC + 0.485 * γ-glu-phe + 0.447 * 3-OH butyrylcarnitine + 0.081 * estrone sulfate + 0.524 * hydroxyproline + 0.079 * bilirubin - 0.066 * 2-hydroxydadipic acid - 0.015 * methionine sulfoxide - 0.089 * hexadecanedioic acid - 0.219 * quinolinic acid + 0.942 * thyroxine;

or P = -14.286 + 0.804 * pipecolic acid + 0.791 * NAAG + 0.448 * ureidopropionic acid - 0.396 * formiminoglutamic acid - 0.338 * γ-CEHC + 0.434 * γ-glu-phe + 0.425 * 3-OH butyrylcarnitine + 0.107 * estrone sulfate + 0.356 * hydroxyproline + 0.091 * bilirubin - 0.082 * 2-hydroxydadipic acid + 0.015 * methionine sulfoxide - 0.066 * hexadecanedioic acid - 0.154 * quinolinic acid;

or P = -14.392 + 0.799 * pipecolic acid + 0.792 * NAAG + 0.383 * ureidopropionic acid - 0.403 * formiminoglutamic acid - 0.352 * γ-CEHC + 0.428 * γ-glu-phe + 0.388 * 3-OH butyrylcarnitine + 0.121 * estrone sulfate + 0.353 * hydroxyproline + 0.095 * bilirubin - 0.153 * 2-hydroxydadipic acid + 0.009 * methionine sulfoxide - 0.025 * hexadecanedioic acid;

or P = -14.5 + 0.794 * pipecolic acid + 0.806 * NAAG + 0.376 * ureidopropionic acid-0.403 * formiminoglutamic acid - 0.352 * γ-CEHC + 0.429 * γ-glu-phe + 0.378 * 3-OH butyrylcarnitine + 0.114 * estrone sulfate + 0.362 * hydroxyproline + 0.095 * bilirubin - 0.16 * 2-hydroxydadipic acid + 0.007 * methionine sulfoxide;

or P = -14.476 + 0.795 * pipecolic acid + 0.807 * NAAG + 0.376 * ureidopropionic acid - 0.404 * formiminoglutamic acid - 0.353 * γ-CEHC + 0.43 * γ-glu-phe + 0.375 * 3-OH butyrylcarnitine + 0.115 * estrone sulfate + 0.364 * hydroxyproline + 0.096 * bilirubin - 0.157 * 2-hydroxydadipic acid;

or P = -14.315 + 0.777 * pipecolic acid + 0.77 * NAAG + 0.342 * ureidopropionic acid - 0.411 * formiminoglutamic acid - 0.352 * γ-CEHC + 0.432 * γ-glu-phe + 0.325 * 3-OH butyrylcarnitine + 0.109 * estrone sulfate + 0.357 * hydroxyproline + 0.1 * bilirubin;

or P = -13.898 + 0.825 * pipecolic acid + 0.744 * NAAG + 0.322 * ureidopropionic acid + -0.391 * formiminoglutamic acid + -0.269 * γ-CEHC + 0.424 * γ-glu-phe + 0.345 * 3-OH butyrylcarnitine + 0.162 * estrone sulfate + 0.276 * hydroxyproline;

or P = -12.473 + 0.857 * pipecolic acid + 0.781 * NAAG + 0.415 * ureidopropionic acid - 0.405 * formiminoglutamic acid - 0.257 * γ-CEHC + 0.42 * γ-glu-phe + 0.343 * 3-OH butyrylcarnitine + 0.14 * estrone sulfate;

or P = -13.147 + 0.947 * pipecolic acid + 0.832 * NAAG + 0.435 * ureidopropionic acid - 0.407 * formiminoglutamic acid - 0.292 * γ-CEHC + 0.428 * γ-glu-phe + 0.334 * 3-OH butyrylcarnitine;

or P = -12.838 + 0.839 * pipecolic acid + 1.125 * NAAG + 0.49 * ureidopropionic acid - 0.39 * formiminoglutamic acid - 0.263 * γ-CEHC + 0.39 * γ-glu-phe;

or P=-10.874 + 0.685 * pipecolic acid + 1.105 * NAAG + 0.51 * ureidopropionic acid-0.214 * formiminoglutamic acid - 0.22 * γ-CEHC;

or P = **-11.911** + 0.81 * pipecolic acid + 0.992 * NAAG + 0.559 * ureidopropionic acid - 0.2 * formiminoglutamic acid;

or

$$P = -11.25 + 0.73 * \text{pipecolic acid} + 0.917 * \text{NAAG} + 0.435 * \text{ureidopropionic acid},$$

wherein P represents the prediction value for predicting the mortality risk of the ACLF patient.

37. The system according to claim 36, wherein the logistic regression equation further comprises one or more of clinical indicators: age, international normalized ratio (INR), creatine, Sodium, albumin, neutrophil/lymphocyte count ratio, overt hepatic encephalopathy, and total bilirubin (TB).

38. The system according to claim 35, wherein the logistic regression equation is: P = -11.911 + 0.81 * pipecolic acid + 0.992 * NAAG + 0.559 * ureidopropionic acid - 0.2 * formiminoglutamic acid; or P = -11.25 + 0.73 * pipecolic acid + 0.917 * NAAG + 0.435 * ureidopropionic acid, wherein P represents a prediction value for predicting a mortality risk of the ACLF patient within 90 days.

39. The system according to claim 38, wherein when the logistic regression equation is:

P = -11.911 + 0.81 * pipecolic acid + 0.992 * NAAG + 0.559 * ureidopropionic acid - 0.2 * formiminoglutamic acid, when P is greater than 0.331, the mortality risk of the ACLF patient within 90 days is predicted to be high; and when P is less than 0.331, the mortality risk of the ACLF patient within 90 days is predicted to be low; and when the logistic regression equation is:

P = -11.25 + 0.73 * pipecolic acid + 0.917 * NAAG + 0.435 * ureidopropionic acid, when P is greater than 0.320, the mortality risk of the ACLF patient within 90 days is predicted to be high; and when P is less than 0.320, the mortality risk of the ACLF patient within 90 days is predicted to be low.

40. The system according to claim 26, further comprising a data storage module, a data input interface, and a data output interface, wherein the data storage module is configured to store the detected value of the biomarker; the data input interface is configured to input the detected value of the biomarker; and the data output interface is configured to output a prediction result.

41. A system for predicting progression to acute-on-chronic liver failure (ACLF) of a non-ACLF patient, comprising a data analysis module, wherein the data analysis module is configured to analyze a detected value of a biomarker, and the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

42. The system according to claim 41, wherein the detected value of the biomarker refers to detection of the biomarker in a blood, serum, plasma, or whole blood sample from the non-ACLF patient, and is used for detecting a presence or absence, relative abundance, or concentration of the biomarker in the sample.

43. The system according to claim 41, wherein the biomarker comprises ureidopropionic acid and γ-CEHC; or, the biomarker comprises ureidopropionic acid, γ-CEHC, and thyroxine; or, the biomarker comprises ureidopropionic acid, γ-CEHC, and pipecolic acid; or, the biomarker comprises ureidopropionic acid, γ-CEHC, thyroxine, and pipecolic acid; or, the biomarker comprises ureidopropionic acid, γ-CEHC, thyroxine, pipecolic acid, and 3-OH butyrylcarnitine; or, the biomarker comprises ureidopropionic acid, γ-CEHC, thyroxine, pipecolic acid, 3-OH butyrylcarnitine, and 2-hydroxydadipic acid; or, the biomarker comprises ureidopropionic acid, γ-CEHC, thyroxine, pipecolic acid, 3-OH butyrylcarnitine, 2-hydroxydadipic acid, and estrone sulfate; or, the biomarker comprises ureidopropionic acid, γ-CEHC, thyroxine, pipecolic acid, 3-OH butyrylcarnitine, 2-hydroxydadipic acid, estrone sulfate, and hydroxyproline; or, the biomarker comprises ureidopropionic acid, γ-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glu-phe; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and NAAG.

44. The system according to claim 41, wherein the data analysis module comprises an operational equation and calculates a prediction value for predicting progression to ACLF of the non-ACLF patient using an operational method, thereby assessing a progression risk to ACLF of the non-ACLF patient.

45. The system according to claim 44, wherein the equation is a logistic regression equation.

46. The system according to claim 45, wherein the logistic regression equation is obtained by constructing and training with detected values of biomarkers from known samples.

47. The system according to claim 46, wherein the training is to construct a training set and train the training set using a training method to obtain the logistic regression equation; and the training set comprises serum samples from non-ACLF patients.

48. The system according to claim 47, wherein the training method comprises: dividing the training set into a survivor group and a non-survivor group according to a survival status within a short term; detecting biomarker levels in the survivor group and the non-survivor group respectively to obtain the detected values; performing logistic regression according to the detected values of the biomarkers to analyze relationships between the detected values in the survivor group and the non-survivor group; and constructing the logistic regression equation.

49. The system according to claim 48, wherein the short term comprises any one or more of within 180 days, within 90 days, within 80 days, within 70 days, within 60 days, within 28 days, within 3 months, within 4 months, within 5 months, within half a year, within 7 months, within 8 months, within 9 months, within 10 weeks, within 9 weeks, within 8 weeks, within 7 weeks, within 6 weeks, within 5 weeks, within 4 weeks, within 3 weeks, within 2 weeks, and within 1 week.

50. The system according to claim 49, wherein the data analysis module calculates a prediction value for predicting progression to ACLF of the non-ACLF patient within 180 days by substituting the detected value of the biomarker into the logistic regression equation.

51. The system according to claim 49, wherein the data analysis module calculates the prediction value for predicting progression to ACLF of the non-ACLF patient by substituting the detected value of the biomarker into the logistic regression equation, and the logistic regression equation is:

P = -3.271 + 0.716 * ureidopropionic acid - 0.512 * γ-CEHC - 1.1 * thyroxine + 0.357 * pipecolic acid + 0.134 * 3-OH butyrylcarnitine - 1.225 * 2-hydroxydadipic acid + 0.029 * estrone sulfate + 0.168 * hydroxyproline - 0.04 * quinolinic acid + 0.078 * γ-glu-phe - 0.014 * formiminoglutamic acid - 0.03 * bilirubin + 0.519 * hexadecanedioic acid - 0.139 * methionine sulfoxide + 0.653 * NAAG;

or P = -1.518 + 0.724 * ureidopropionic acid - 0.494 * γ-CEHC - 1.216 * thyroxine + 0.409 * pipecolic acid + 0.157 * 3-OH butyrylcarnitine - 1.131 * 2-hydroxydadipic acid + 0.051 * estrone sulfate + 0.202 * hydroxyproline - 0.024 * quinolinic acid + 0.11 * γ-glu-phe - 0.007 * formiminoglutamic acid + 0.009 * bilirubin + 0.479 * hexadecanedioic acid - 0.17 * methionine sulfoxide;

or P = -2.436 + 0.726 * ureidopropionic acid + -0.498 * γ-CEHC - 1.18 * thyroxine + 0.382 * pipecolic acid + 0.195 * 3-OH butyrylcarnitine - 1.121 * 2-hydroxydadipic acid + 0.044 * estrone sulfate + 0.199 * hydroxyproline - 0.009 * quinolinic acid + 0.046 * γ-glu-phe - 0.014 * formiminoglutamic acid - 0.012 * bilirubin + 0.446 * hexadecanedioic acid;

or P = -2.837 + 0.819 * ureidopropionic acid - 0.514 * γ-CEHC - 1.019 * thyroxine + 0.399 * pipecolic acid + 0.362 * 3-OH butyrylcarnitine - 0.932 * 2-hydroxydadipic acid + 0.173 * estrone sulfate + 0.235 * hydroxyproline - 0.08 * quinolinic acid + 0.074 * γ-glu-phe + 0.001 * formiminoglutamic acid - 0.02 * bilirubin;

or P = -2.894 + 0.816 * ureidopropionic acid - 0.512 * γ-CEHC - 1.016 * thyroxine + 0.395 * pipecolic acid + 0.362 * 3-OH butyrylcarnitine - 0.93 * 2-hydroxydadipic acid + 0.166 * estrone sulfate + 0.238 * hydroxyproline - 0.076 * quinolinic acid + 0.071 * γ-glu-phe + 0.001 * formiminoglutamic acid;

or P = -2.899 + 0.816 * ureidopropionic acid - 0.512 * γ-CEHC - 1.015 * thyroxine + 0.396 * pipecolic acid + 0.362 * 3-OH butyrylcarnitine - 0.929 * 2-hydroxydadipic acid + 0.166 * estrone sulfate + 0.238 * hydroxyproline - 0.076 * quinolinic acid + 0.071 * γ-glu-phe;

or P = -2.647 + 0.828 * ureidopropionic acid - 0.512 * γ-CEHC - 1.039 * thyroxine + 0.388 * pipecolic acid + 0.347 * 3-OH butyrylcarnitine - 0.926 * 2-hydroxydadipic acid + 0.163 * estrone sulfate + 0.241 * hydroxyproline - 0.047 * quinolinic acid;

or P = -2.647 + 0.816 * ureidopropionic acid - 0.513 * γ-CEHC - 1.049 * thyroxine + 0.393 * pipecolic acid + 0.34 * 3-OH butyrylcamitine - 0.939 * 2-hydroxydadipic acid + 0.164 * estrone sulfate + 0.23 * hydroxyproline;

or P = -0.998 + 0.856 * ureidopropionic acid - 0.502 * γ-CEHC - 1.104 * thyroxine + 0.408 * pipecolic acid + 0.325 * 3-OH butyrylcarnitine + -0.916 * 2-hydroxydadipic acid + 0.154 * estrone sulfate;

or P = -1.935 + 0.919 * ureidopropionic acid - 0.537 * γ-CEHC - 1.018 * thyroxine + 0.464 * pipecolic acid + 0.296 * 3-OH butyrylcarnitine - 0.867 * 2-hydroxydadipic acid;

or P = -2.077 + 0.745 * ureidopropionic acid - 0.539 * γ-CEHC - 0.806 * thyroxine + 0.191 * pipecolic acid + 0.088 * 3-OH butyrylcarnitine;

or P = -1.878 + 0.77 * ureidopropionic acid - 0.54 * γ-CEHC - 0.827 * thyroxine + 0.188 * pipecolic acid;

or

$$P = -0.983 + 0.845 * \text{ureidopropionic acid} - 0.565 * \gamma\text{-CEHC} - 0.835 * \text{thyroxine};$$

or

$$P = -4.744 + 1.015 * \text{ureidopropionic acid} - 0.584 * \gamma\text{-CEHC} + 0.122 * \text{pipecolic acid};$$

or

$$P = -4.803 + 0.930 * \text{ureidopropionic acid} - 0.563 * \gamma\text{-CEHC},$$

wherein P represents the prediction value for predicting progression to ACLF of the non-ACLF patient.

52. The system according to claim 51, wherein the logistic regression equation further comprises one or more of clinical indicators: total bilirubin (TB), international normalized ratio (INR), and Cirrhosis.

53. The system according to claim 51, wherein the logistic regression equation is:

$$P = -0.983 + 0.845 * \text{ureidopropionic acid} - 0.565 * \gamma\text{-CEHC} - 0.835 * \text{thyroxine};$$

or

$$P = -4.803 + 0.930 * \text{ureidopropionic acid} - 0.563 * \gamma\text{-CEHC},$$

wherein P represents a prediction value for predicting progression to ACLF of the non-ACLF patient within 28 days.

54. The system according to claim 52, wherein when the logistic regression equation is: P = -0.983 + 0.845 * ureidopropionic acid - 0.565 * γ-CEHC - 0.835 * thyroxine, when P is greater than 0.150, a progression risk to ACLF of the non-ACLF patient within 28 days is predicted to be high; and when P is less than 0.150, a progression risk to ACLF of the non-ACLF patient within 28 days is predicted to be low; and
when the logistic regression equation is: P = -4.803 + 0.930 * ureidopropionic acid - 0.563 * γ-CEHC, when P is greater than 0.123, a progression risk to ACLF of the non-ACLF patient within 28 days is predicted to be high; and when P is less than 0.123, a progression risk to ACLF of the non-ACLF patient within 28 days is predicted to be low.

55. The system according to claim 41, further comprising a data storage module, a data input interface, and a data output interface, wherein the data storage module is configured to store the detected value of the biomarker; the data input interface is configured to input the detected value of the biomarker; and the data output interface is configured to output a prediction result.

56. A method for predicting a mortality risk of an acute-on-chronic liver failure (ACLF) patient, used for predicting the mortality risk of the ACLF patient by analyzing a detected value of a biomarker in a liquid sample, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

57. The method according to claim 56, wherein the detected value of the biomarker refers to detection of the biomarker in a blood, serum, plasma, or whole blood sample from the ACLF patient, and is used for detecting a presence or absence, relative abundance, or concentration of the biomarker in the sample.

58. The method according to claim 56, wherein the biomarker further comprises pipecolic acid, NAAG, and ureidopropionic acid; or the biomarker comprises pipecolic acid, NAAG, ureidopropionic acid, and formiminoglutamic acid; or, the biomarker comprises pipecolic acid, NAAG, ureidopropionic acid, formiminoglutamic acid, and γ-CEHC; or, the biomarker comprises pipecolic acid, NAAG, ureidopropionic acid, formiminoglutamic acid, γ-CEHC, and γ-glu-phe; or, the biomarker comprises pipecolic acid, NAAG, ureidopropionic acid, formiminoglutamic acid, γ-CEHC, γ-glu-phe, and 3-OH butyrylcarnitine; or, the biomarker comprises pipecolic acid, NAAG, ureidopropionic acid, formiminoglutamic acid, γ-CEHC, γ-glu-phe, 3-OH butyrylcarnitine, and estrone sulfate; or, the biomarker comprises pipecolic acid, NAAG, ureidopropionic acid, formiminoglutamic acid, γ-CEHC, γ-glu-phe, 3-OH butyrylcarnitine, estrone sulfate, and hydroxyproline; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, and bilirubin; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, and 2-hydroxydadipic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, and methionine sulfoxide; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, and hexadecanedioic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, and quinolinic acid; or, the biomarker comprises pipecolic acid, NAAG, ureidopropionic acid, formiminoglutamic acid, γ-CEHC, γ-glu-phe, 3-OH butyrylcarnitine, estrone sulfate, hydroxyproline, bilirubin, 2-hydroxydadipic acid, methionine sulfoxide, hexadecanedioic acid, quinolinic acid, and thyroxine.

59. The method according to claim 56, comprising calculating a prediction value for predicting a mortality risk of the ACLF patient by substituting the detected value of the biomarker into an equation, thereby assessing the mortality risk of the ACLF patient.

60. The method according to claim 59, wherein the equation is a regression equation.

61. The method according to claim 56, wherein the predicting the mortality risk of the ACLF patient refers to predicting a mortality risk of the ACLF patient within a short term.

62. The method according to claim 59, wherein the equation is obtained by constructing and training with detected values of biomarkers from known samples.

63. The method according to claim 62, wherein the training is to construct a training set and train the training set using a training method to obtain the equation; and the training set comprises serum samples from ACLF patients.

64. A method for predicting progression to acute-on-chronic liver failure (ACLF) of a non-ACLF patient, used for predicting a short-term progression risk to ACLF of the non-ACLF patient by analyzing a detected value of a biomarker in a liquid sample, wherein the biomarker comprises one or more of: 2-hydroxydadipic acid, hexadecanedioic acid, hydroxyproline, methionine sulfoxide, 3-OH butyrylcarnitine, pipecolic acid, estrone sulfate, N-acetylaspartylglutamate (NAAG), quinolinic acid, ureidopropionic acid, γ-carboxyethylhydroxychroman (γ-CEHC), thyroxine, formiminoglutamic acid, γ-glutamylphenylalanine (γ-glu-phe), and bilirubin.

65. The method according to claim 64, wherein the detected value of the biomarker refers to detection of the biomarker in a blood, serum, plasma, or whole blood sample from the ACLF patient, and is used for detecting a presence or absence, relative abundance, or concentration of the biomarker in the sample.

**66.** The method according to claim 64, wherein the predicting progression to ACLF of the non-ACLF patient refers to predicting progression to ACLF of the non-ACLF patient within a short term.

**67.** The method according to claim 64, wherein the biomarker comprises ureidopropionic acid and γ-CEHC; or, the biomarker comprises ureidopropionic acid, γ-CEHC, and thyroxine; or, the biomarker comprises ureidopropionic acid, γ-CEHC, and pipecolic acid; or, the biomarker comprises ureidopropionic acid, γ-CEHC, thyroxine, and pipecolic acid; or, the biomarker comprises ureidopropionic acid, γ-CEHC, thyroxine, pipecolic acid, and 3-OH butyrylcarnitine; or, the biomarker comprises ureidopropionic acid, γ-CEHC, thyroxine, pipecolic acid, 3-OH butyrylcarnitine, and 2-hydroxydadipic acid; or, the biomarker comprises ureidopropionic acid, γ-CEHC, thyroxine, pipecolic acid, 3-OH butyrylcarnitine, 2-hydroxydadipic acid, and estrone sulfate; or, the biomarker comprises ureidopropionic acid, γ-CEHC, thyroxine, pipecolic acid, 3-OH butyrylcarnitine, 2-hydroxydadipic acid, estrone sulfate, and hydroxyproline; or, the biomarker comprises ureidopropionic acid, γ-CEHC, thyroxine, pipecolic acid, 3-OH butyrylcarnitine, 2-hydroxydadipic acid, estrone sulfate, hydroxyproline, and quinolinic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, and γ-glu-phe; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, and formiminoglutamic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, and bilirubin; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, and hexadecanedioic acid; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, and methionine sulfoxide; or, the biomarker comprises ureidopropionic acid, γ-CEHC, thyroxine, pipecolic acid, 3-OH butyrylcarnitine, 2-hydroxydadipic acid, estrone sulfate, hydroxyproline, quinolinic acid, γ-glu-phe, formiminoglutamic acid, bilirubin, hexadecanedioic acid, methionine sulfoxide, and NAAG.

**68.** The method according to claim 64, comprising calculating a prediction value for predicting progression to ACLF of the non-ACLF patient by substituting the detected value of the biomarker into an equation, thereby assessing the progression risk to ACLF of the non-ACLF patient.

**69.** The method according to claim 68, wherein the equation is a logistic regression equation.

**70.** The method according to claim 68, wherein the equation is obtained by constructing and training with detected values of biomarkers from known samples.

**71.** The method according to claim 70, wherein the training is to construct a training set and train the training set using a training method to obtain the equation; and the training set comprises serum samples from non-ACLF patients.

**72.** The method according to claim 71, wherein the training method comprises: dividing the training set into a pre-ACLF group and a non-ACLF group according to an ACLF progression status within a short term; detecting biomarker levels in the pre-ACLF group and the non-ACLF group respectively to obtain the detected values; performing calculation according to the detected values of the biomarkers to analyze relationships between the detected values in the pre-ACLF group and the non-ACLF group; and constructing the equation.

**73.** The method according to claim 69, comprising calculating a prediction value for predicting progression to ACLF of the non-ACLF patient within 180 days by substituting the detected value of the biomarker into the logistic regression equation.

Fig. 1

Fig. 2

Fig. 3

EP 4 692 783 A1

Fig. 4

Fig. 5

Fig. 6

Fig. 7

Fig. 8

Fig. 9

Validation set

Fig. 10

Fig. 11

Fig. 12

Discovery set

| | | AUROC (95% CI) | P value vs. Combined model |
|---|---|---|---|
| ~~~~ | Combined model | 0.81 (0.75-0.87) | – |
| ~~~~ | CLIF-C ACLF | 0.76 (0.69-0.82) | 0.016 |
| ~~~~ | MELD-Na | 0.73 (0.66-0.80) | 0.009 |
| ~~~~ | 4-Metabolite model | 0.77 (0.70-0.83) | 0.047 |

Fig. 13

Validation set

| | | AUROC (95% CI) | P value vs. Combined model |
|---|---|---|---|
| ~~~ | Combined model | 0.80 (0.73-0.86) | - |
| ~~~ | CLIF.C ACLF | 0.75 (0.68-0.82) | 0.044 |
| ~~~ | MELD-Na | 0.74 (0.66-0.81) | 0.015 |
| ~~~ | 4-Metabolite model | 0.75 (0.68-0.82) | 0.044 |

Fig. 14

Discovery set

| | | AUROC (95% CI) | P value vs. Combined model |
|---|---|---|---|
| ~~~~ | Combined model | 0.85 (0.80-0.90) | - |
| ~~~~ | CLIF.C ACLF-D | 0.73 (0.67-0.80) | <0.001 |
| ~~~~ | MELD-Na | 0.80 (0.74-0.86) | 0.013 |
| ~~~~ | 3-Metabolite model | 0.78 (0.71-0.84) | 0.005 |

Fig. 15

Fig. 16

## INTERNATIONAL SEARCH REPORT

| International application No. |
|---|
| **PCT/CN2023/093183** |

**A. CLASSIFICATION OF SUBJECT MATTER**

G01N 30/02(2006.01)i

According to International Patent Classification (IPC) or to both national classification and IPC

**B. FIELDS SEARCHED**

Minimum documentation searched (classification system followed by classification symbols)

IPC: G01N

Documentation searched other than minimum documentation to the extent that such documents are included in the fields searched

Electronic data base consulted during the international search (name of data base and, where practicable, search terms used)

WPABS, CNTXT, ENTXT, VEN, CNKI, 万方, WANFANG, 读秀, DUXIU, ISI Web of Knowledge, Elsevier Science, ACS, Pubmed, RSC, IEEE, Wiley: 肝, 衰竭, 损伤, 肝损, 障碍, 硬化, 慢加急, 亚急, ACLF, 标志, 标记, 标识, 指示, 指标, acute-on-chronic liver failure, liver, hepat+, lung, injur+, failure+, marker+, biomarker+

**C. DOCUMENTS CONSIDERED TO BE RELEVANT**

| Category* | Citation of document, with indication, where appropriate, of the relevant passages | Relevant to claim No. |
|---|---|---|
| X | Valantine B et al. "Predictors of early mortality among patients with acute-on-chronic liver failure"<br>*JGH Open*, Vol. 5, No. 6, 19 May 2021 (2021-05-19),<br><br>pages 686-693 | 1, 5-9, 13-19, 26-27, 29-35, 37, 40-42, 44-50, 52, 55-57, 59-66, 68-73 |
| Y | Valantine B et al. "Predictors of early mortality among patients with acute-on-chronic liver failure"<br>*JGH Open*, Vol. 5, No. 6, 19 May 2021 (2021-05-19),<br>pages 686-693 | 3-4, 11 |
| X | US 2008213768 A1 (CAI ZHAUHUI et al.) 04 September 2008 (2008-09-04)<br>claims 1-4, and description, paragraphs 24-104 | 1, 3-9, 11, 13-19, 26-27, 29-35, 37, 40-42, 44-50, 52, 55-57, 59-66, 68-73 |
| Y | US 2008213768 A1 (CAI ZHAUHUI et al.) 04 September 2008 (2008-09-04)<br>claims 1-4, and description, paragraphs 24-104 | 3-4, 11 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 December 2023** | **28 December 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | PCT/CN2023/093183 |

**C. DOCUMENTS CONSIDERED TO BE RELEVANT**

| Category* | Citation of document, with indication, where appropriate, of the relevant passages | Relevant to claim No. |
|---|---|---|
| X | CN 112964807 A (ZHEJIANG UNIVERSITY) 15 June 2021 (2021-06-15)<br>description, paragraphs 4-19 | 1, 5-9, 13-19, 26-27, 29-35, 37, 40-42, 44-50, 52, 55-57, 59-66, 68-73 |
| X | US 2015377883 A1 (THE REGENTS OF THE UNIVERSITY OF MICHIGAN) 31 December 2015 (2015-12-31)<br>description, paragraphs 4-16 and 27-130 | 1, 5-9, 13-19, 26-27, 29-35, 37, 40-42, 44-50, 52, 55-57, 59-66, 68-73 |
| X | WO 2015155165 A1 (FUNDACIÓ CLINIC PER A LA RECERCA BIOMÈDICA (FCRB))<br>15 October 2015 (2015-10-15)<br>description, pages 30-34 | 1, 5-9, 13-19, 26-27, 29-35, 37, 40-42, 44-50, 52, 55-57, 59-66, 68-73 |
| X | Irwin Jack Kurland et al. "Integrative Metabolic Signatures for Hepatic Radiation Injury"<br>*PLOS ONE*, Vol. 10, No. (6), 05 June 2015 (2015-06-05),<br><br>pages 1-24 | 1, 3-9, 11, 13-19, 26-27, 29-35, 37, 40-42, 44-50, 52, 55-57, 59-66, 68-73 |
| X | Kama A Wlodzimirow et al. "A systematic review on prognostic indicators of acute on chronic liver failure and their predictive value for mortality"<br>*Liver International*, Vol. 33, No. (1), 31 January 2013 (2013-01-31),<br><br>pages 40-52 | 1, 5-9, 13-19, 26-27, 29-35, 37, 40-42, 44-50, 52, 55-57, 59-66, 68-73 |
| X | Jorge A López-Velázquez et al. "Bilirubin alone as abiomarker for S-Hort-term mortality in acute-on-chronic liver failure: animportant prognostic indicator"<br>*Annals of Hepatology*, Vol. (13), No. 1, 28 February 2014 (2014-02-28),<br><br>pages 98-104 | 1, 5-9, 13-19, 26-27, 29-35, 37, 40-42, 44-50, 52, 55-57, 59-66, 68-73 |
| X | 李巍 (LI, Wei). "肝衰竭患者呼出气体中标志性化合物与血清生化指标的相关研究 (Non-official translation: Related Research on Symbolic Compounds in Exhaled Gas of Patients with Liver Failure and Biochemical Indexes of Serum)"<br>*中国优秀硕士学位论文全文数据库 医药卫生科技辑 (Medicine and Health Sciences, China Master's Theses Full-text Database)*, No. 4, 15 April 2018 (2018-04-15),<br>abstract | 1, 5-9, 13-19, 26-27, 29-35, 37, 40-42, 44-50, 52, 55-57, 59-66, 68-73 |
| X | 于梦倩 (YU, Mengqian). "基于超高效液相色谱质谱联用的人工肝治疗肝衰竭血清代谢组学分析 (Non-official translation: Human Liver Failure Serum Metabonomics Analysis based on Ultra-High Performance Liquid Chromatography-Mass Spectrometry)"<br>*中国优秀硕士学位论文全文数据库 医药卫生科技辑 (Medicine and Health Sciences, China Master's Theses Full-text Database)*, No. 1, 15 January 2023 (2023-01-15),<br>abstract, and pages 36-37 | 1, 5-9, 13-19, 26-27, 29-35, 37, 40-42, 44-50, 52, 55-57, 59-66, 68-73 |
| X | 刘春燕 (LIU, Chunyan). "296例慢加急性肝衰竭临床特征及预警预后分析 (Non-official translation: Clinical Features of 296 Examples of Chronic Acute Liver Failure and Early Warning Prognosis Analysis)"<br>*中国优秀硕士学位论文全文数据库 医药卫生科技辑 (Medicine and Health Sciences, China Master's Theses Full-text Database)*, No. 1, 15 January 2019 (2019-01-15),<br>abstract | 1, 5-9, 13-19, 26-27, 29-35, 37, 40-42, 44-50, 52, 55-57, 59-66, 68-73 |
| X | 陆敏嘉等 (LU, Minjia et al.). "慢加急性肝衰竭前期预警标志物的研究进展 (Research Advances on Warning Markers of Acute-on-Chronic Pre-Liver Failure)"<br>*抗感染药学 (Anti-infection Pharmacy)*, Vol. 18, No. 10, 31 December 2021 (2021-12-31),<br><br>pages 1405-1411 | 1, 5-9, 13-19, 26-27, 29-35, 37, 40-42, 44-50, 52, 55-57, 59-66, 68-73 |
| A | CN 113528639 A (NANFANG HOSPITAL OF SOUTHERN MEDICAL UNIVERSITY) 22 October 2021 (2021-10-22)<br>entire document | 1-73 |

Form PCT/ISA/210 (second sheet) (July 2022)

## INTERNATIONAL SEARCH REPORT
### Information on patent family members

International application No.

**PCT/CN2023/093183**

| Patent document cited in search report | | | Publication date (day/month/year) | Patent family member(s) | | | Publication date (day/month/year) |
|---|---|---|---|---|---|---|---|
| US | 2008213768 | A1 | 04 September 2008 | None | | | |
| CN | 112964807 | A | 15 June 2021 | None | | | |
| US | 2015377883 | A1 | 31 December 2015 | US | 2018031558 | A1 | 01 February 2018 |
| WO | 2015155165 | A1 | 15 October 2015 | EP | 2930247 | A1 | 14 October 2015 |
| CN | 113528639 | A | 22 October 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023103293484
- CN 2023103293785